# EUROPÄISCHE PATENTSCHRIFT

(11) **EP 1 758 966 B1**
(45) Veröffentlichungstag und Bekanntmachung des Hinweises auf die Patenterteilung: **27.07.2011**
(21) Anmeldenummer: 05754989.1
(22) Anmeldetag: 08.06.2005
(51) Int. Cl.: C09K 19/12, G02F 1/13, C07C 25/24, C07C 25/18, C07C 381/00

(54) **FLÜSSIGKRISTALLINES MEDIUM**
LIQUID CRYSTAL MEDIUM
AGENT EN CRISTAUX LIQUIDES

(30) Priorität: 18.06.2004 DE 102004029492
(43) Veröffentlichungstag der Anmeldung: 07.03.2007
(73) Patentinhaber: Merck Patent GmbH, 64293 Darmstadt (DE)
(72) Erfinder: MANABE, Atsutaka, 64625 Bensheim (DE); MONTENEGRO, Elvira, 69469 Weinheim (DE); PAULUTH, Detlef, 64372 Ober-Ramstadt (DE)
(86) Internationale Anmeldenummer: PCT/EP2005/006138
(87) Internationale Veröffentlichungsnummer: WO 2005/123878

(56) Entgegenhaltungen:
- EP-A- 0 439 089
- EP-A- 0 949 231
- EP-A- 1 346 995
- EP-A- 1 352 944
- WO-A-89/02425
- WO-A-2004/035710
- DE-A1- 10 128 017
- US-A1- 2002 061 368

## Beschreibung

Die vorliegende Erfindung betrifft flüssigkristalline Verbindungen und ein flüssigkristallines Medium, sowie dessen Verwendung für elektrooptische Zwecke und dieses Medium enthaltende Anzeigen.

Flüssige Kristalle werden vor allem als Dielektrika in Anzeigevorrichtungen verwendet, da die optischen Eigenschaften solcher Substanzen durch eine angelegte Spannung beeinflusst werden können. Elektrooptische Vorrichtungen auf der Basis von Flüssigkristallen sind dem Fachmann bestens erkannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("superbirefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben.

Weiterhin sollten sie bei üblichen Betriebstemperaturen, d.h. in einem möglichst breiten Bereich unterhalb und oberhalb Raumtemperatur eine geeignete Mesophase besitzen, beispielsweise für die oben genannten Zellen eine nematische oder cholesterische Mesophase. Da Flüssigkristalle in der Regel als Mischungen mehrerer Komponenten zur Anwendung gelangen, ist es wichtig, dass die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine geringe elektrische Leitfähigkeit aufweisen.

Beispielsweise sind für Matrix-Flüssigkristallanzeigen mit integrierten nichtlinearen Elementen zur Schaltung einzelner Bildpunkte (MFK-Anzeigen) Medien mit großer positiver dielektrischer Anisotropie, breiten nematischen Phasen, relativ niedriger Doppelbrechung, sehr hohem spezifischen Widerstand, guter UV- und Temperaturstabilität und geringerem Dampfdruck erwünscht.

Weiterhin sind auch LCoS^{™}-Anzeigen und Anzeigen, die auf einem Doppelbrechungseffekt beruhen, wie OCB-Anzeigen, interessant.

OCB-Anzeigen ("optically compensated bend") beruhen auf einem Doppelbrechungseffekt und enthalten eine Flüssigkristallschicht mit einer sogenannten "bend"-Struktur. Die "bend"-Zelle, auch bekannt als "pi"-Zelle, wurde erstmals von P. Bos et al., SID 83 Digest, 30 (1983) für eine elektrisch kontrollierbare λ/2-Platte vorgeschlagen, während der OCB-Modus für Anzeigen von Y. Yamaguchi, T. Miyashita und T. Uchida, SID 93 Digest, 277 (1993), und danach in Arbeiten von T. Miyashita et al. in, u.a., Proc. Eurodisplay, 149 (1993), J.Appl.Phys. 34, L177 (1995), SID 95 Digest, 797 (1995), C.-L. Kuo et al., SID 94 Digest, 927 (1994) und M. Suzuki, SID 96 Digest, 618 (1996), beschrieben wurde. Eine OCB-Zelle enthält eine Flüssigkristallzelle mit "bend"-Orientierung und ein Flüssigkristallmedium mit positivem Δs. Darüber hinaus enthalten die aus den oben genannten Dokumenten bekannten OCB-Anzeigen einen oder mehrere doppelbrechende optische Retardationsfilme, um unerwünschte Lichtdurchlässigkeit der "bend"-Zelle im dunklen Zustand zu vermeiden. OCB-Anzeigen besitzen gegenüber den üblichen Anzeigen, die auf verdrillten nematischen ("twisted nematic", TN) Zellen beruhen, mehrere Vorteile, wie zum Beispiel einen weiteren Blickwinkel und kürzere Schaltzeiten.

Die oben genannten Dokumente haben gezeigt, dass flüssigkristalline Phasen hohe Werte für die optische Anisotropie Δn und einen relativ hohen positiven Wert für die dielektrische Anisotropie Δε sowie vorzugsweise recht niedrige Werte für das Verhältnis der elastischen Konstanten K₃₃/K₁₁ und für die Viskosität aufweisen müssen, um für hochinformative Anzeigeelemente beruhend auf dem OCB-Effekt eingesetzt werden zu können. Für die technische Anwendung des OCB-Effekts in elektrooptischen Anzeigen werden FK-Phasen benötigt, die einer Vielzahl von Anforderungen genügen müssen. Besonders wichtig sind hier die chemische Beständigkeit gegenüber Feuchtigkeit, Luft und physikalischen Einflüssen wie Wärme, Strahlung im infraroten, sichtbaren und ultravioletten Bereich sowie elektrischen Gleich- und Wechselstromfeldern. Ferner wird von technisch verwendbaren FK-Phasen eine flüssigkristalline Mesophase in einem geeigneten Temperaturbereich, eine relativ hohe Doppelbrechung, eine positive dielektrische Anisotropie und eine niedrige Viskosität gefordert.

LCoS^{™} (Liquid Crystal on Silicon)-Anzeigen sind aus dem Stand der Technik bekannt und von Three-Five Systems Inc. (Tempe, Arizona, USA) erhältlich. LCoS^{™}-Mikroanzeigen sind reflektive Anzeigen, die typischerweise eine Flüssigkristallschicht mit verdrillter nematischer Struktur zwischen einer Rückwand aus Silizium und einem Deckglas enthalten. Die Rückwand aus Silizium ist eine Anordnung von Bildpunkten, die jeweils über eine spiegelbildliche Oberfläche verfügen, die gleichzeitig als elektrischer Leiter wirkt. Jeder Bildpunkt enthält einen feststehenden Spiegel, der von einer aktiven Flüssigkristallschicht mit verdrillter nematischer Orientierung bedeckt ist, die durch Anlegen einer Spannung auf homeotrope Orientierung umgeschaltet werden kann. LCoS^{™}-Mikroanzeigen sind klein mit einer Diagonalen von typischerweise weniger als 1,0 Zoll, ermöglichen jedoch hohe Auflösungen von ¼ VGA (78 Tausend Bildpunkte) bis UXGA+ (über 2 Millionen Bildpunkte).

Aufgrund der geringen Bildpunktgröße haben LCoS^{™}-Anzeigen auch eine sehr geringe Zelldicke, die typischerweise etwa 1 Mikrometer beträgt. Die flüssigkristallinen Phasen, die in diesen Anzeigen verwendet werden, müssen daher insbesondere hohe Werte für die optische Anisotropie Δn aufweisen, im Gegensatz zu herkömmlichen FK-Anzeigen des reflektiven Typs, die normalerweise FK-Phasen mit niedrigem Δn erfordern. OCB-Modus- und LCoS^{™}-Anzeigen können als Matrixanzeigen betrieben werden. Matrix-Flüssigkristallanzeigen (MFK-Anzeigen) sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei man zwei Typen unterscheiden kann:
1. MOS (Metal Oxide Semiconductor) oder andere Dioden auf Silizium-Wafer als Substrat.
2. Dünnfilm-Transistoren (TFT) auf einer Glasplatte als Substrat.

Bei Typ 1 wird als elektrooptischer Effekt üblicherweise die dynamische Streuung oder der Guest-Host-Effekt verwendet.

Die Verwendung von einkristallinem Silizium als Substratmaterial beschränkt die Displaygröße, da auch die modulartige Zusammensetzung verschiedener Teildisplays an den Stößen zu Problemen führt.

Bei dem aussichtsreicheren Typ 2, welcher bevorzugt ist, wird als elektrooptischer Effekt üblicherweise der TN-Effekt verwendet. Man unterscheidet zwei Technologien: TFT's aus Verbindungshalbleitern wie z.B. CdSe oder TFT's auf der Basis von polykristallinem oder amorphem Silizium. An letzterer Technologie wird weltweit mit großer Intensität gearbeitet.

Die TFT-Matrix ist auf der Innenseite der einen Glasplatte der Anzeige aufgebracht, während die andere Glasplatte auf der Innenseite die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, dass je ein Filterelement einem schaltbaren Bildelement gegenüber liegt.

Die TFT-Anzeigen arbeiten üblicherweise als TN-Zellen mit gekreuzten Polarisatoren in Transmission und sind von hinten beleuchtet.

Der Begriff MFK-Anzeigen umfasst hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen (z.B. Taschenfernseher) oder für hochinformative Displays für Rechneranwendungen (Laptop) und im Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris]. Mit abnehmendem Widerstand verschlechtert sich der Kontrast einer MFK-Anzeige und es kann das Problem der "after image elimination" auftreten. Da der spezifische Widerstand der Flüssigkristallmischung durch Wechselwirkung mit den inneren Oberflächen der Anzeige im allgemeinen über die Lebenszeit einer MFK-Anzeige abnimmt, ist ein hoher (Anfangs)-Widerstand sehr wichtig, um akzeptable Standzeiten zu erhalten. Insbesondere bei low-volt-Mischungen war es bisher nicht möglich, sehr hohe spezifische Widerstände zu realisieren. Weiterhin ist es wichtig, dass der spezifische Widerstand eine möglichst geringe Zunahme bei steigender Temperatur sowie nach Temperatur- und/oder UV-Belastung zeigt. Besonders nachteilig sind auch die Tieftemperatureigenschaften der Mischungen aus dem Stand der Technik. Gefordert wird, dass auch bei tiefen Temperaturen keine Kristallisation und/oder smektische Phasen auftreten und die Temperaturabhängigkeit der Viskosität möglichst gering ist. Die MFK-Anzeigen aus dem Stand der Technik genügen somit nicht den heutigen Anforderungen.

Neben Flüssigkristallanzeigen, die eine Hintergrundbeleuchtung verwenden, also transmissiv und gegebenenfalls transflektiv betrieben werden, sind besonders auch reflektive Flüssigkristallanzeigen interessant. Diese reflektiven Flüssigkristallanzeigen benutzen das Umgebungslicht zur Informationsdarstellung. Somit verbrauchen sie wesentlich weniger Energie als hintergrundbeleuchtete Flüssigkristallanzeigen mit entsprechender Größe und Auflösung. Da der TN-Effekt durch einen sehr guten Kontrast gekennzeichnet ist, sind derartige reflektive Anzeigen auch bei hellen Umgebungsverhältnissen noch gut abzulesen. Dies ist bereits von einfachen reflektiven TN-Anzeigen wie sie in z. B. Armbanduhren und Taschenrechnern verwendet werden, bekannt. Jedoch ist das Prinzip auch auf hochwertige, höher auflösende Aktiv-Matrix angesteuerte Anzeigen wie z. B. TFT-Displays anwendbar. Hier ist wie bereits bei den allgemeinen üblichen transmissiven TFT-TN-Anzeigen die Verwendung von Flüssigkristallen mit niedriger Doppelbrechung (Δn) nötig, um eine geringe optische Verzögerung (d · Δn) zu ereichen. Diese geringe optische Verzögerung führt zu einer meist akzeptablen geringen Blickwinkelabhängigkeit des Kontrastes (vgl. DE 30 22 818). Bei reflektiven Anzeigen ist die Verwendung von Flüssigkristallen mit kleiner Doppelbrechung noch wichtiger als bei transmissiven Anzeigen, da bei reflektiven Anzeigen die effektive Schichtdicke, die das Licht durchquert, ungefähr doppelt so groß ist wie bei transmissiven Anzeigen mit derselben Schichtdicke.

Es besteht somit immer noch ein großer Bedarf nach flüssigkristallinen Medien für MFK-, OCB-, IPS-, TN-, LCoS- oder STN-Anzeigen, die eine hohe UV-Stabilität, relativ hohe Δε-Werte, bei gleichzeitig großem Arbeitstemperaturbereich, kurzen Schaltzeiten auch bei tiefen Temperaturen und niedriger Schwellenspannung, die diese Nachteile nicht oder nur in geringerem Maße zeigen.

Bei TN-(Schadt-Helfrich)-Zellen sind Medien erwünscht, die folgende Vorteile in den Zellen ermöglichen:
- erweiterter nematischer Phasenbereich (insbesondere zu tiefen Temperaturen)
- lagerstabil, auch bei extrem tiefen Temperaturen
- Schaltbarkeit bei extrem tiefen Temperaturen (out-door-use, Automobil, Avionik)
- erhöhte Beständigkeit gegenüber UV-Strahlung (längere Lebensdauer)
- höhere optische Anisotropien für schnellere Schaltzeiten aufgrund dünnerer Zelldicken (d · Δn)

Mit den aus dem Stand der Technik zur Verfügung stehenden Medien ist es nicht möglich, diese Vorteile unter gleichzeitigem Erhalt der übrigen Parameter zu realisieren.

Bei höher verdrillten Zellen (STN) sind Medien erwünscht, die eine höhere Multiplexierbarkeit und/oder kleinere Schwellenspannung und/oder breitere nematische Phasenbereiche (insbesondere bei tiefen Temperaturen) ermöglichen. Hierzu ist eine weitere Ausdehnung des zur Verfügung stehenden Parameterraumes (Klärpunkt, Übergang smektisch-nematisch bzw. Schmelzpunkt, Viskosität, dielektrische Größen, elastische Größen) dringend erwünscht.

Der Erfindung liegt die Aufgabe zugrunde Medien, insbesondere für derartige MFK-, OCB-, IPS-, LCoS-, TN- oder STN-Anzeigen, bereitzustellen, die die oben angegebenen Nachteile nicht oder nur in geringerem Maße, und vorzugsweise gleichzeitig relativ hohe Klärpunkte, niedrige Schwellen und relativ kleine Rotationsviskositäten γ₁ aufweisen. Weiterhin sollten die Mischungen sich durch eine hohe UV-Stabilität auszeichnen.

Es wurde nun gefunden, dass diese Aufgabe gelöst werden kann, wenn man in Anzeigen erfindungsgemäße Medien verwendet. Die erfindungsgemäßen Medien zeichnen sich durch ihre hohe UV-Stabilität aus. Gleichzeitig besitzen die Medien sehr niedrige Schwellenspannungen und relativ kleine Rotationsviskositäten γ₁.

Gegenstand der Erfindung ist somit ein flüssigkristallines Medium auf der Basis eine Gemisches von polaren Verbindungen, dadurch gekennzeichnet, dass es eine, zwei oder mehr Verbindungen der Formeln I-1 bis I-10, 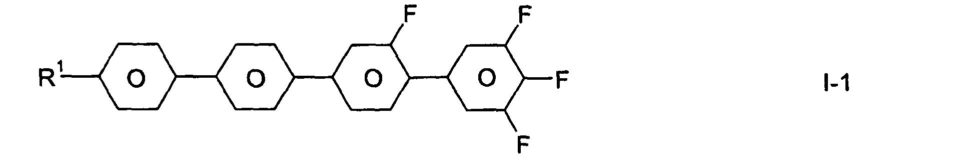 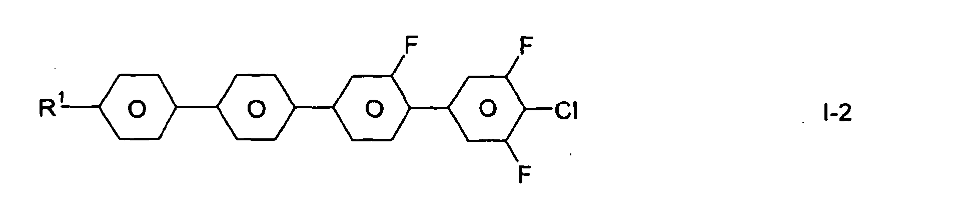 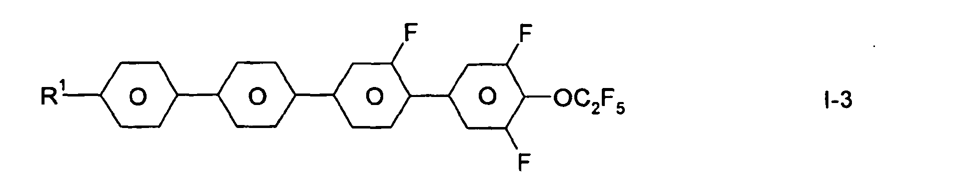 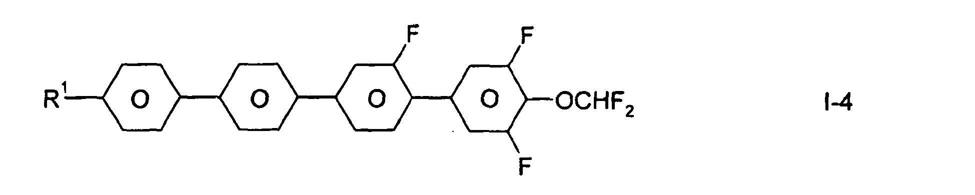 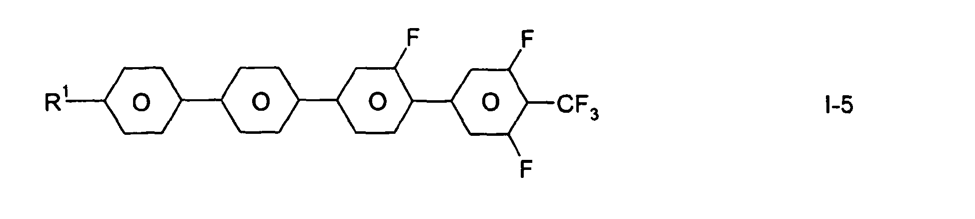 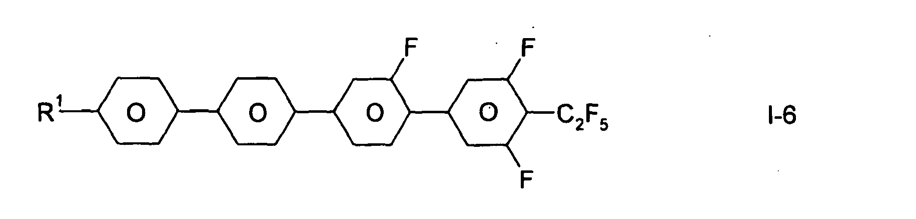 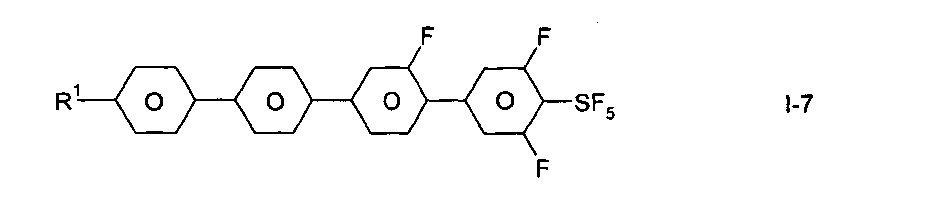 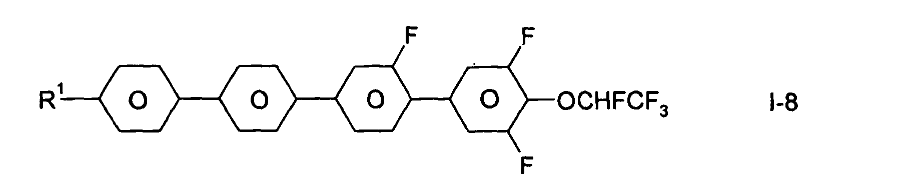 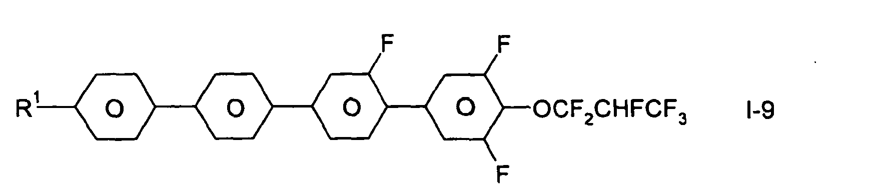 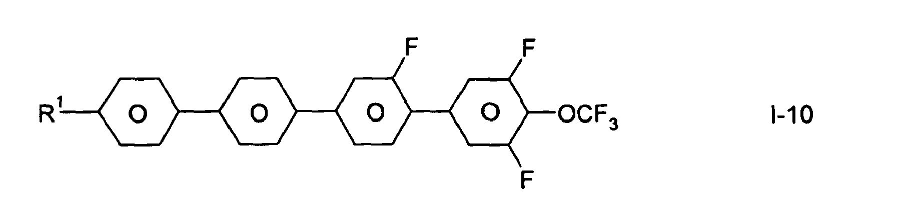 worin R¹ einen halogenierten oder unsubstituierten Alkyl- oder Alkoxyrest mit 1 bis 15 C-Atomen, wobei in diesen Resten auch eine oder mehrere CH₂-Gruppen jeweils unabhängig voneinander durch 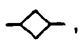 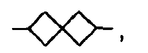
- C≡C-, -CH=CH-, -O-, -CF₂O- -OCF₂-, -CO-O- oder
- O-CO- so ersetzt sein können, dass O-Atome nicht direkt miteinander verknüpft sind,
bedeutet,
und
eine oder mehrere Verbindungen der Formel I* 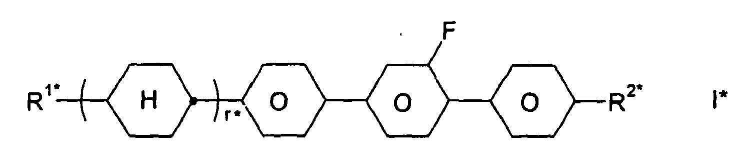 worin
R^{1*} und R^{2*} jeweils unabhängig voneinander Alkyl, Alkoxy, Oxaalkyl, Fluoralkyl, Alkenyloxy oder Alkenyl mit jeweils bis zu 9 C-Atomen, und
- r*: 0 oder 1.
bedeuten,
enthält.

Überraschenderweise wurde gefunden, dass flüssigkristalline Mischungen enthaltend Verbindungen der Formel I hohe Klärpunkte und relativ niedrige Schwellen aufweisen.

Im Stand der Technik werden fluorierte Quarterphenyle z. B. in der EP 0 949 231 A1, EP 1 346 995 A1, EP 1 352 944 A1, WO 89/02425 A1, DE 101 28 077 A1, U.S. 6,669,998 B2, U.S. 6,565,933 B2, U.S. 6,596,350 A2, WO 89/02884, WO 90/01056, WO 91/03450, EP 0 439 089 B1, DE 44 45 224, WO 98/23564, EP 1 302 523 A1, EP 1 346 995 beschrieben. Die erfindungsgemäßen Verbindungen werden aber nicht explizit genannt. Aus der WO 2004/035 710 A1 sind Verbindungen der Formel 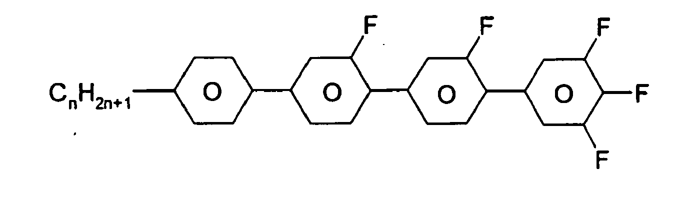 bekannt.

EP 949 231 A1 und WO 2004/035710 A1 offenbaren eine Verbindung der Formel I* (vgl. unten).

Die Verbindungen der Formeln I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren. Überraschenderweise sind die erfindungsgemäßen Vierkerner sehr gut löslich. So lassen sich erfindungsgemäße Mischungen herstellen, die 0,01-30,0 Gew.% bezogen auf die Mischung an Verbindungen der Formel I enthaltend.

Die Verbindungen der Formeln I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Falls R¹ in Formel I einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradedoxy.

Oxaalkyl bedeutet vorzugsweise geradkettiges 2-Oxapropyl (= Methoxymethyl), 2-(= Ethoxymethyl) oder 3-Oxabutyl (= 2-Methoxyethyl), 2-, 3-oder 4-Oxapentyl, 2-, 3-, 4- oder 5-Oxahexyl, 2-, 3-, 4-, 5- oder 6-Oxaheptyl, 2-, 3-, 4-, 5-, 6-, oder 7-Oxaoctyl, 2-, 3-, 4-, 5-, 6-, 7-oder 8-Oxanonyl, 2-, 3-, 4-, 5-, 6-, 7-, 8- oder 9-Oxadexyl.

Falls R¹ einen Alkylrest bedeutet, in dem eine CH₂-Gruppe durch -CH=CH-ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R¹ einen Alkylrest bedeutet, in dem eine CH₂-Gruppe durch -O- und eine durch -CO- ersetzt ist, so sind diese bevorzugt benachbart. Somit beinhalten diese eine Acyloxygruppe -CO-O- oder eine Oxycarbonylgruppe -O-CO-. Vorzugsweise sind diese geradkettig und haben 2 bis 6 C-Atome. Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 2-Acetyloxypropyl, 3-Propionyl-oxypropyl, 4-Acetyl-oxybutyl, Methoxycarbonyl, Ethoxycarbonyl, Propoxycarbonyl, Butoxycarbonyl, Pentoxycarbonyl, Methoxycarbonylmethyl, Ethoxycarbonylmethly, Propoxycarbonylmethyl, Butoxycarbonylmethyl, 2-(Methoxycarbonyl)ethyl, 2-(Ethoxycarbonyl)ethyl, 2-(Propoxycarbonyl)-ethyl, 3-(Methoxycarbonyl)-propyl, 3-(Ethoxy-carbonyl)-propyl oder 4-(Methoxycarbonyl)-butyl.

Falls R¹ einen Alkylrest bedeutet, in dem eine CH₂-Gruppe durch unsubstituiertes oder substituiertes -CH=CH- und eine benachbarte CH₂-Gruppe durch CO oder CO-O oder O-CO ersetzt ist, so kann dieser geradkettig oder verzweigt sein. Vorzugsweise ist er geradkettig und hat 4 bis 12 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R¹ einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen mit verzweigten Flügelgruppen R¹ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien. Verzweigte Gruppen dieser Art enthalten in der Regel nicht mehr als eine Kettenverzweigung. Bevorzugte verzweigte Reste R¹ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R¹ einen Alkylrest darstellt, in dem zwei oder mehr CH₂-Gruppen durch -O- und/oder -CO-O- ersetzt sind, so kann dieser geradkettig oder verzweigt sind. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxy-carbonyl)-methyl, 2,2-Bis-(methoxycarbonyl)-ethyl, 3,3-Bis-(methoxy-carbonyl)-propyl, 4,4-Bis-(methoxycarbonyl)-butyl, 5,5-Bis-(methoxy-carbonyl)-pentyl, 6,6-Bis-(methoxycarbonyl)-hexyl, 7,7-Bis-(methoxy-carbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Die Verbindungen der Formeln I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen. Die Verbindungen der Formel I können z. B. wie folgt hergestellt werden.

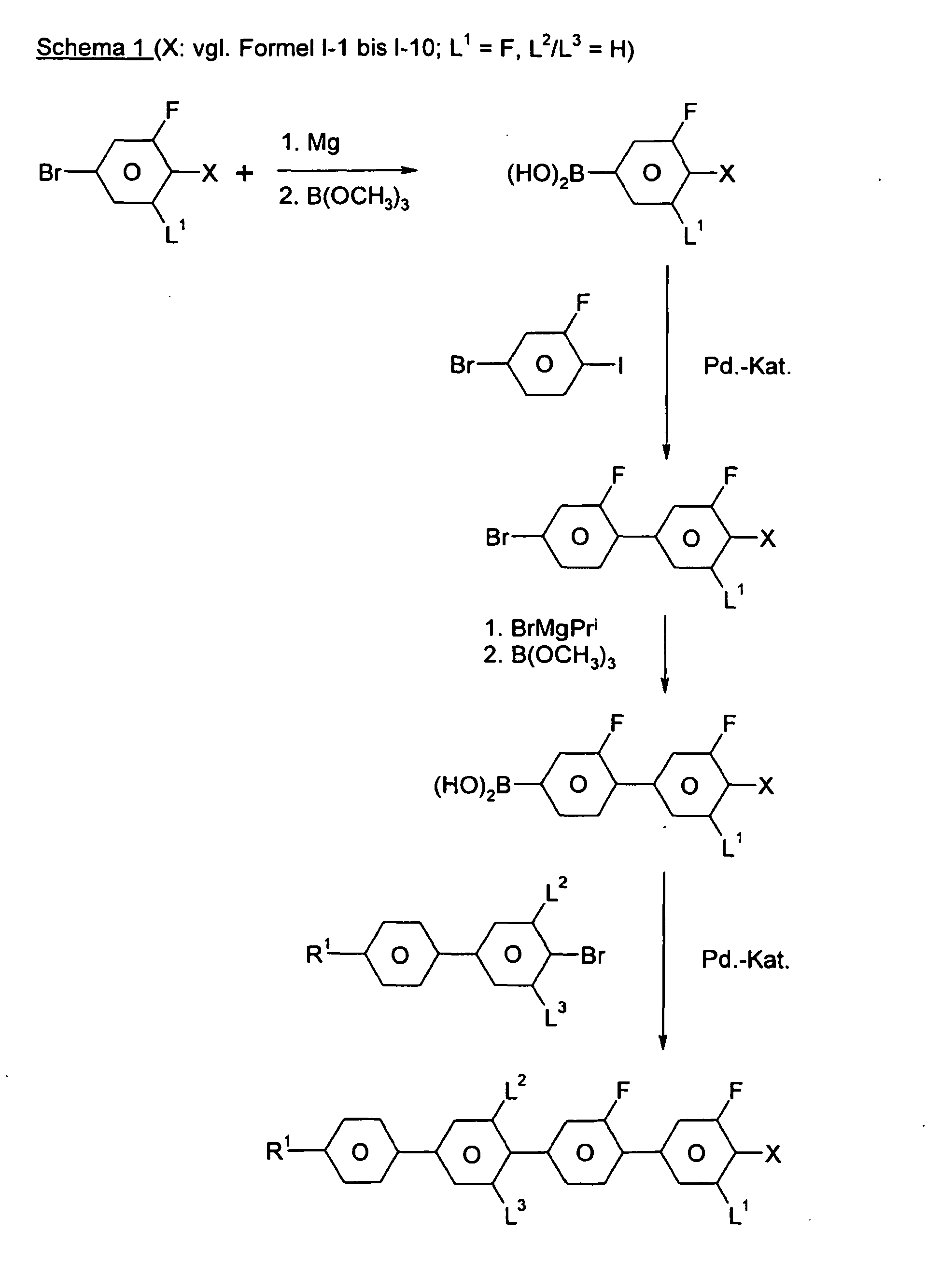

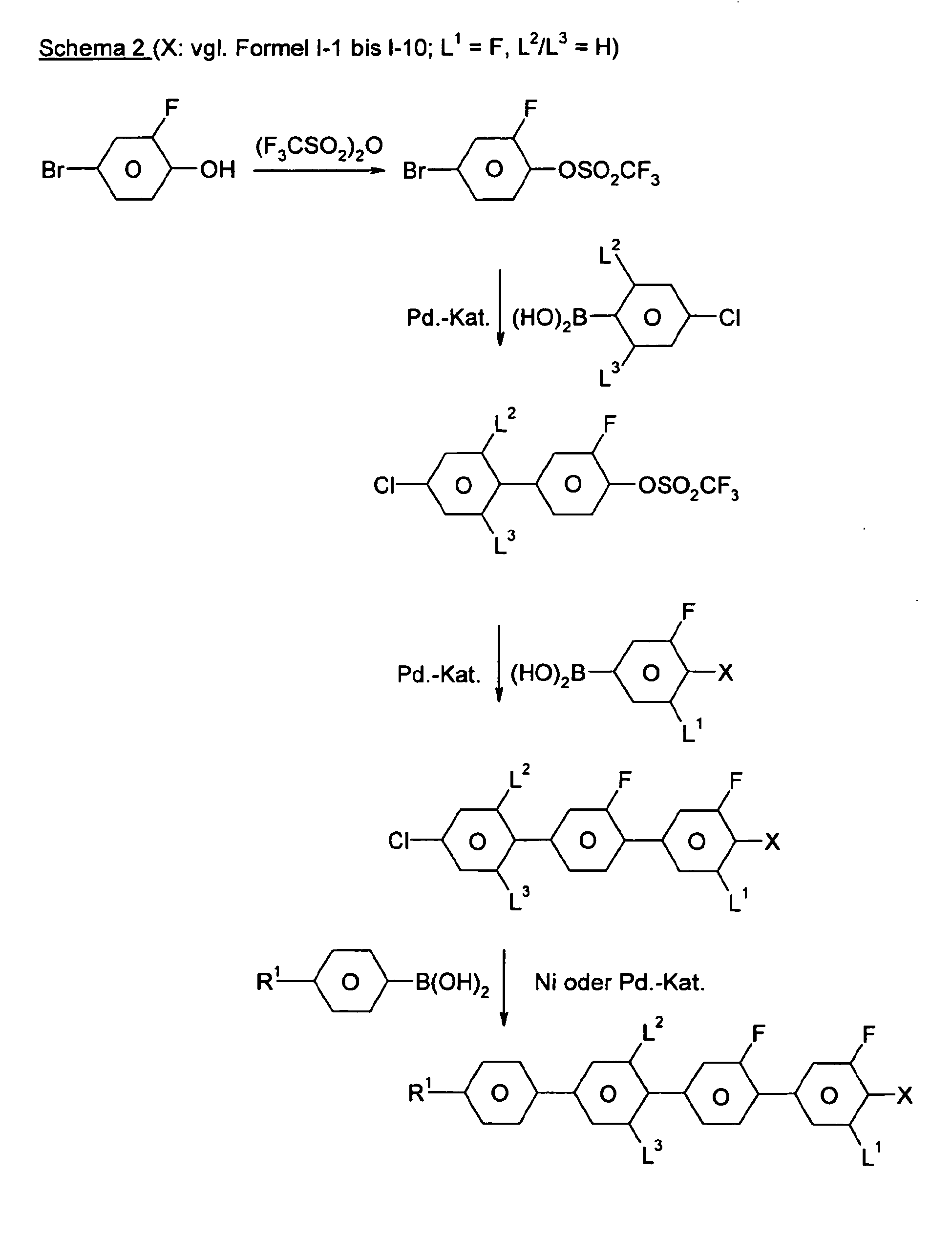

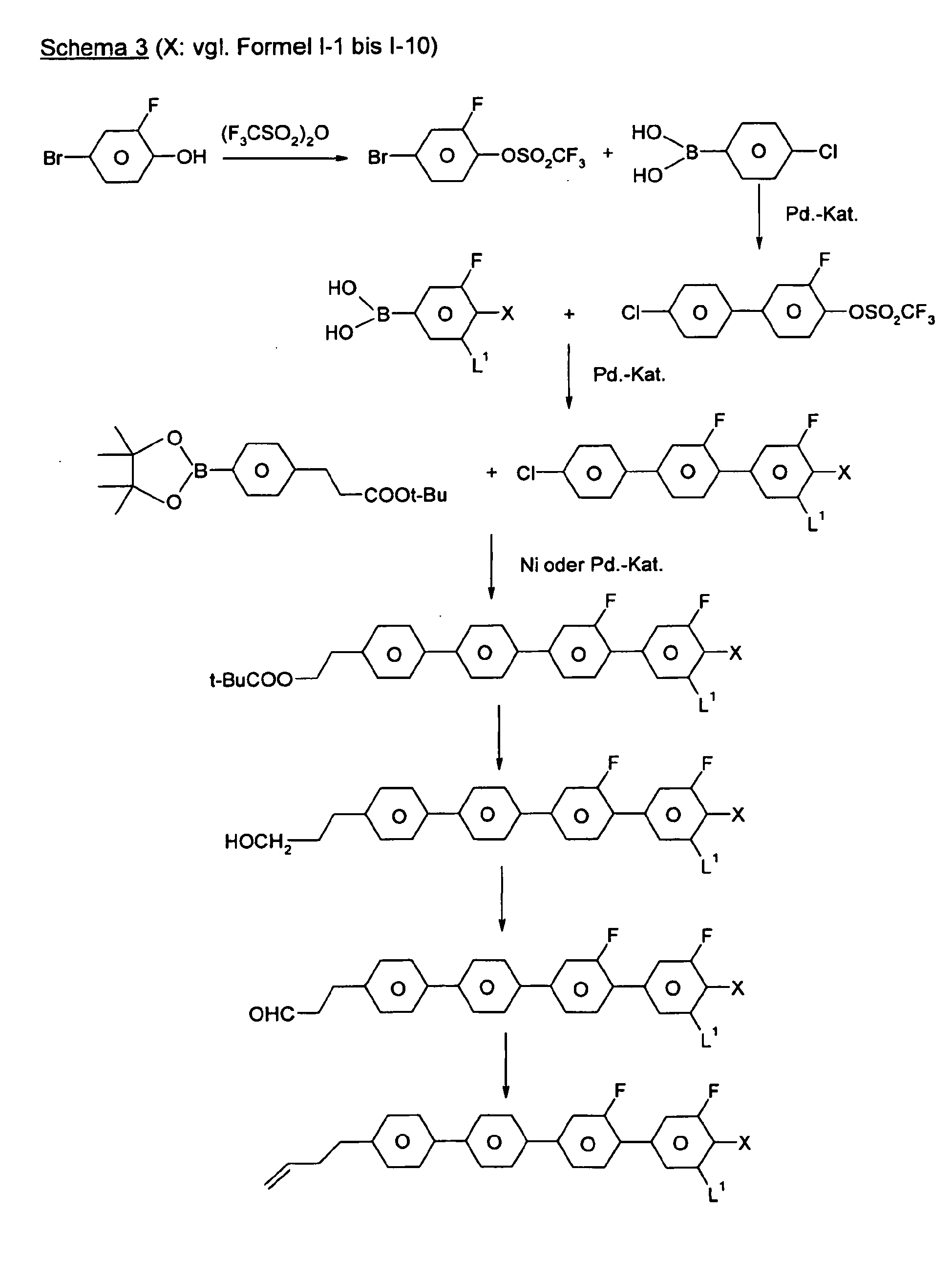

Gegenstand der Erfindung sind auch elektrooptische Anzeigen, insbesondere MFK-Anzeigen, ferner STN-Anzeigen, mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit sehr hoher optischer Anisotropie und hohem spezifischem Widerstand, die derartige Medien enthalten sowie die Verwendung dieser Medien für elektrooptische Zwecke.

Die erfindungsgemäßen Mischungen sind insbesondere für schnell schaltende Monitore, TV-Monitor-Kombigeräte und high Δn-TFT-Anwendungen, wie z. B. Projektionsfernseher, LCoS und OCB, geeignet.

Die erfindungsgemäßen Flüssigkristallmischungen ermöglichen eine bedeutende Erweiterung des zur Verfügung stehenden Parameterraumes. Die erzielbaren Kombinationen aus Klärpunkt, Viskosität bei tiefer Temperatur, thermischer und UV-Stabilität und hoher optischer Anisotropie übertreffen bei weitem bisherige Materialien aus dem Stand der Technik.

Die erfindungsgemäßen Flüssigkristallmischungen ermöglichen es bei Beibehaltung der nematischen Phase bis -20°C und bevorzugt bis -30 °C, besonders bevorzugt bis -40 °C, Klärpunkt oberhalb 60°C, vorzugsweise oberhalb 70 °C, besonders bevorzugt oberhalb 80°C, gleichzeitig dielektrische Anisotropiewerte Δε ≥ 4, vorzugsweise ≥ 5 und einen hohen Wert für den spezifischen Widerstand zu erreichen, wodurch hervorragende STN- und MFK-Anzeigen erzielt werden können. Insbesondere sind die Mischungen durch kleine Operationsspannungen gekennzeichnet. Die TN-Schwellen liegen unterhalb 2,5 V, vorzugsweise unterhalb 2,0 V, besonders bevorzugt < 1,8 V.

Es versteht sich, dass durch geeignete Wahl der Komponenten der erfindungsgemäßen Mischungen auch höhere Klärpunkte (z.B. oberhalb 110°C) bei höheren Schwellenspannungen oder niedrigere Klärpunkte bei niedrigeren Schwellenspannungen unter Erhalt der anderen vorteilhaften Eigenschaften realisiert werden können. Ebenso können bei entsprechend wenig erhöhten Viskositäten Mischungen mit größerem Δε und somit geringen Schwellen erhalten werden. Die erfindungsgemäßen MFK-Anzeigen arbeiten vorzugsweise im ersten Transmissionsminimum nach Gooch und Tarry [C.H. Gooch und H.A. Tarry, Electron. Lett. 10, 2-4, 1974; C.H. Gooch und H.A. Tarry, Appl. Phys., Vol. 8, 1575-1584, 1975], wobei hier neben besonders günstigen elektrooptischen Eigenschaften, wie z.B. hohe Steilheit der Kennlinie und geringe Winkelabhängigkeit des Kontrastes (DE-PS 30 22 818) bei gleicher Schwellenspannung wie in einer analogen Anzeige im zweiten Minimum, eine kleinere dielektrische Anisotropie ausreichend ist. Hierdurch lassen sich unter Verwendung der erfindungsgemäßen Mischungen im ersten Minimum deutlich höhere spezifische Widerstände verwirklichen als bei Mischungen mit Cyanverbindungen. Der Fachmann kann durch geeignete Wahl der einzelnen Komponenten und deren Gewichtsanteilen mit einfachen Routinemethoden die für eine vorgegebene Schichtdicke der MFK-Anzeige erforderliche Doppelbrechung einstellen.

Die Fließviskosität ν₂₀ bei 20°C ist vorzugsweise < 150 mm²·s⁻¹, besonders bevorzugt < 120 mm²·s⁻¹ und insbesondere < 80 mm²·s⁻¹. Die Rotationsviskosität γ₁ der erfindungsgemäßen Mischungen bei 20°C ist vorzugsweise < 200 mPa·s, besonders bevorzugt < 180 mPa·s. Der nematische Phasenbereich ist vorzugsweise mindestens 90°, insbesondere mindestens 100°. Vorzugsweise erstreckt sich dieser Bereich mindestens von -20° bis +80°.

Bei Flüssigkristallanzeigen ist eine kleine Schaltzeit erwünscht. Dies gilt besonders für Anzeigen die Videowiedergabe-fähig sind. Für derartige Anzeigen werden Schaltzeiten (Summe: tₒₙ + t_{off}) von maximal 25 ms benötigt. Die Obergrenze der Schaltzeit wird durch die Bildwiederholfrequenz bestimmt. Neben der Rotationsviskosität γ₁ beeinflußt auch der Tiltwinkel die Schaltzeit.

Messungen des "Voltage Holding-ratio" (HR) [S. Matsumoto et al., Liquid Crystals 5, 1320 (1989); K. Niwa et al., Proc. SID Conference, San Francisco, June 1984, p. 304 (1984); G. Weber et al., Liquid Crystals 5, 1381 (1989)] haben ergeben, dass erfindungsgemäße Mischungen enthaltend Verbindungen der Formel I eine deutlich kleinere Abnahme des HR mit steigender Temperatur aufweisen als analoge Mischungen enthaltend anstelle den Verbindungen der Formel I Cyanophenylcyclohexane der Formel 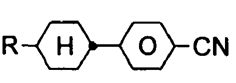 oder Ester der
Formel

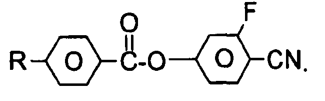

Die UV-Stabilität der erfindungsgemäßen Mischungen ist erheblich besser, d.h. sie zeigen eine deutlich kleinere Abnahme des HR unter UV-Belastung. Bereits geringe Konzentrationen (< 10 Gew.%) der Verbindungen der Formel I in den Mischungen erhöhen die HR gegenüber Mischungen aus dem Stand der Technik um 6 % und mehr.

Besonders bevorzugte Verbindungen der Formel I sind Verbindungen der Formeln I-1,I-4,I-7 und I-10: 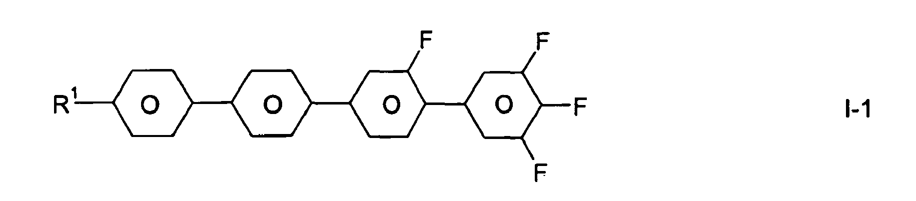 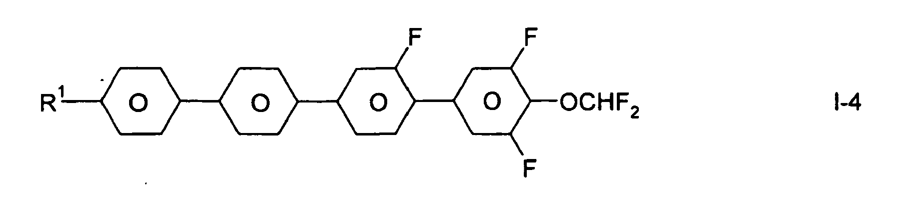 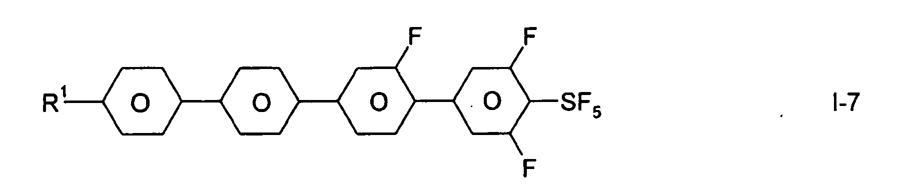 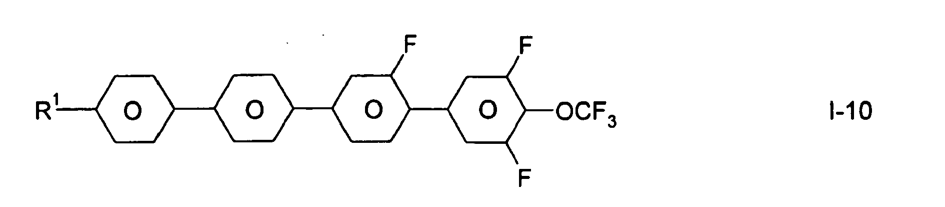 worin R¹ die in Formel angegebenen Bedeutung hat. Vorzugsweise bedeutet R¹ Alkyl, ferner Alkenyl.

Von diesen bevorzugten Verbindungen sind besonders bevorzugt solche der Formeln I-1 und 1-10.

R¹ bedeutet in den Unterformeln I-1 bis I-10 vorzugsweise C₂H₅, n-C₃H₇, n-C₅H₁₁, ferner CH₃, n-C₄H₉, n-C₆H₁₃, n-C₇H₁₅, CH₂=CH, CH₃CH=CH, CH₂=CHCH₂CH₂ oder CH₃CH=CHCH₂CH₂. Ganz besonders bevorzugt bedeutet R¹ n-C₃H₇.

Bevorzugte Ausführungsformen sind im folgenden angegeben:
- Das Medium enthält vorzugsweise mindestens eine der folgenden Verbindungen 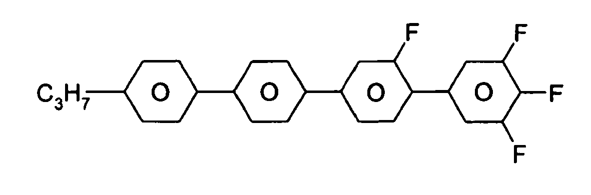 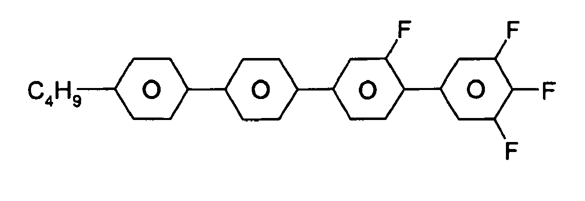 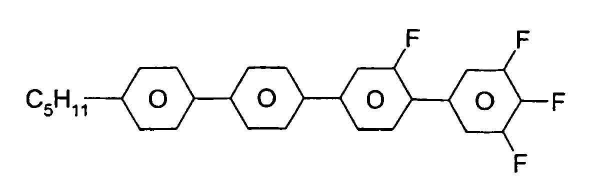 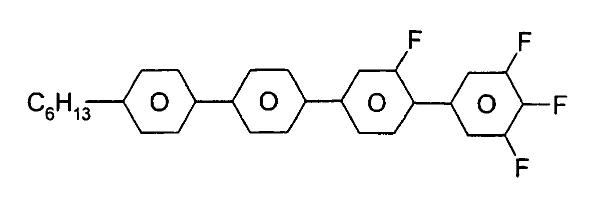 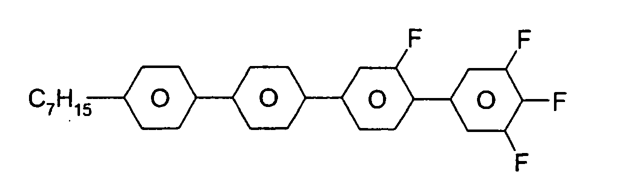 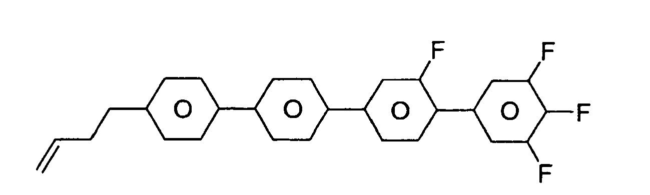 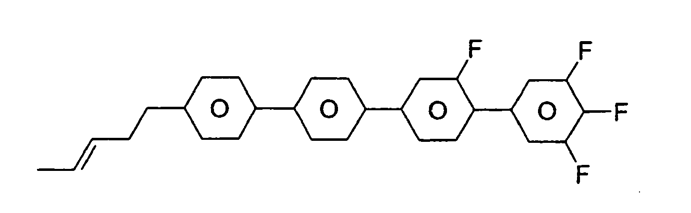 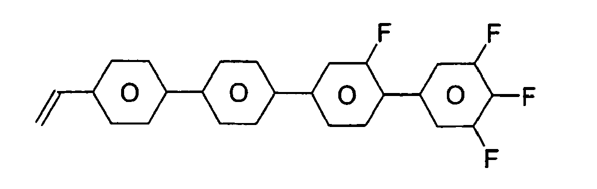 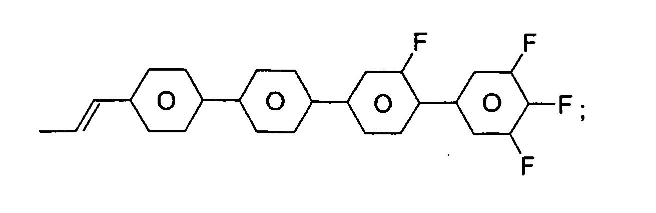
- Das Medium enthält vorzugsweise eine oder mehrere Verbindungen der Formeln I*-1 bis I*-8, 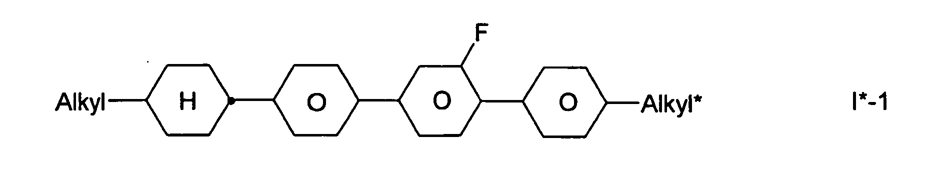 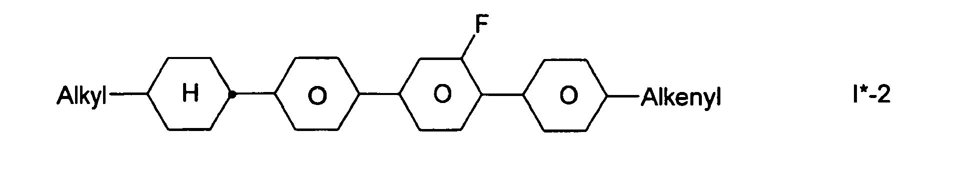 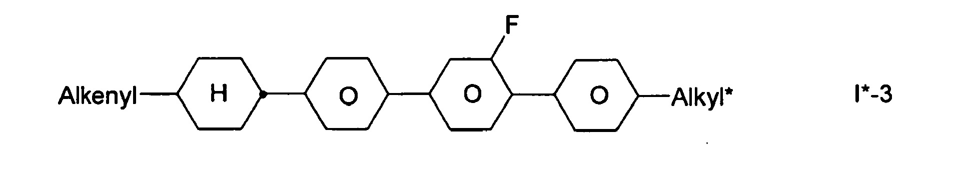 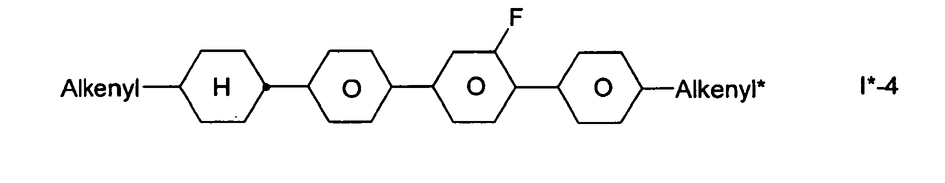 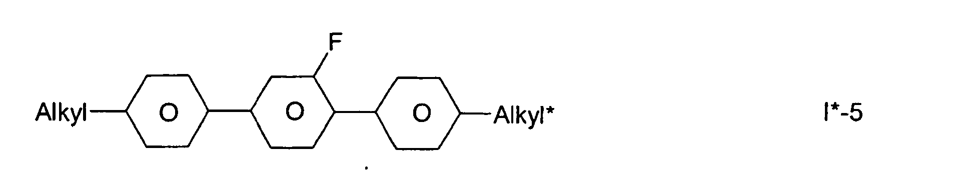 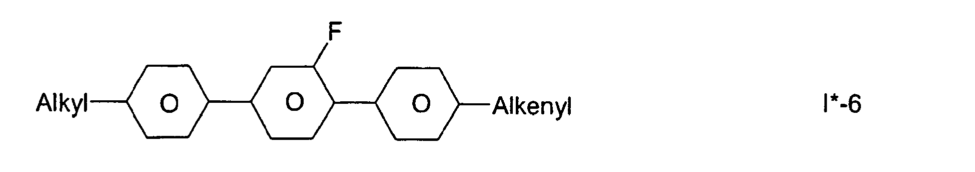 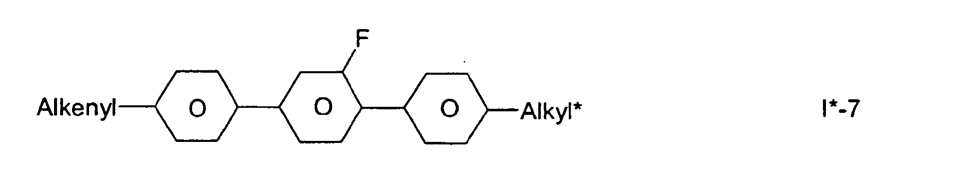 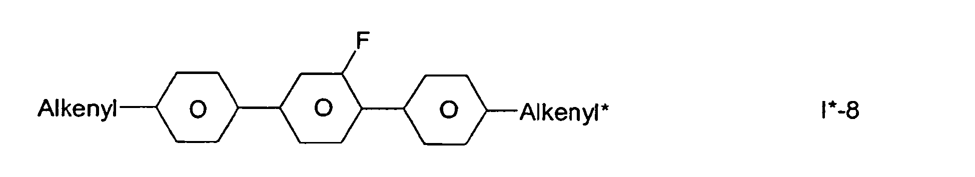
worin
- Alkyl und Alkyl*: jeweils unabhängig voneinander geradkettiges Alkyl mit 1-6 C-Atomen, und
- Alkenyl und Alkenyl*: jeweils unabhängig voneinander geradkettiges Alkenyl mit 2-6 C-Atomen,
bedeuten.

Von den Verbindungen I*-1 bis I*-8 sind insbesondere die Verbindungen I*-5 bis I*-7 bevorzugt. Ganz besonders bevorzugt ist die Verbindung I*-5.
- Das Medium enthält eins, zwei, drei oder vier Verbindungen der Formel I*. Die Konzentration der Verbindung(en) der Fomel I* in der erfindungsgemäßen Mischung beträgt 2-50 Gew.%, vorzugsweise 2-40 Gew.%, insbesondere 5-40 Gew.%.
- Das Medium enthält zusätzlich eine oder mehrere Verbindungen ausgewählt aus der Gruppe bestehend aus den allgemeinen Formeln II bis VI: 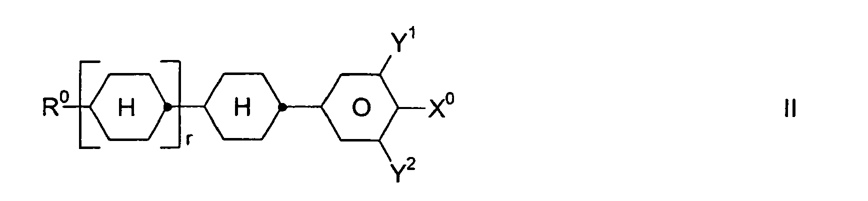 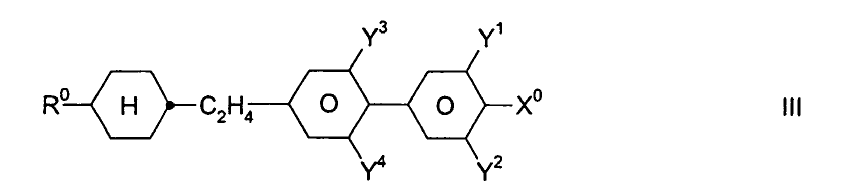 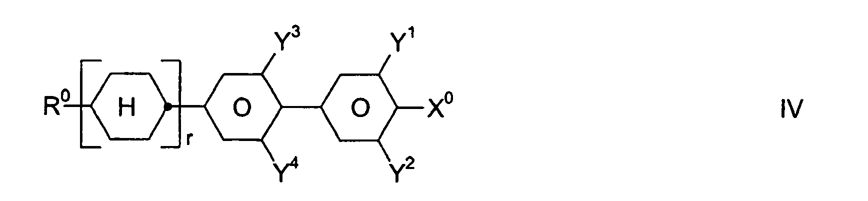 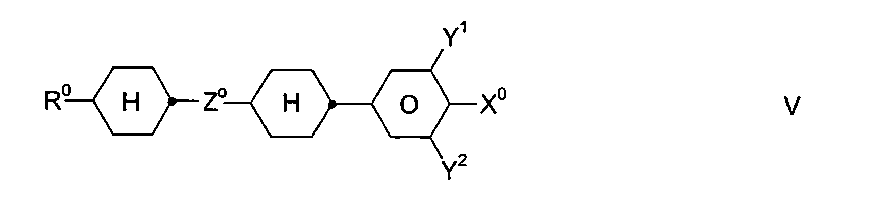 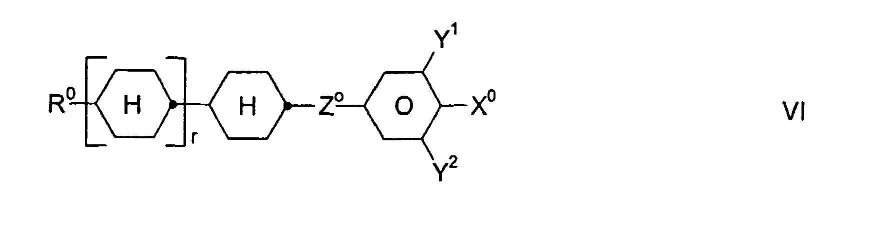 worin die einzelnen Reste die folgenden Bedeutungen haben:
   - R°: Alkyl, Alkoxy, Oxaalkyl, Fluoralkyl oder Alkenyl mit jeweils bis zu 9 C-Atomen,
   - X°: F, Cl, halogeniertes Alkyl, halogeniertes Alkenyl, halogeniertes Oxaalkyl, halogeniertes Alkenyloxy oder halogeniertes Alkoxy mit bis zu 6 C-Atomen,
   - Z°: -C₂F₄-, -CF=CF, -C₂H₄-, -CH=CH-, -(CH₂)₄-, -OCH₂-, -CH₂O-, -CF₂O- oder -OCF₂-,
   - Y¹ bis Y⁴: jeweils unabhängig voneinander H oder F,
   - r: 0 oder 1.

Die Verbindung der Formel IV ist vorzugsweise 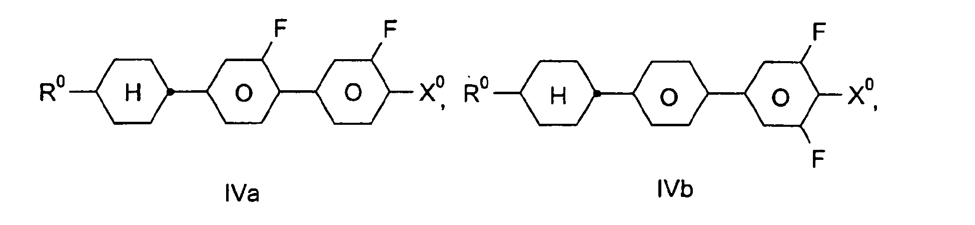 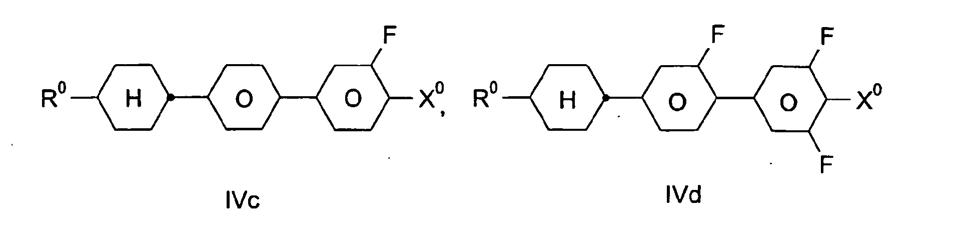 oder

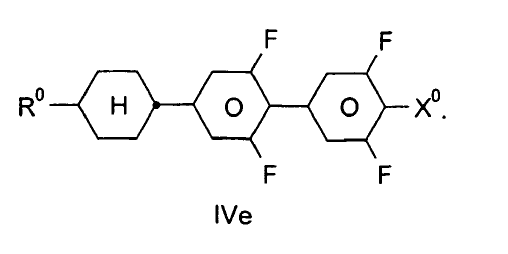

Das Medium enthält zusätzlich eine oder mehrere Verbindungen ausgewählt aus der Gruppe bestehend aus den allgemeinen Formeln VII bis XII: 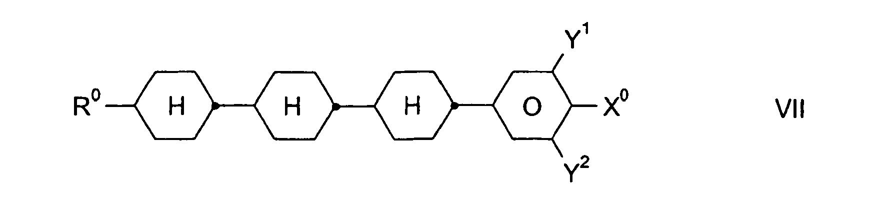 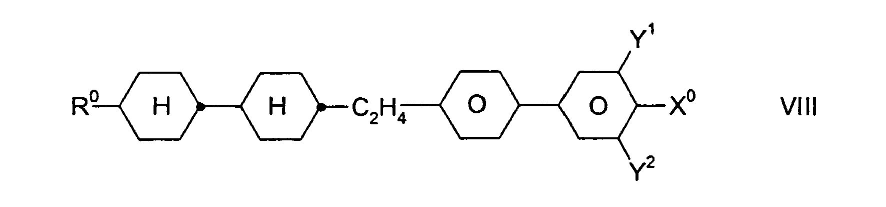 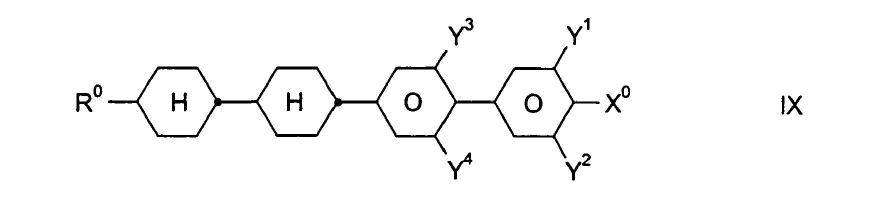 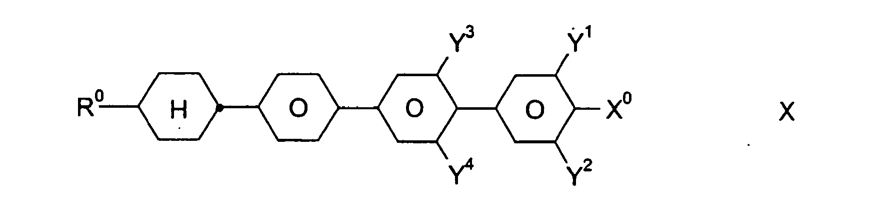 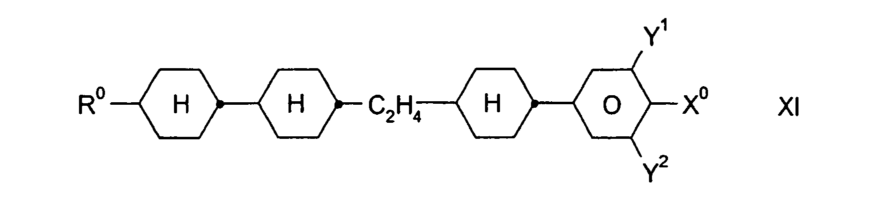 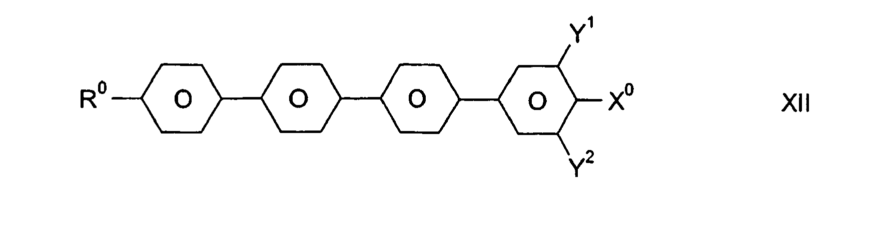 worin R°, X° und Y¹⁻⁴ jeweils unabhängig voneinander eine der in Anspruch 8 angegebenen Bedeutungen haben. X° ist vorzugsweise F, Cl, CF₃, OCF₃ oder OCHF₂. R° bedeutet vorzugsweise Alkyl, Alkoxy, Oxaalkyl, Fluoralkyl oder Alkenyl mit jeweils bis zu 6 C-Atomen.

Das Medium enthält zusätzlich eine oder mehrere Verbindungen der Formeln E-a bis E-d, 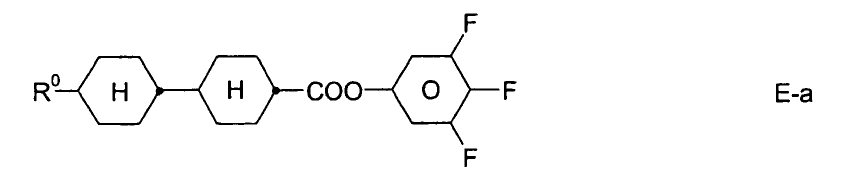 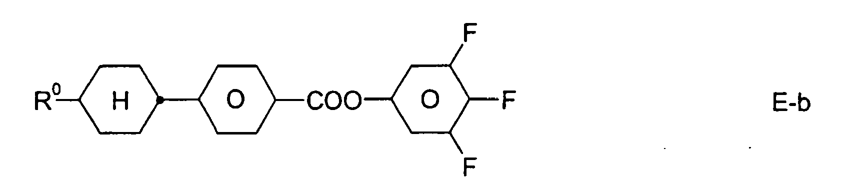 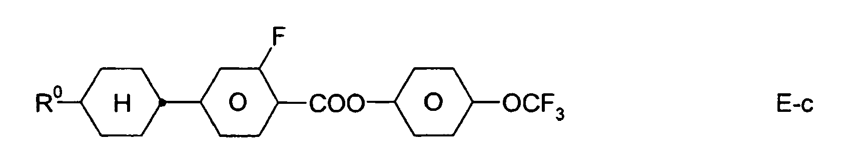 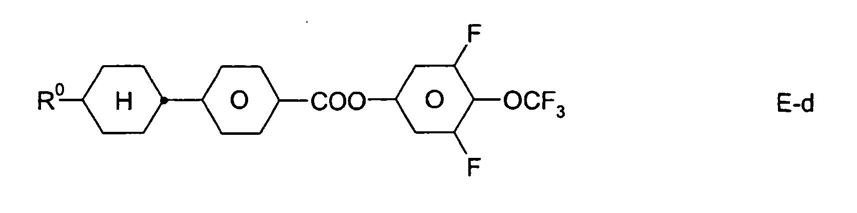 worin R° die in Anspruch 8 angegebenen Bedeutungen hat.
- Der Anteil der Verbindungen der Formeln E-a bis E-d ist vorzugsweise 10-30 Gew.%, insbesondere 15-25 Gew.%.
- Der Anteil an Verbindungen der Formeln I und I* zusammen beträgt im Gesamtgemisch mindestens 5 Gew.%, vorzugsweise ≥ 10 Gew.% und insbesondere ≥ 15 Gew.%.
- Der Anteil an Verbindungen der Formel I beträgt im Gesamtgemisch 0,01 bis 30 Gew.%, besonders bevorzugt 0,5 bis 20 Gew.%.
- Der Anteil an Verbindungen der Formeln II bis VI im Gesamtgemisch beträgt 10 bis 80 Gew.%; 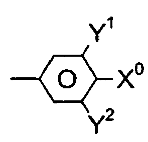 ist vorzugsweise 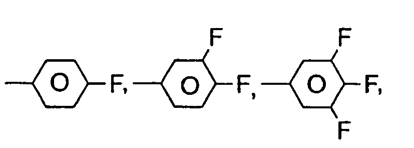 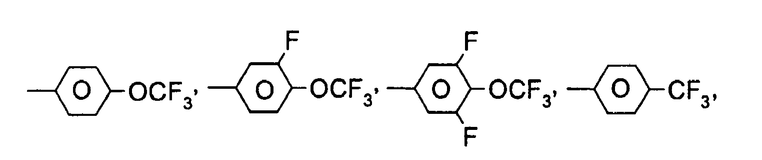 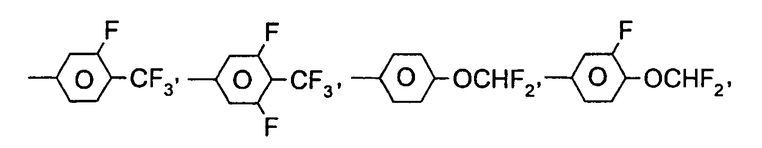 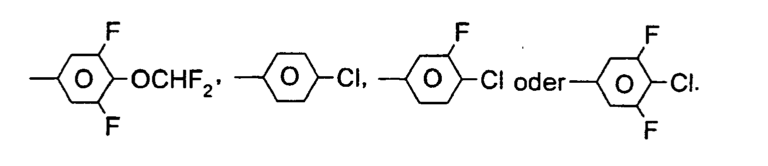
- Das Medium enthält Verbindungen der Formeln II, III, IV, V und/oder VI.
- R° in den Verbindungen der Formeln II bis XIX ist vorzugsweise geradkettiges Alkyl oder Alkenyl mit 2 bis 7 C-Atomen.
- Das Medium besteht im wesentlichen aus Verbindungen der Formeln I, I* und XIII bis XIX, wobei im wesentlichen ≥ 50 Gew.% bedeutet.
- Das Medium enthält weitere Verbindungen, vorzugsweise ausgewählt aus der folgenden Gruppe bestehend aus den allgemeinen Formeln XIII bis XIX: 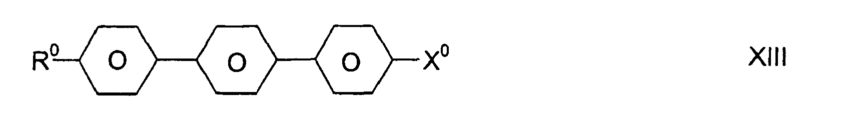 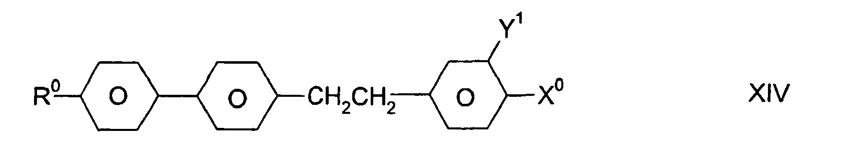 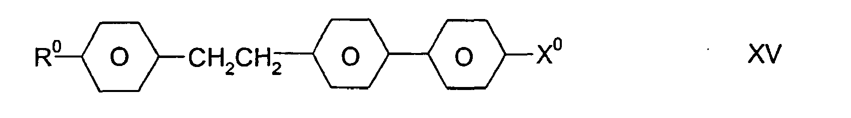 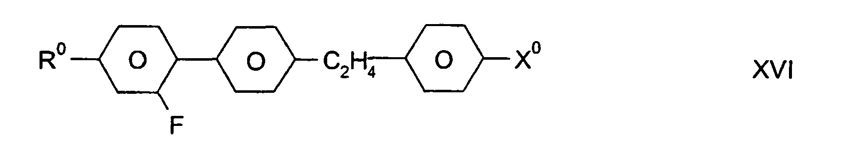 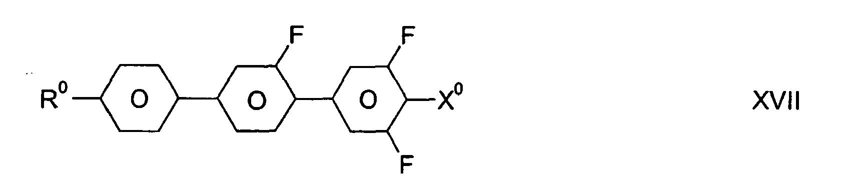 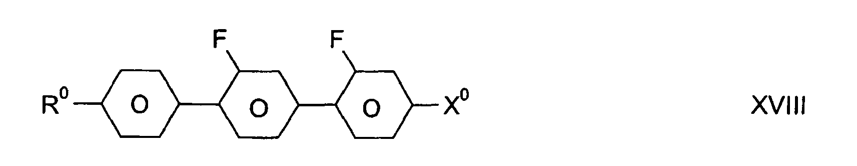 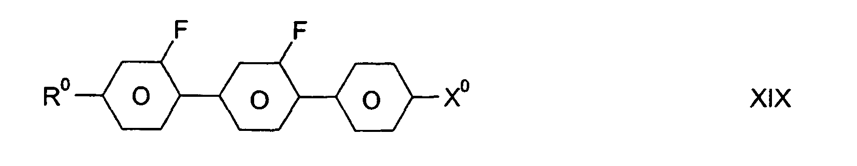 worin R° und X° die oben angegebenen Bedeutungen haben. X° bedeutet vorzugsweise F oder Cl. Die Konzentration der Verbindungen der Formeln XIII bis XIX beträgt vorzugsweise 0,05-30 Gew.%, insbesondere 1-25 Gew.%.
- Das Medium enthält vorzugsweise 5-35 Gew.% der Verbindung IVa.
- Das Medium enthält vorzugsweise eine, zwei oder drei Verbindungen der Formel IVa, worin X° F oder OCF₃ bedeutet.
- Das Medium enthält vorzugsweise eine oder mehrere Verbindungen der Formeln IIa bis IIg, 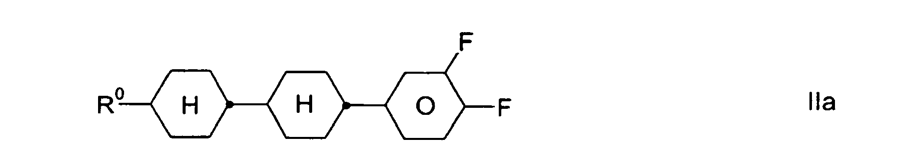 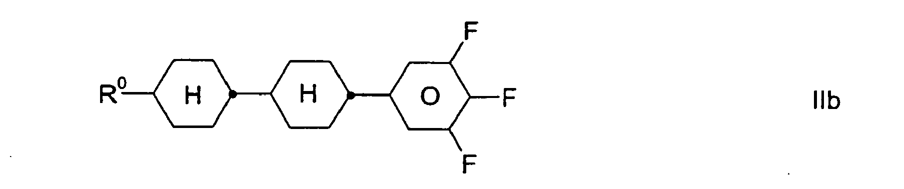 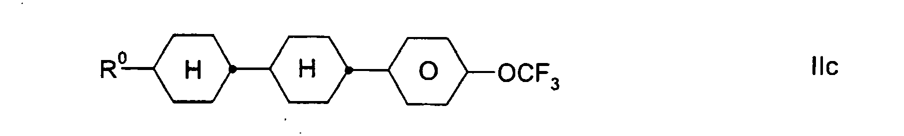 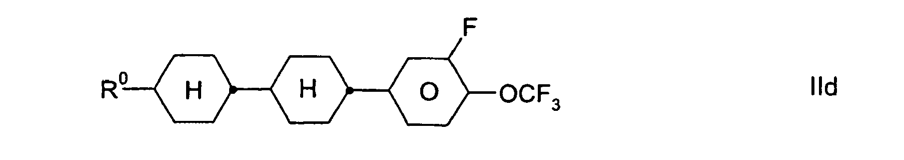 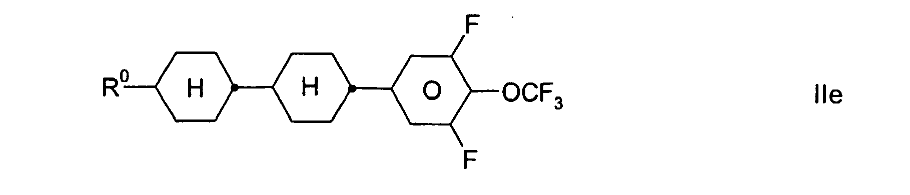 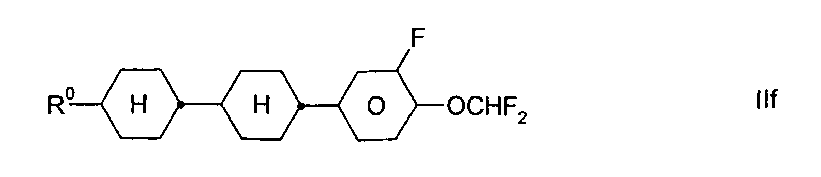 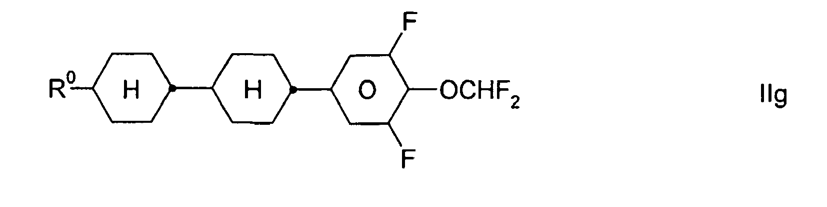 worin R° die oben angegebenen Bedeutungen hat. In den Verbindungen der Formeln IIa-IIg bedeutet R° vorzugsweise Methyl, Ethyl, n-Propyl, n-Butyl und n-Pentyl.
- Das Gewichtsverhältnis I bzw. I + I* : (II + III + IV + V + VI) ist vorzugsweise 1 : 10 bis 10 : 1.
- Das Medium besteht im wesentlichen aus Verbindungen ausgewählt aus der Gruppe bestehend aus den allgemeinen Formeln I bis XIX.
- Der Anteil der Verbindungen der Formel IVb, IVc und/oder IVd, worin X° Fluor und R° CH₃, C₂H₅, n-C₃H₇, n-C₄H₉ oder n-C₅H₁₁ bedeutet, beträgt im Gesamtgemisch 2 bis 25 Gew.%, insbesondere 2 bis 20 Gew.%.
- Das Medium enthält vorzugsweise ein, zwei oder mehr, vorzugsweise ein, zwei oder mehr Dioxan-Verbindungen der Formeln D-1 bis D-4, 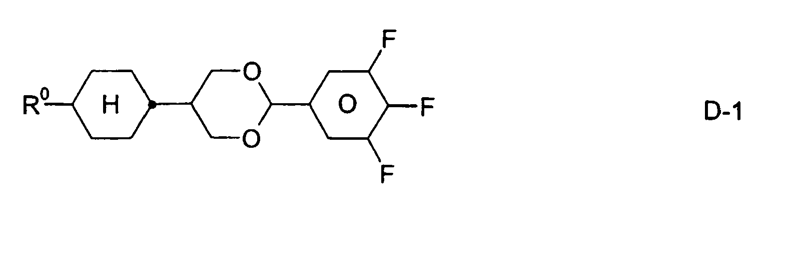 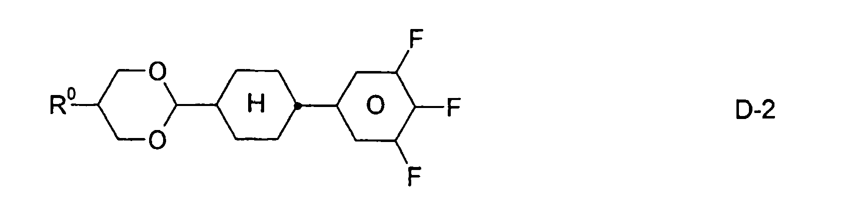 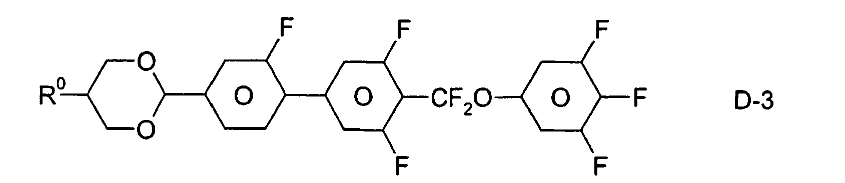 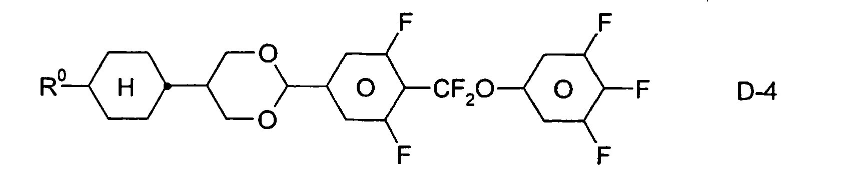
worin R⁰ die oben angegebenen Bedeutungen hat.

Der Anteil der Dioxan-Verbindungen D-1 bis D-4 in den erfindungsgemäßen Mischungen beträgt vorzugsweise 0-30 Gew.%, insbesondere 5-25 Gew.% und ganz besonders bevorzugt 8-20 Gew.%.
- Das Medium enthält zusätzlich ein, zwei oder mehr Zweikern-Verbindungen der Formeln Z-1 bis Z-8, 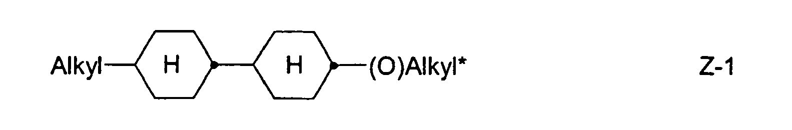 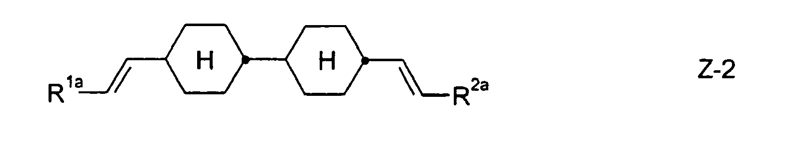 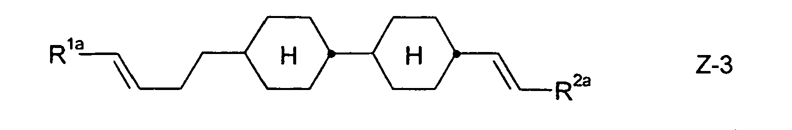 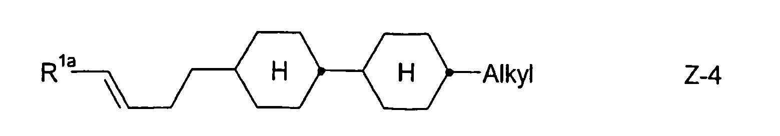 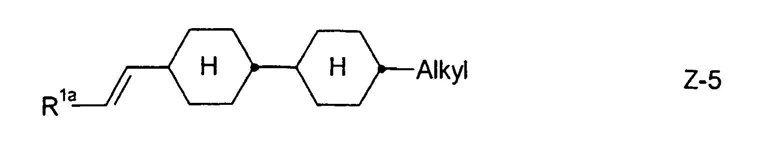 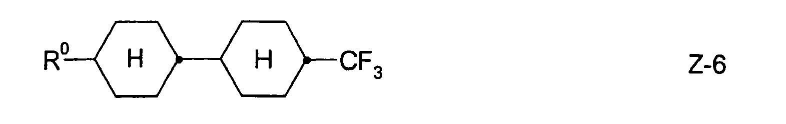 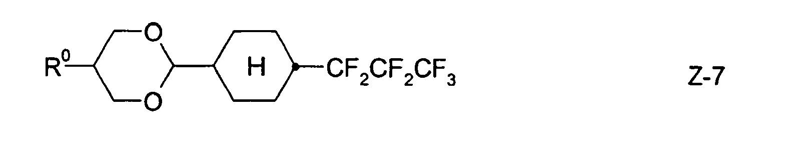 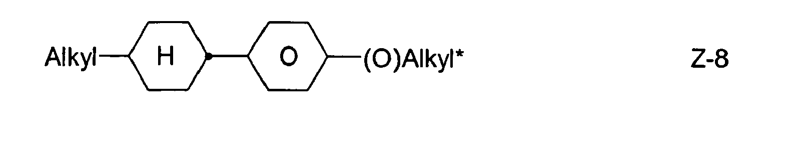 worin R^{1a} und R^{2a} jeweils unabhängig voneinander H, CH₃, C₂H₅ oder n-C₃H₇ bedeuten. Alkyl und Alkyl* bedeuten jeweils unabhängig voneinander eine geradkettige oder verzweigte Alkylkette mit 1-7 C-Atomen. R° hat die oben angegebenen Bedeutungen. In den Verbindungen Z-6 und Z-7 bedeutet R° vorzugsweise geradkettiges Alkyl oder Alkenyl.

Von den genannten Zweikern-Verbindungen sind besonders bevorzugt die Verbindungen der Formeln Z-1, Z-2, Z-5, Z-6 und Z-8.
- Das Medium enthält zusätzlich eine, zwei oder mehr Verbindungen mit annellierten Ringen der Formeln AN1 bis AN11: 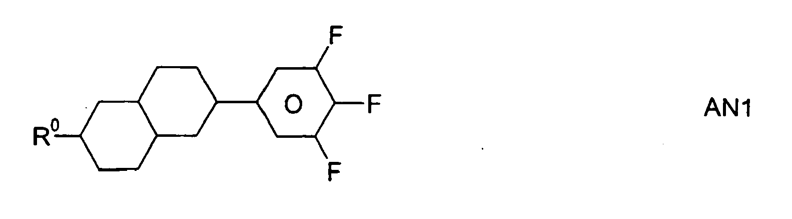 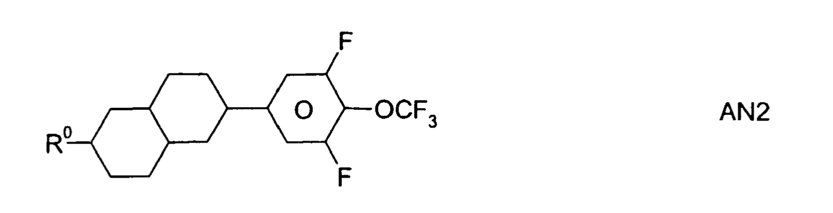 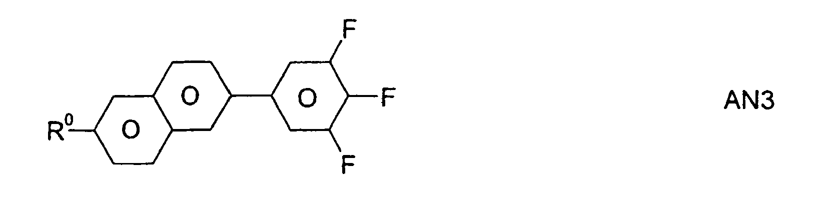 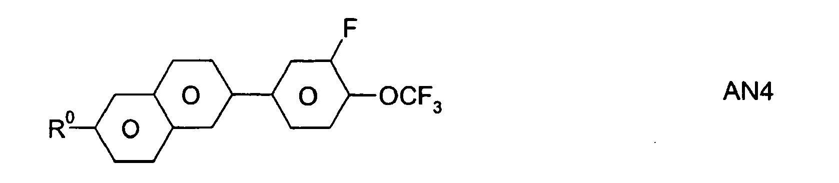 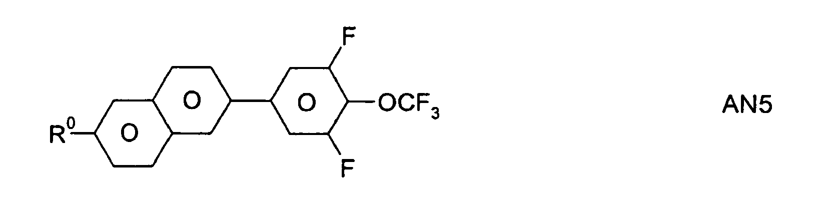 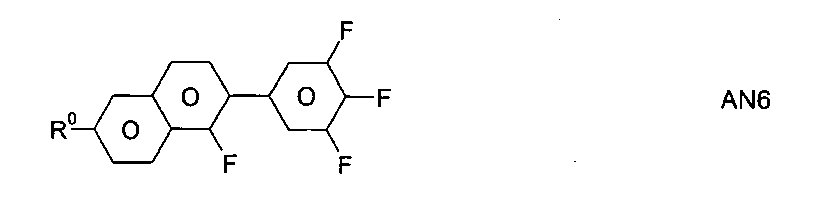 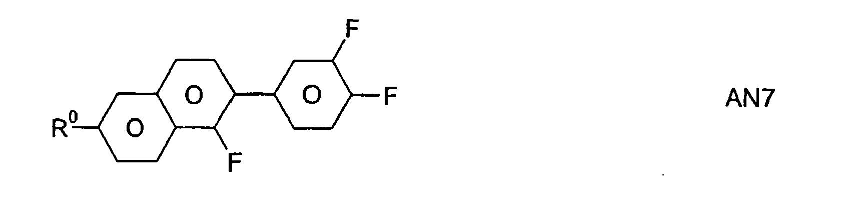 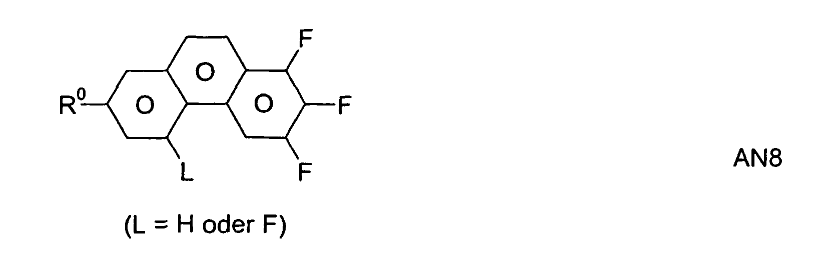 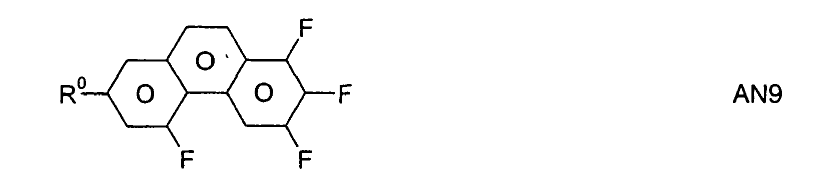 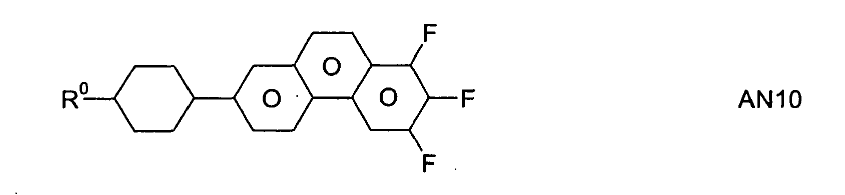 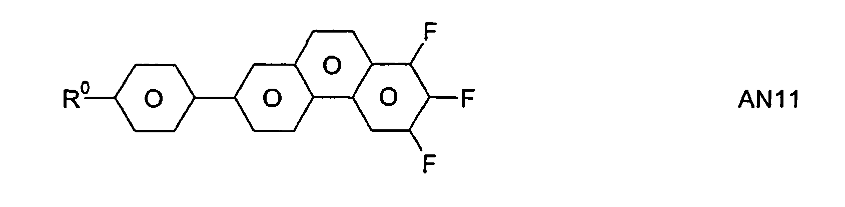 worin R° die oben angegebenen Bedeutungen hat;
- Die erfindungsgemäßen Mischungen zeichnen sich insbesondere dadurch aus, dass sie Klärpunkte von > 75°C und Schwellen von < 2,0 V aufweisen.

Es wurde gefunden, dass bereits ein relativ geringer Anteil an Verbindungen der Formeln I und I* im Gemisch mit üblichen Flüssigkristallmaterialien, insbesondere jedoch mit einer oder mehreren Verbindungen der Formeln II, III, IV, V, VI, XIII, XIV, XV, XVI, XVII, XVIII und/oder XIX zu einer beträchtlichen Erniedrigung der Schwellenspannung führt, wobei gleichzeitig breite nematische Phasen mit tiefen Übergangstemperaturen smektisch-nematisch beobachtet werden, wodurch die Lagerstabilität verbessert wird. Gleichzeitig zeigen die Mischungen sehr gute Werte für die VHR bei UV-Belastung.

Der Ausdruck "Alkyl" bzw. "Alkyl*" umfasst geradkettige und verzweigte Alkylgruppen mit 1-7 Kohlenstoffatomen, insbesondere die geradkettigen Gruppen Methyl, Ethyl, Propyl, Butyl, Pentyl, Hexyl und Heptyl. Gruppen mit 1-6 Kohlenstoffatomen sind im allgemeinen bevorzugt.

Der Ausdruck "Alkenyl" umfasst geradkettige und verzweigte Alkenylgruppen mit 2-7 Kohlenstoffatomen, insbesondere die geradkettigen Gruppen. Bevorzugte Alkenylgruppen sind C₂-C₇-1 E-Alkenyl, C₄-C₇-3E-Alkenyl, C₅-C₇-4-Alkenyl, C₆-C₇-5-Alkenyl und C₇-6-Alkenyl, insbesondere C₂-C₇-1 E-Alkenyl, C₄-C₇-3E-Alkenyl und C₅-C₇-4-Alkenyl. Beispiele besonders bevorzugter Alkenylgruppen sind Vinyl, 1 E-Propenyl, 1 E-Butenyl, 1 E-Pentenyl, 1 E-Hexenyl, 1 E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfasst vorzugsweise geradkettige Gruppen mit endständigem Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl" bzw. "Alkoxy" umfasst vorzugsweise geradkettige Reste der Formel CₙH₂ₙ₊₁-O-(CH₂)ₘ, worin n und m jeweils unabhängig voneinander 1 bis 6 bedeuten. m kann auch 0 bedeuten. Vorzugsweise ist n = 1 und m 1-6 oder m = 0 und n = 1-3.

Durch geeignete Wahl der Bedeutungen von R° und X° können die Ansprechzeiten, die Schwellenspannung, die Steilheit der Transmissionskennlinien etc. in gewünschter Weise modifiziert werden. Beispielsweise führen 1E-Alkenylreste, 3E-Alkenylreste, 2E-Alkenyloxyreste und dergleichen in der Regel zu kürzeren Ansprechzeiten, verbesserten nematischen Tendenzen und einem höheren Verhältnis der elastischen Konstanten k₃₃ (bend) und k₁₁ (splay) im Vergleich zu Alkyl- bzw. Alkoxyresten. 4-Alkenylreste, 3-Alkenylreste und dergleichen ergeben im allgemeinen tiefere Schwellenspannungen und kleinere Werte von k₃₃/k₁₁ im Vergleich zu Alkyl- und Alkoxyresten.

Eine -CH₂CH₂-Gruppe führt im allgemeinen zu höheren Werten von k₃₃/k₁₁ im Vergleich zu einer einfachen Kovalenzbindung. Höhere Werte von k₃₃/k₁₁ ermöglichen z.B. flachere Transmissionskennlinien in TN-Zellen mit 90° Verdrillung (zur Erzielung von Grautönen) und steilere Transmissionskennlinien in STN-, SBE- und OMI-Zellen (höhere Multiplexierbarkeit) und umgekehrt. Höhere Werte für K₁ ermöglichen schnellere Schaltzeiten.

Das optimale Mengenverhältnis der Verbindungen der Formeln I und II + III + IV + V + VI hängt weitgehend von den gewünschten Eigenschaften, von der Wahl der Komponenten der Formeln I, II, III, IV, V und/oder VI und der Wahl weiterer gegebenenfalls vorhandener Komponenten ab.

Geeignete Mengenverhältnisse innerhalb des oben angegebenen Bereichs können von Fall zu Fall leicht ermittelt werden.

Die Gesamtmenge an Verbindungen der Formeln I*, I bis XIX in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die Ansprechzeiten und die Schwellenspannung ist jedoch in der Regel umso größer je höher die Gesamtkonzentration an Verbindungen der Formeln I*, I bis XIX sind.

In einer besonders bevorzugten Ausführungsform enthalten die erfindungsgemäßen Medien Verbindungen der Formel II bis VI (vorzugsweise II, III und/oder IV, insbesondere IVa), worin X° F, OCF₃, OCHF₂, OCH=CF₂, OCF=CF₂ oder OCF₂-CF₂H bedeutet. Eine günstige synergistische Wirkung mit den Verbindungen der Formeln I führt zu besonders vorteilhaften Eigenschaften. Insbesondere Mischungen enthaltend Verbindungen der Formeln I, I* und IVa zeichnen sich durch ihre niedrige Schwellenspannung aus.

Die einzelnen Verbindungen der Formeln I, I* und II bis XIX und deren Unterformeln, die in den erfindungsgemäßen Medien verwendet werden können, sind entweder bekannt, oder sie können analog zu den bekannten Verbindungen hergestellt werden.

Der Aufbau der erfindungsgemäßen MFK-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der MFK-Anzeige, insbesondere auch Matrix-Anzeigeelemente auf Basis poly-Si TFT oder MIM.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die Herstellung der erfindungsgemäß verwendbaren Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation.

Die Dielektrika können auch weitere, dem Fachmann bekannte und in der Literatur beschriebene Zusätze, wie z. B. UV-Stabilisatoren wie Tinuvin^{®} der Fa. Ciba, Antioxidantien, Radikalfänger, etc., enthalten. Beispielsweise können 0-15 % pleochroitische Farbstoffe oder chirale Dotierstoffe zugesetzt werden. Geeignete Stabilisatoren und Dotierstoffe werden nachfolgend in den Tabellen C und D genannt.

C bedeutet eine kristalline, S eine smektische, S_{c} eine smektisch C, N eine nematische und I die isotrope Phase.

V₁₀ bezeichnet die Spannung für 10 % Transmission (Blickrichtung senkrecht zur Plattenoberfläche). tₒₙ bezeichnet die Einschaltzeit und t_{off} die Ausschaltzeit bei einer Betriebsspannung entsprechend dem 2,0fachen Wert von V₁₀. Δn bezeichnet die optische Anisotropie. Δε bezeichnet die dielektrische Anisotropie (Δε = ε_{∥} - ε_{⊥}, wobei ε_{∥} die Dielektrizitätskonstante parallel zu den Moleküllängsachsen und ε_{⊥} die Dielektrizitätskonstante senkrecht dazu bedeutet). Die elektro-optischen Daten werden in einer TN-Zelle im 1. Minimum (d.h. bei einem d . Δn-Wert von 0,5 µm) bei 20 °C gemessen, sofern nicht ausdrücklich etwas anderes angegeben wird. Die optischen Daten werden bei 20°C gemessen, sofern nicht ausdrücklich etwas anderes angegeben wird.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen; n und m sind ganze Zahlen und bedeuten vorzugsweise 0, 1, 2, 3, 4, 5, 6, 7, 8, 9, 10, 11 oder 12. Die Codierung gemäß Tabelle B versteht sich von selbst. In Tabelle A ist nur das Acronym für den Grundkörper angegeben. Im Einzelfall folgt getrennt von Acronym für den Grundkörper mit einem Strick ein Code für die Substituenten R^{1*}, R^{2*} , L^{1*} und L^{2*}:

| Code für R^{1*}, | R^{1*} | R^{2*} | L^{1*} | L^{2*} |
|---|---|---|---|---|
| R^{2*}, L^{1*}, L^{2*}, | | | | |
| L^{3*} | | | | |
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | F | H |
| nN.F.F | CₙH₂ₙ₊₁ | CN | F | F |
| nF | CₙH₂ₙ₊₁ | F | H | H |
| nCl | CₙH₂ₙ₊₁ | Cl | H | H |

| | | | | |
|---|---|---|---|---|
| nOF | OCₙH₂ₙ₊₁ | F | H | H |
| nF.F | CₙH₂ₙ₊₁ | F | F | H |
| nF.F.F | CₙH₂ₙ₊₁ | F | F | F |
| nmF | CₙH₂ₙ₊₁ | CₘH₂ₘ₊₁ | F | H |
| nOCF₃ | CₙH₂ₙ₊₁ | OCF₃ | H | H |
| nOCF₃.F | CₙH₂ₙ₊₁ | OCF₃ | F | H |
| n-Vm | CₙH₂ₙ₊₁ | -CH=CH-CₘH₂ₘ₊₁ | H | H |
| nV-Vm | CₙH₂ₙ₊₁-CH=CH- | -CH=CH-CₘH₂ₘ₊₁ | H | H |

Bevorzugte Mischungskomponenten finden sich in den Tabellen A und B.

**Tabelle A**

| |
|---|
| 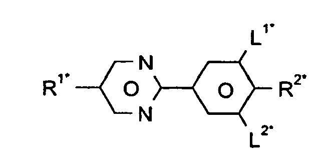 **PYP** |
| 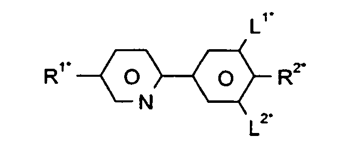 **PYRP** |
| 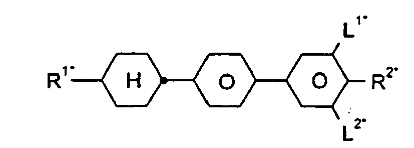 **BCH** |
| 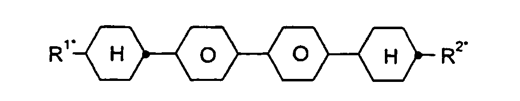 **CBC** |
| 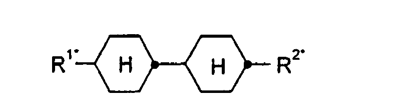 **CCH** |
| 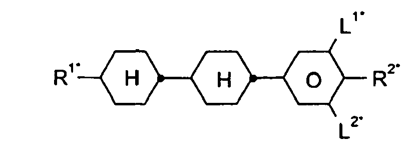 **CCP** |
| 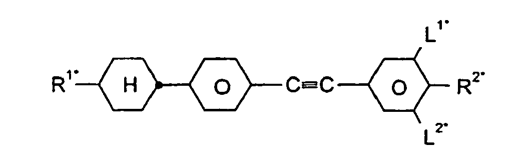 **CPTP** |
| 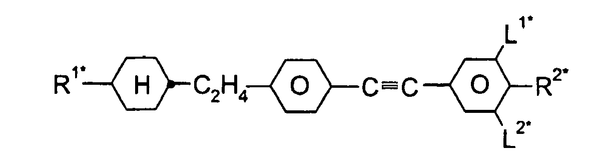 **CEPTP** |
| 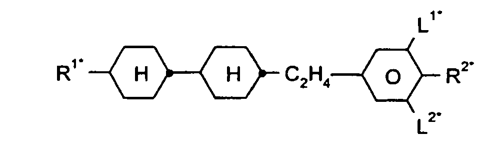 **ECCP** |
| 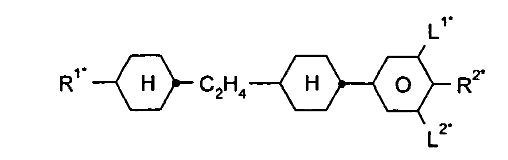 **CECP** |
| 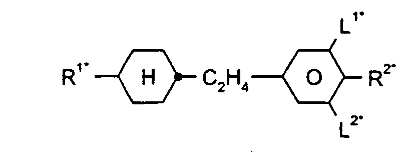 **EPCH** |
| 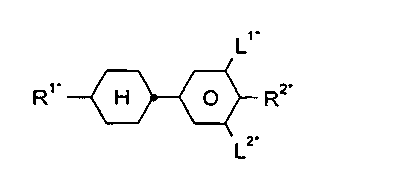 **PCH** |
| 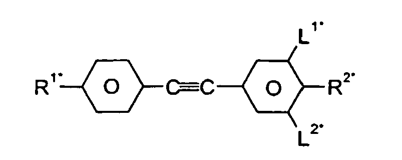 **PTP** |
| 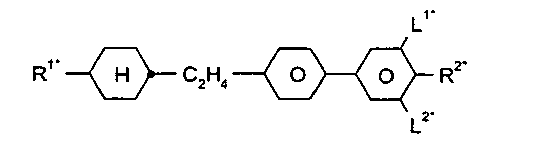 **BECH** |
| 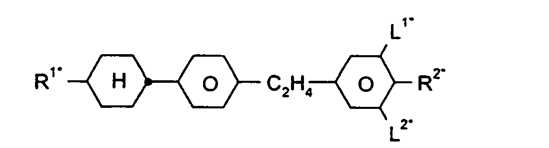 **EBCH** |
| 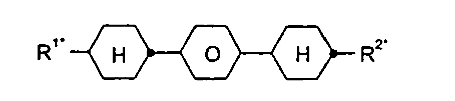 **CPC** |
| 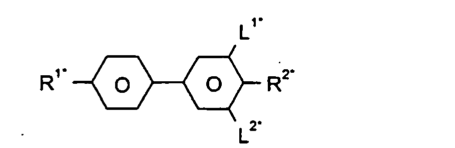 **B** |
| 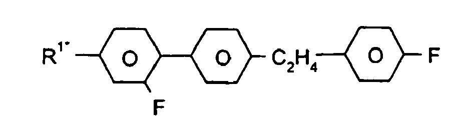 **FET-nF** |
| 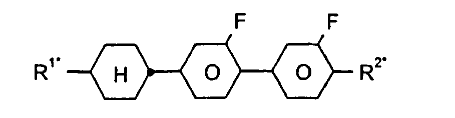 **CGG** |
| 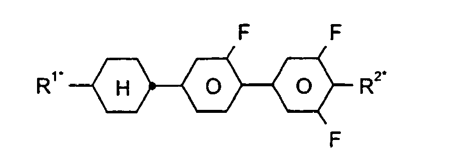 **CGU** |
| 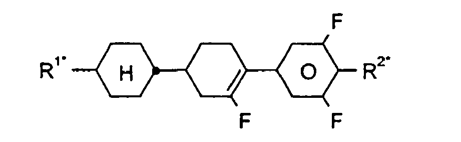 **CFU** |

**Tabelle B**

| |
|---|
| 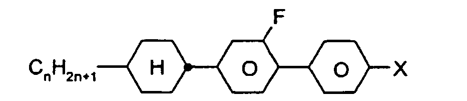 **BCH-n.Fm** |
| 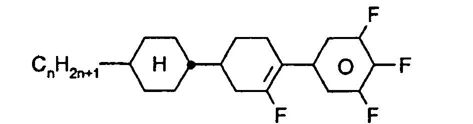 **CFU-n-F** |
| 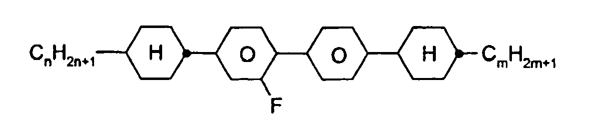 **CBC-nmF** |
| 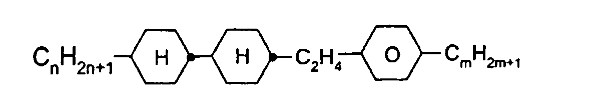 **ECCP-nm** |
| 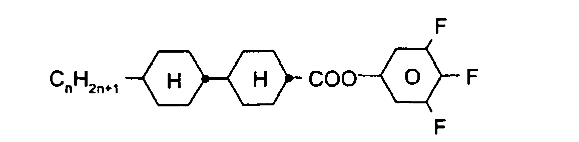 **CCZU-n-F** |
| 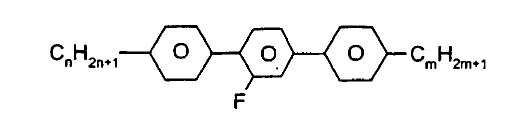 **T-nFm** |
| 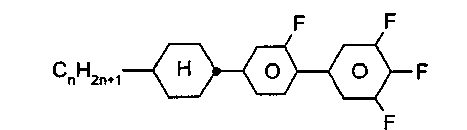 **CGU-n-F** |
| 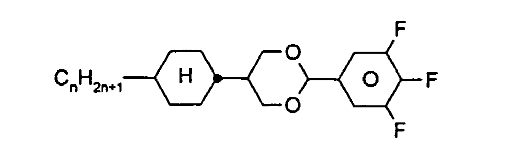 **CDU-n-F** |
| 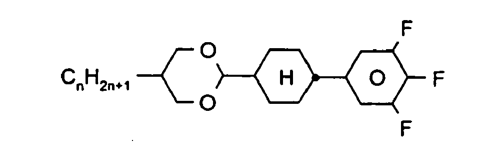 **DCU-n-F** |
| 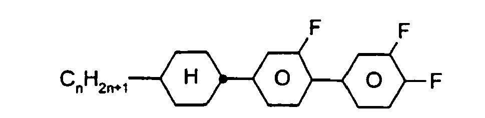 **CGG-n-F** |
| 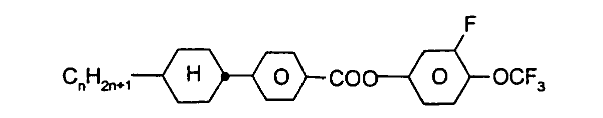 **CPZG-n-OT** |
| 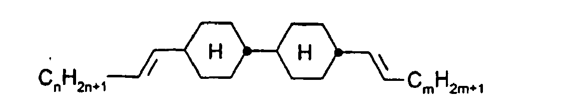 **CC-nV-Vm** |
| 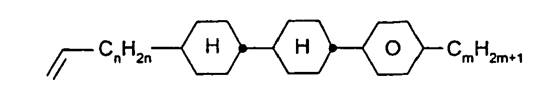 **CCP-Vn-m** |
| 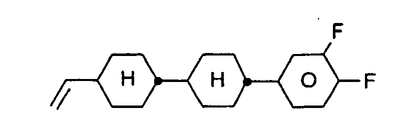 **CCG-V-F** |
| 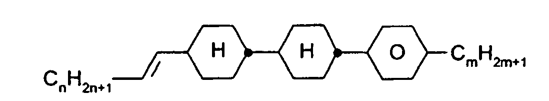 **CCP-nV-m** |
| 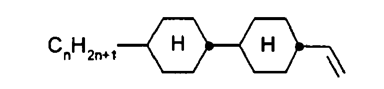 **CC-n-V** |
| 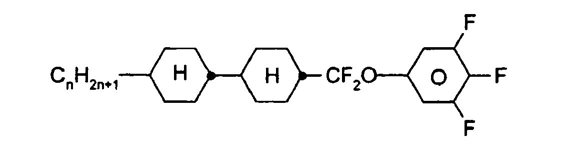 **CCQU-n-F** |
| 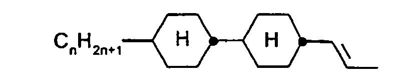 **CC-n-V1** |
| 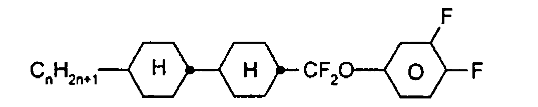 **CCQG-n-F** |
| 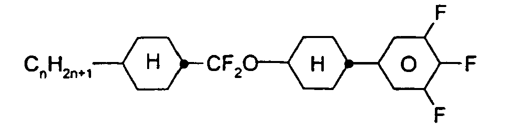 **CQCU-n-F** |
| 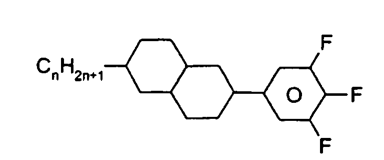 **Dec-U-n-F** |
| 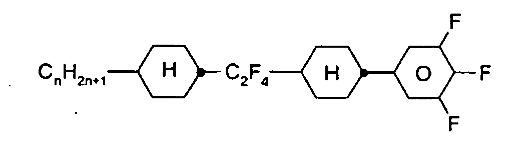 **CWCU-n-F** |
| 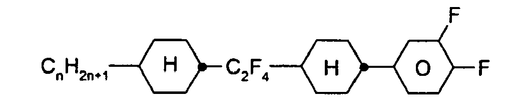 **CWCG-n-F** |
| 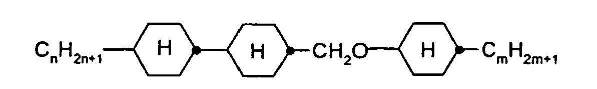 **CCOC-n-m** |
| 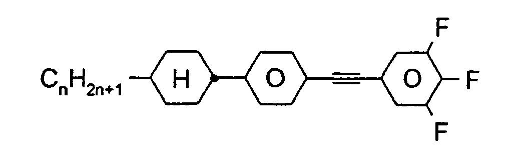 **CPTU-n-F** |
| 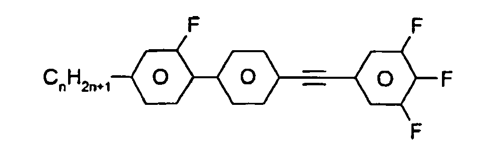 **GPTU-n-F** |
| 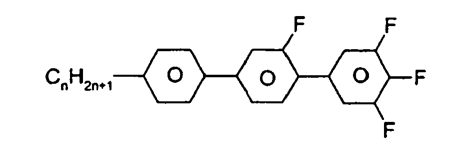 **PGU-n-F** |
| 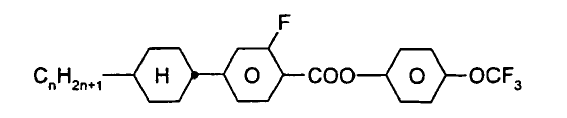 **CGZP-n-OT** |
| 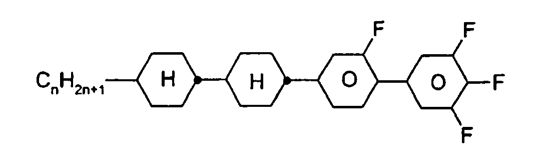 **CCGU-n-F** |
| 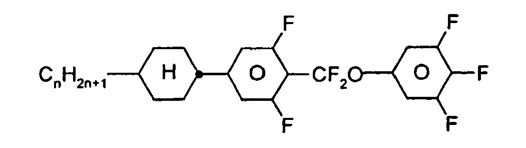 **CUQU-n-F** |
| 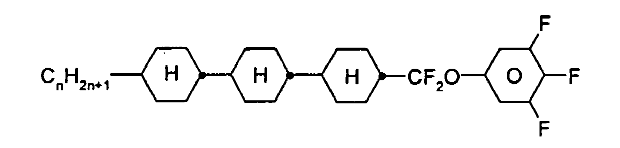 **CCCQU-n-F** |
| 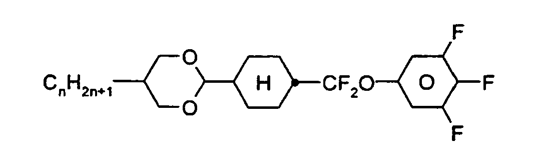 **DCQU-n-F** |
| 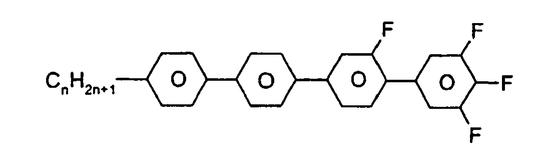 **PPGU-n-F** |
| 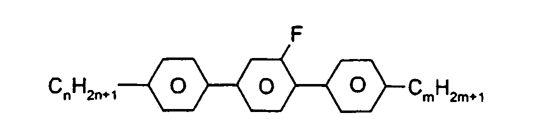 **PGP-n-m** |
| 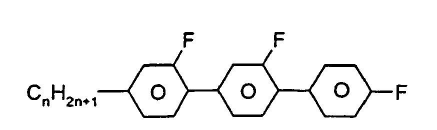 **GGP-n-F** |

Besonders bevorzugt sind flüssigkristalline Mischungen, die neben den Verbindungen der Formeln I mindestens ein, zwei, drei, vier oder mehr Verbindungen aus der Tabelle B enthalten.

**Tabelle C**

| |
|---|
| In der Tabelle C werden mögliche Dotierstoffe angegeben, die in der Regel den erfindungsgemäßen Mischungen zugesetzt werden. Vorzugsweise enthalten die Mischungen 0-10 Gew.%, insbesondere 0,01-5 Gew.% und besonders bevorzugt 0,01-3 Gew.% an Dotierstoffen. |
| 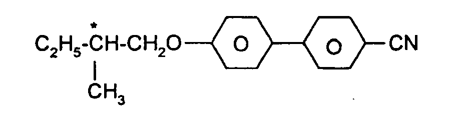 **C15** |
| 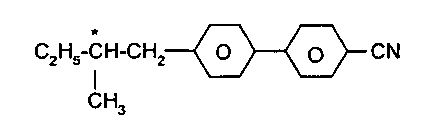 **CB 15** |
| 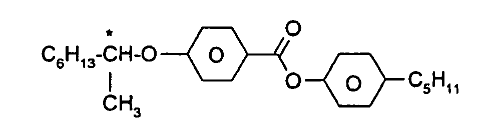 **CM 21** |
| 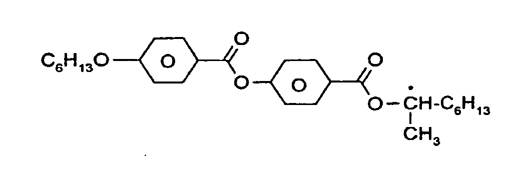 **R/S-811** |
| 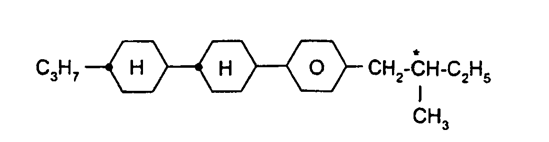 **CM 44** |
| 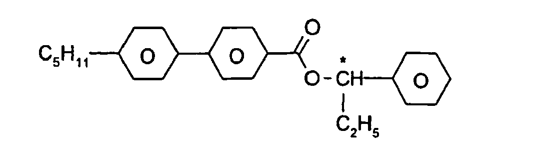 **CM 45** |
| 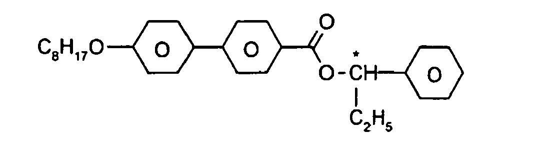 **CM 47** |
| 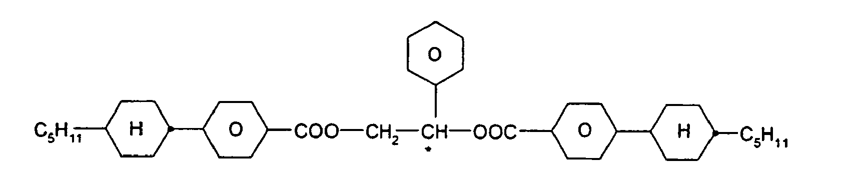 **R/S-1011** |
| 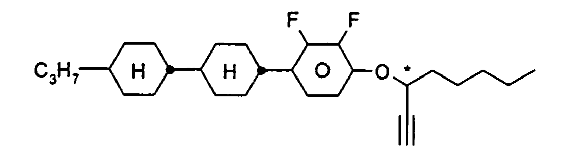 **R/S-3011** |
| 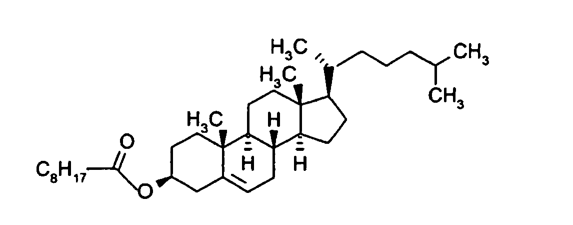 **CN** |
| 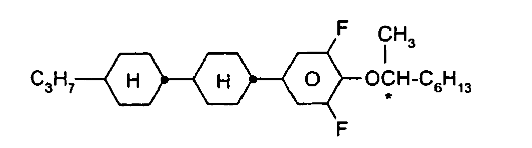 **R/S-2011** |
| 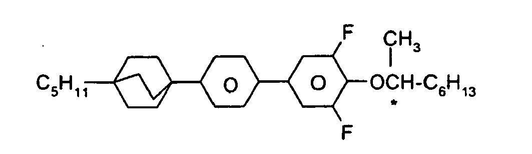 **R/S-4011** |
| 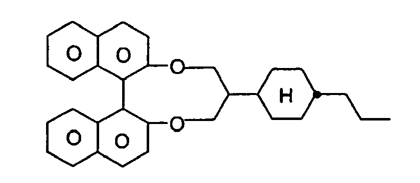 **R/S-5011** |

**Tabelle D**

| | |
|---|---|
| Stabilisatoren, die beispielsweise den erfindungsgemäßen Mischungen in Mengen von 0-10 Gew.% zugesetzt werden können, werden nachfolgend genannt. | |
| 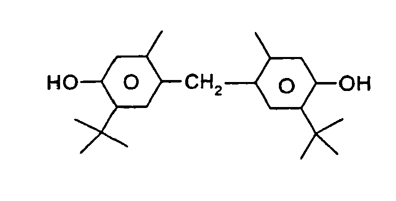 | 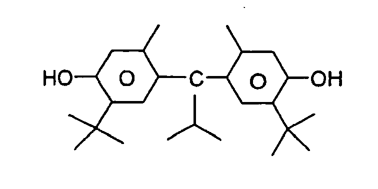 |
| 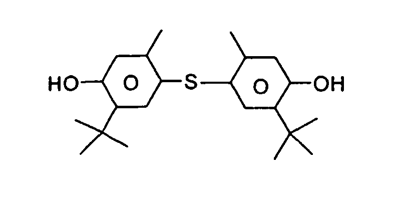 | 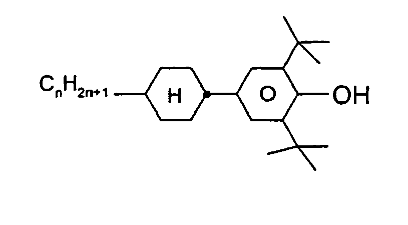 |
| 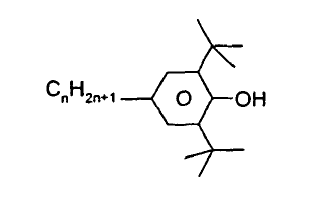 | 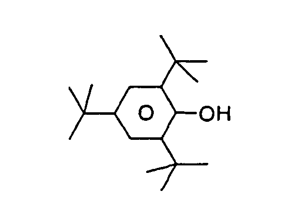 |
| 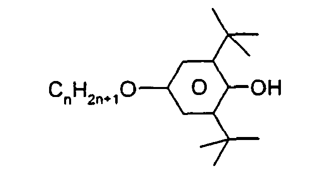 | 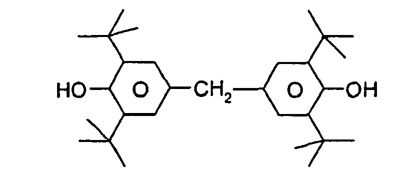 |
| 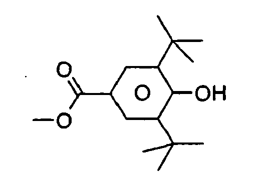 | 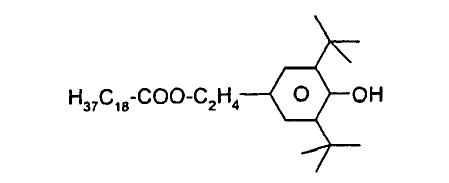 |
| 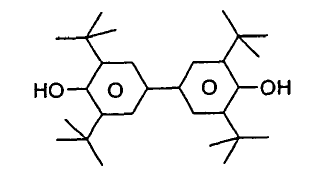 | 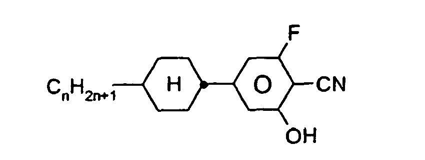 |
| 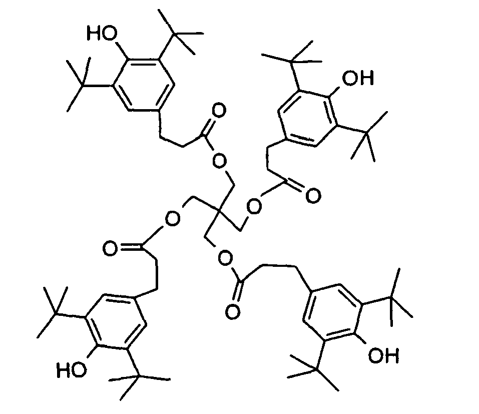 | |
| 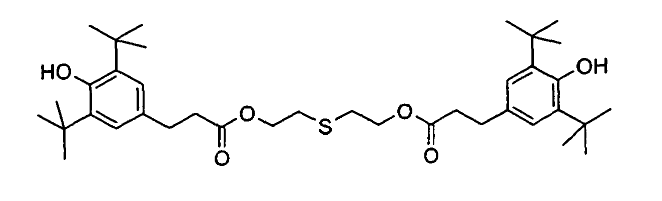 | |
| 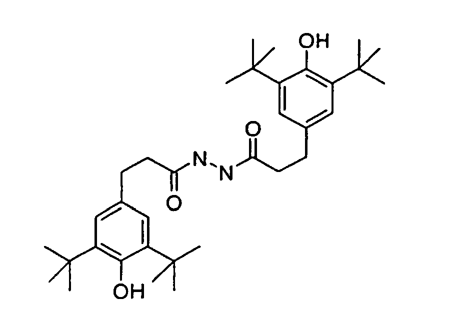 | |
| 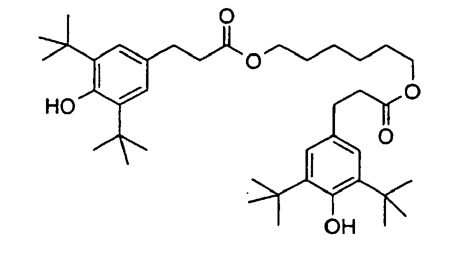 | |
| 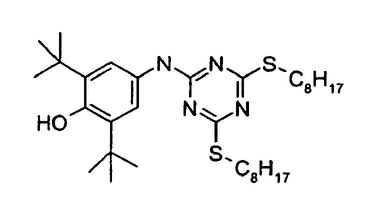 | 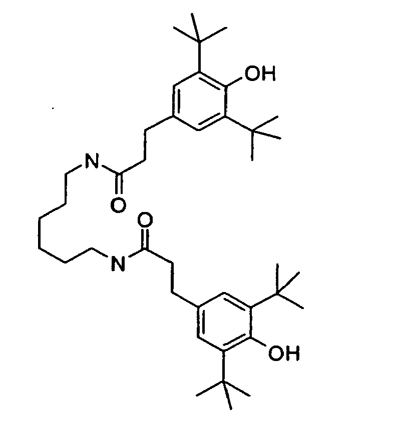 |
| 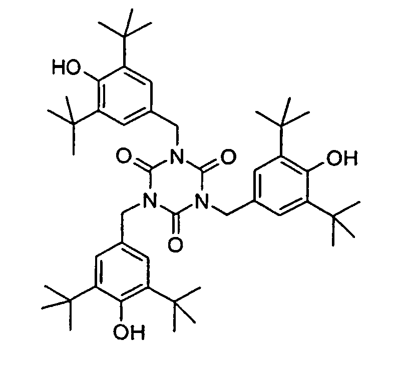 | 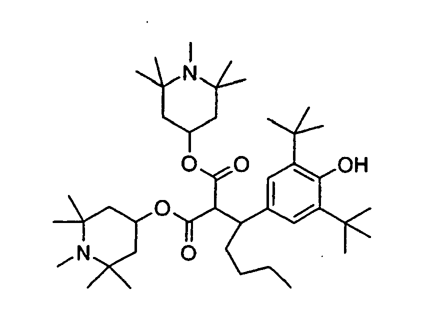 |
| 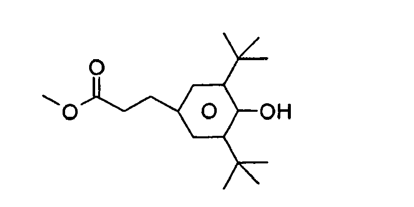 | 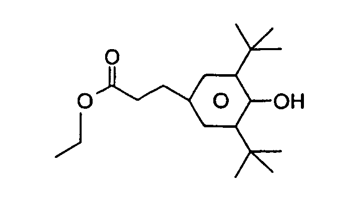 |
| 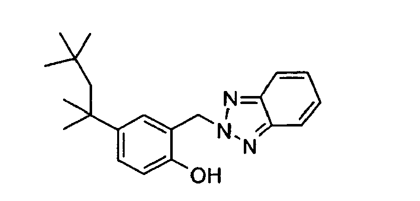 | 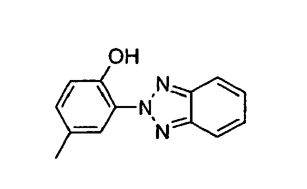 |
| 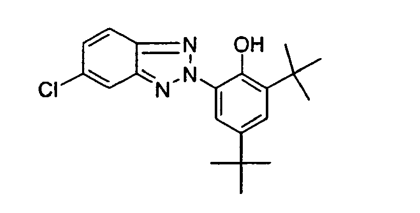 | 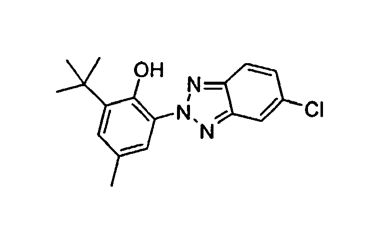 |
| 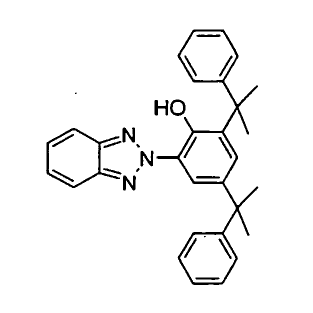 | 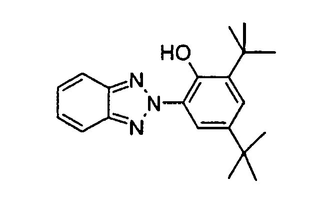 |
| 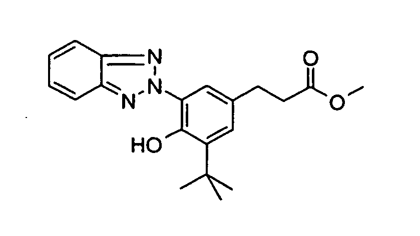 | 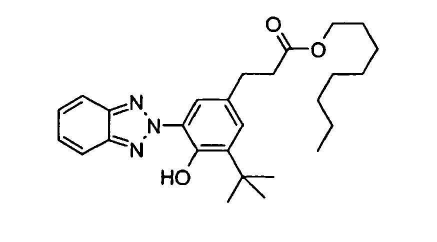 |
| 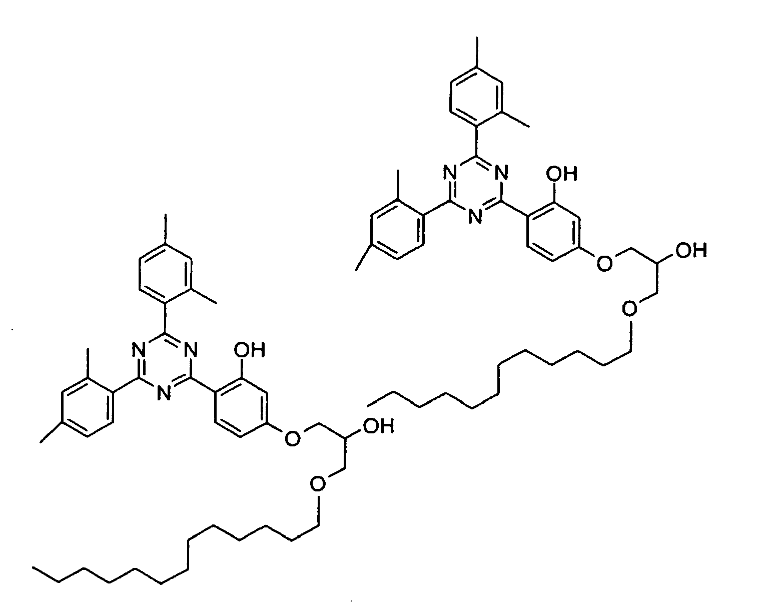 | |
| 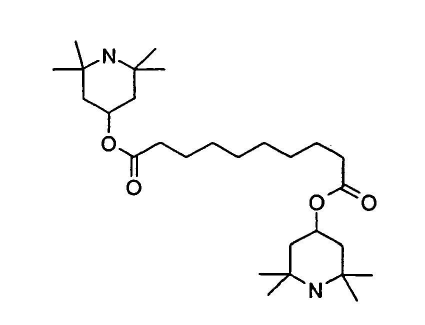 | 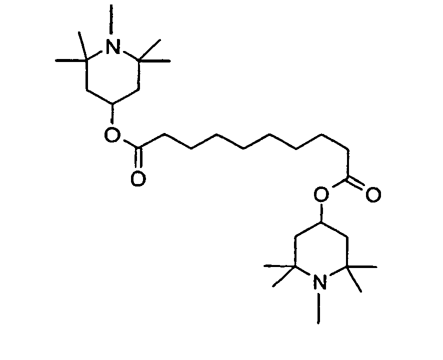 |

Die folgenden Beispiele sollen die Erfindung erläutern . Vor- und nachstehend bedeuten Prozentangaben Gewichtsprozent. Alle Temperaturen sind in Grad Celsius angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20°C), die Fließviskosität v₂₀ (mm²/sec) und die Rotationsviskosität γ₁ (mPa·s) werden jeweils bei 20°C bestimmt.

"Übliche Aufarbeitung" bedeutet: Man gibt gegebenenfalls Wasser hinzu, extrahiert mit Methylenchlorid, Diethylether oder Toluol, trennt ab, trocknet die organische Phase, dampft ein und reinigt das Produkt durch Destillation unter reduziertem Druck oder Kristallisation und/oder Chromatographie.

### Beispiel 1

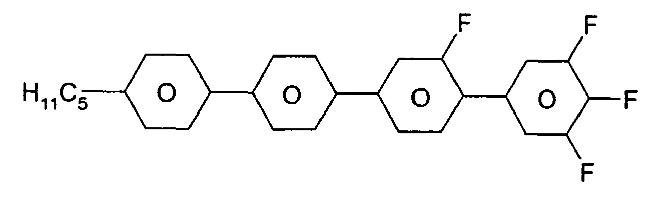

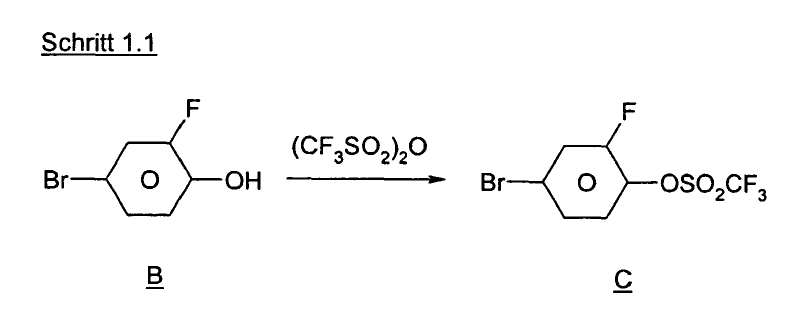

Zu einer kalten Lösung (5 °C) von 262 mmol B in 767 ml CH₂Cl₂ werden zunächst 51 ml Triethylamin und 650 mg 4-Dimethylaminopyridin und dann 262 mmol Trifluormethansulfonsäureanhydrid zugegeben. Man lässt das Reaktionsgemisch auf Raumtemperatur erwärmen und rührt über Nacht. Nach Zugabe von 800 ml n-Heptan wird das Produkt C mittels Säulenchromatographie gereinigt.

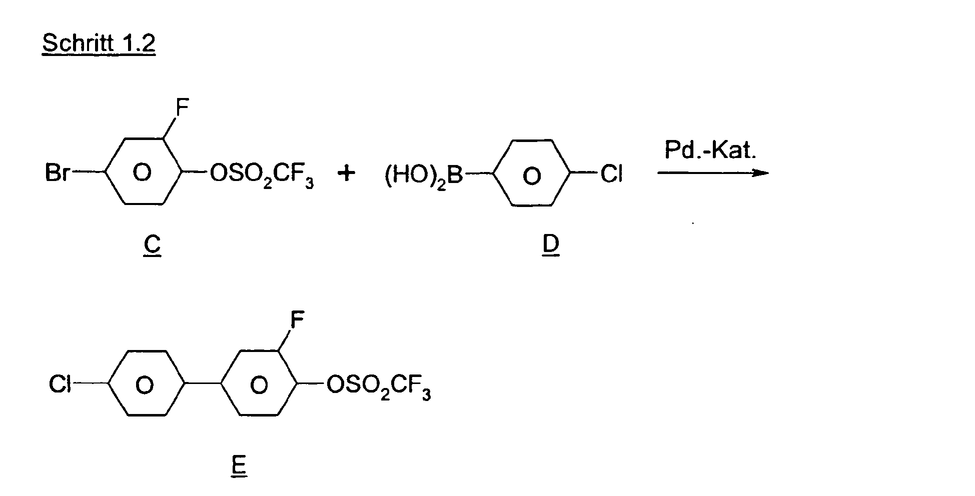

58 mmol C, 58 mmol D, 87 mmol Natriummetaborat · 8 H₂O, 1,1 mmol Bis(triphenylphosphin)PdCl₂ und 1,7 mmol Hydrazinhydroxid werden in 34 ml Wasser und 66 ml THF gelöst und bei 70°C über Nacht gerührt. Zu der erkalteten Reaktionslösung werden 100 ml Wasser zugegeben. Nach der Extraktion mit Methyl-tert.butylether werden die vereinigten organischen Phasen abgetrennt, mit Wasser gewaschen und wie üblich aufgearbeitet.

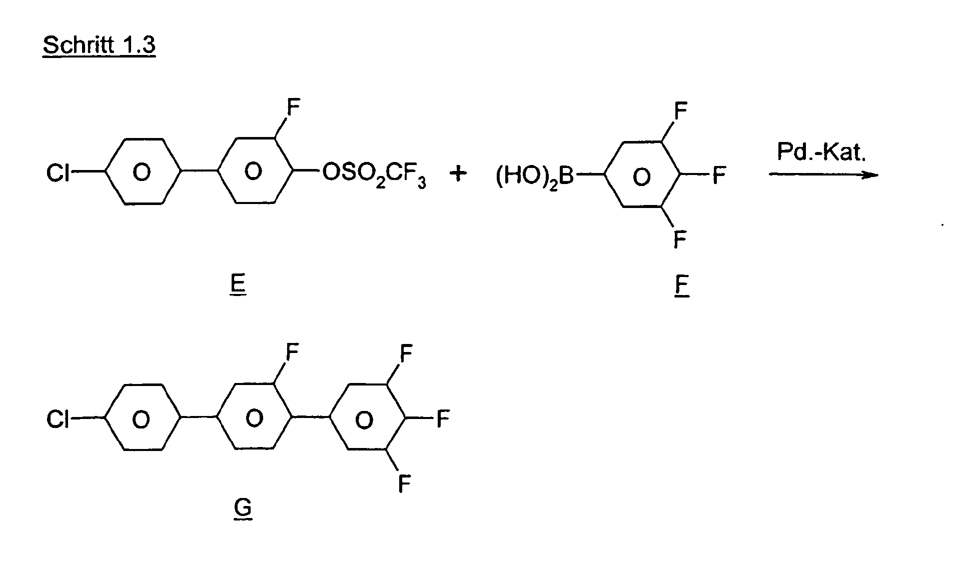

113 mmol E, 113 mmol F und 275 mmol Natriummetaborat · 8 H₂O, 2,2 mmol Bis(triphenylphosphin)PdCl₂ und 3,4 mmol Hydrazinhydroxid werden in 67 ml Wasser und 130 ml THF gelöst und über Nacht auf 70 °C erhitzt. Zu der erkalteten Reaktionslösung werden 200 ml Wasser zugegeben. Nach der Extraktion mit Methyl-tert.butylether werden die vereinigten organischen Phasen mit Wasser gewaschen und wie üblich aufgearbeitet.

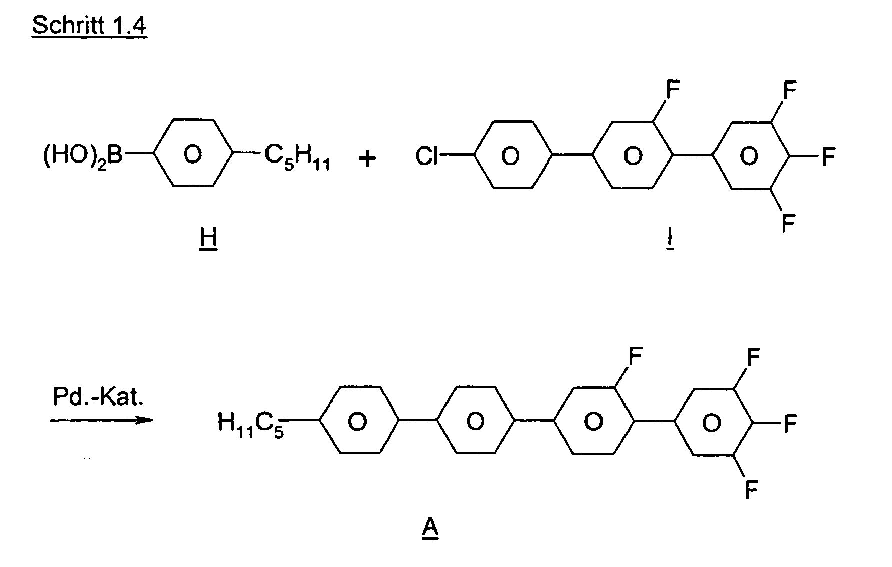

113 mmol I, 10,8 mmol H, 152 mmol Cesiumfluorid und 0,49 mmol Bis(tricyclohexylphosphin)PdCl₂ werden in 30 ml 1,4-Dioxan gelöst und über Nacht auf 100°C erhitzt. Zu der erkalten Reaktionslösung werden 50 ml Wasser zugegeben. Nach der Extraktion mit Methyl-tert.butylether werden die vereinigten organischen Phasen mit Wasser gewaschen und wie üblich aufgereinigt.

K 83 S_{E} 112 S_{A} 215 N 237,3 I ; Δn = 0,3060; Δε = 17,6

Analog werden die folgenden Verbindungen der Formel 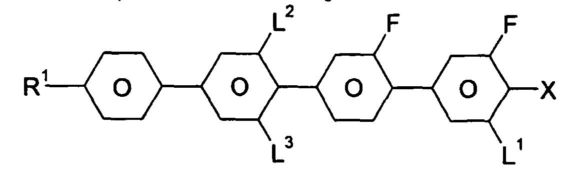 hergestellt:

| R¹ | X | L¹ | L² | L³ | |
|---|---|---|---|---|---|
| CH₃ | F | F | H | H | K178SA215N |
| | | | | | 270,6 I |
| C₂H₅ | F | F | H | H | K 149 S_{E} (139) |
| | | | | | S_{A} 209 N 249,5 I |
| | | | | | Δn = 0,3186; |
| | | | | | Δε = 20,5 |
| C₃H₇ | F | F | H | H | K 111 S_{E} 135 |
| | | | | | S_{A} 213 N 250,2 I |
| | | | | | Δn = 0,3270; |
| | | | | | Δε = 19,9 |

| R¹ | | X | | L¹ | L² | L³ | |
|---|---|---|---|---|---|---|---|
| C₄H₉ | | F | | F | H | H | K 92 S_{H} 108 |
| | | | | | | | S_{E} 114 S_{c} 115 |
| | | | | | | | S_{A} 215 N |
| | | | | | | | 239,9 I; |
| | | | | | | | Δn = 0,3082; |
| | | | | | | | Δε = 19,0 |
| C₆H₁₃ | | F | | F | H | H | K 79 S_{E} 106 |
| | | | | | | | S_{A} 213 N |
| | | | | | | | 226,6 I; |
| | | | | | | | Δn = 0,2970; |
| | | | | | | | Δε = 17,7 |

| R¹ | X | | L¹ | L² | L³ | |
|---|---|---|---|---|---|---|
| C₇H₁₅ | F | | F | H | H | K 68 S_{E} 103 |
| | | | | | | S_{A} 214 N |
| | | | | | | 224,1 I; |
| | | | | | | Δn = 0,2938; |
| | | | | | | Δε = 16,4 |
| CH₂₌CH₂ | F | | F | H | H | |
| CH₃CH=CH | F | | F | H | H | |
| CH₃O | F | | F | H | H | |
| C₂H₅O | F | | F | H | H | |

| R¹ | X | | L¹ | L² | L³ | |
|---|---|---|---|---|---|---|
| C₃H₇O | F | | F | H | H | |
| CH₃OCH₂ | F | | F | H | H | |
| CH₃ | Cl | | F | H | H | |
| C₂H₅ | Cl | | F | H | H | |
| C₃H₇ | Cl | | F | H | H | |
| C₄H₉ | Cl | | F | H | H | |

| R¹ | X | | L¹ | L² | L³ | |
|---|---|---|---|---|---|---|
| C₅H₁₁ | Cl | | F | H | H | |
| C₆H₁₃ | Cl | | F | H | H | |
| CH₂=CH₂ | Cl | | F | H | H | |
| CH₂=CH₂ | Cl | | F | H | H | |
| CH₃CH=CH | Cl | | F | H | H | |
| CH₃O | Cl | | F | H | H | |
| C₂H₅O | Cl | | F | H | H | |

| R¹ | | X | | L¹ | L² | L³ | |
|---|---|---|---|---|---|---|---|
| C₃H₇O | | Cl | | F | H | H | |
| CH₃OCH₂ | | Cl | | F | H | H | |
| CH₃ | | CN | | F | H | H | |
| C₂H₅ | | CN | | F | H | H | |
| C₃H₇ | | CN | | F | H | H | |
| C₄H₉ | | CN | | F | H | H | |

| R¹ | | X | | L¹ | L² | L³ | |
|---|---|---|---|---|---|---|---|
| C₅H₁₁ | | CN | | F | H | H | |
| C₆H₁₃ | | CN | | F | H | H | |
| CH₂=CH₂ | | CN | | F | H | H | |
| CH₃CH=CH | | CN | | F | H | H | |
| CH₃O | | CN | | F | H | H | |
| C₂H₅O | | CN | | F | H | H | |

| R¹ | X | | L¹ | L² | L³ | |
|---|---|---|---|---|---|---|
| C₃H₇O | CN | | F | H | H | |
| CH₃OCH₂ | CN | | F | H | H | |
| CH₃ | OCF₃ | | F | H | H | |
| C₂H₅ | OCF₃ | | F | H | H | |
| C₃H₇ | OCF₃ | | F | H | H | |
| C₄H₉ | OCF₃ | | F | H | H | |

| R¹ | | X | | L¹ | L² | L³ | |
|---|---|---|---|---|---|---|---|
| C₅H₁₁ | | OCF₃ | | F | H | H | |
| C₆H₁₃ | | OCF₃ | | F | H | H | |
| CH₂=CH₂ | | OCF₃ | | F | H | H | |
| CH₃CH=CH | | OCF₃ | | F | H | H | |
| CH₃O | | OCF₃ | | F | H | H | |
| C₂H₅O | | OCF₃ | | F | H | H | |

| R¹ | | X | | L¹ | L² | L³ | |
|---|---|---|---|---|---|---|---|
| C₃H₇O | | OCF₃ | | F | H | H | |
| CH₃OCH₂ | | OCF₃ | | F | H | H | |
| CH₃ | | OCHF₂ | | F | H | H | |
| C₂H₅ | | OCHF₂ | | F | H | H | |
| C₃H₇ | | OCHF₂ | | F | H | H | |
| C₄H₉ | | OCHF₂ | | F | H | H | |

| R¹ | | X | | L¹ | L² | L³ | |
|---|---|---|---|---|---|---|---|
| C₅H₁₁ | | OCHF₂ | | F | H | H | |
| C₆H₁₃ | | OCHF₂ | | F | H | H | |
| CH₂=CH₂ | | OCHF₂ | | F | H | H | |
| CH₃CH=CH | | OCHF₂ | | F | H | H | |
| CH₃O | | OCHF₂ | | F | H | H | |
| C₂H₅O | | OCHF₂ | | F | H | H | |

| R¹ | | X | | L¹ | L² | L³ | |
|---|---|---|---|---|---|---|---|
| C₃H₇O | | OCHF₂ | | F | H | H | |
| CH₃OCH₂ | | OCHF₂ | | F | H | H | |
| CH₃ | | OC₂F₅ | | F | H | H | |
| C₂H₅ | | OC₂F₅ | | F | H | H | |
| C₃H₇ | | OC₂F₅ | | F | H | H | |
| C₄H₉ | | OC₂F₅ | | F | H | H | |

| R¹ | | X | | L¹ | L² | L³ | |
|---|---|---|---|---|---|---|---|
| C₅H₁₁ | | OC₂F₅ | | F | H | H | |
| C₆H₁₃ | | OC₂F₅ | | F | H | H | |
| CH₂=CH₂ | | OC₂F₅ | | F | H | H | |
| CH₃CH=CH | | OC₂F₅ | | F | H | H | |
| CH₃O | | OC₂F₅ | | F | H | H | |

| R¹ | | X | | L¹ | L² | L³ | |
|---|---|---|---|---|---|---|---|
| C₂H₅O | | OC₂F₅ | | F | H | H | |
| C₃H₇O | | OC₂F₅ | | F | H | H | |
| CH₃OCH₂ | | OC₂F₅ | | F | H | H | |
| CH₃ | | OC₃F₇ | | F | H | H | |
| C₂H₅ | | OC₃F₇ | | F | H | H | |
| C₃H₇ | | OC₃F₇ | | F | H | H | |

| R¹ | | X | | L¹ | L² | L³ | |
|---|---|---|---|---|---|---|---|
| C₄H₉ | | OC₃F₇ | | F | H | H | |
| C₅H₁₁ | | OC₃F₇ | | F | H | H | |
| C₆H₁₃ | | OC₃F₇ | | F | H | H | |
| CH₂=CH₂ | | OC₃F₇ | | F | H | H | |
| CH₃CH=CH | | OC₃F₇ | | F | H | H | |
| CH₃O | | OC₃F₇ | | F | H | H | |

| R¹ | | X | | L¹ | L² | L³ | |
|---|---|---|---|---|---|---|---|
| C₂H₅O | | OC₃F₇ | | F | H | H | |
| C₃H₇O | | OC₃F₇ | | F | H | H | |
| CH₃OCH₂ | | OC₃F₇ | | F | H | H | |
| CH₃ | | OCF₂CHFCF₃ | | F | H | H | |
| C₂H₅ | | OCF₂CHFCF₃ | | F | H | H | |
| C₃H₇ | | OCF₂CHFCF₃ | | F | H | H | |

| R¹ | | X | | L¹ | L² | L³ | |
|---|---|---|---|---|---|---|---|
| C₄H₉ | | OCF₂CHFCF₃ | | F | H | H | |
| C₅H₁₁ | | OCF₂CHFCF₃ | | F | H | H | |
| C₆H₁₃ | | OCF₂CHFCF₃ | | F | H | H | |
| CH₂=CH₂ | | OCF₂CHFCF₃ | | F | H | H | |
| CH₃CH=CH | | OCF₂CHFCF₃ | | F | H | H | |
| CH₃O | | OCF₂CHFCF₃ | | F | H | H | |

| R¹ | | X | | L¹ | L² | L³ | |
|---|---|---|---|---|---|---|---|
| C₂H₅O | | OCF₂CHFCF₃ | | F | H | H | |
| C₃H₇O | | OCF₂CHFCF₃ | | F | H | H | |
| CH₃OCH₂ | | OCF₂CHFCF₃ | | F | H | H | |
| CH₃ | | NCS | | F | H | H | |
| C₂H₅ | | NCS | | F | H | H | |
| C₃H₇ | | NCS | | F | H | H | |

| R¹ | | X | | L¹ | L² | L³ | |
|---|---|---|---|---|---|---|---|
| C₄H₉ | | NCS | | F | H | H | |
| C₅H₁₁ | | NCS | | F | H | H | |
| C₆H₁₃ | | NCS | | F | H | H | |
| CH₂=CH₂ | | NCS | | F | H | H | |
| CH₃CH=CH | | NCS | | F | H | H | |
| CH₃O | | NCS | | F | H | H | |

| R¹ | | X | | L¹ | L² | L³ | |
|---|---|---|---|---|---|---|---|
| C₂H₅O | | NCS | | F | H | H | |
| C₃H₇O | | NCS | | F | H | H | |
| CH₃OCH₂ | | NCS | | F | H | H | |
| CH₃ | | SCN | | F | H | H | |
| C₂H₅ | | SCN | | F | H | H | |
| C₃H₇ | | SCN | | F | H | H | |

| R¹ | | X | | L¹ | L² | L³ | |
|---|---|---|---|---|---|---|---|
| C₄H₉ | | SCN | | F | H | H | |
| C₅H₁₁ | | SCN | | F | H | H | |
| C₆H₁₃ | | SCN | | F | H | H | |
| CH₂=CH₂ | | SCN | | F | H | H | |
| CH₃CH=CH | | SCN | | F | H | H | |
| CH₃O | | SCN | | F | H | H | |

| R¹ | | X | | L¹ | L² | L³ | |
|---|---|---|---|---|---|---|---|
| C₂H₅O | | SCN | | F | H | H | |
| C₃H₇O | | SCN | | F | H | H | |
| CH₃OCH₂ | | SCN | | F | H | H | |
| CH₃ | | SF₅ | | F | H | H | |
| C₂H₅ | | SF₅ | | F | H | H | |
| C₃H₇ | | SF₅ | | F | H | H | |

| R¹ | | X | | L¹ | L² | L³ | |
|---|---|---|---|---|---|---|---|
| C₄H₉ | | SF₅ | | F | H | H | |
| C₅H₁₁ | | SF₅ | | F | H | H | |
| C₆H₁₃ | | SF₅ | | F | H | H | |
| CH₂=CH₂ | | SF₅ | | F | H | H | |
| CH₃CH=CH | | SF₅ | | F | H | H | |

| R¹ | | X | | L¹ | L² | L³ | |
|---|---|---|---|---|---|---|---|
| CH₃O | | SF₅ | | F | H | H | |
| C₂H₅O | | SF₅ | | F | H | H | |
| C₃H₇O | | SF₅ | | F | H | H | |
| CH₃OCH₂ | | SF₅ | | F | H | H | |
| CH₃ | | CF₃ | | F | H | H | |
| C₂H₅ | | CF₃ | | F | H | H | |

| R¹ | | X | | L¹ | L² | L³ | |
|---|---|---|---|---|---|---|---|
| C₃H₇ | | CF₃ | | F | H | H | |
| C₄H₉ | | CF₃ | | F | H | H | |
| C₅H₁₁ | | CF₃ | | F | H | H | |
| C₆H₁₃ | | CF₃ | | F | H | H | |
| CH₂=CH₂ | | CF₃ | | F | H | H | |
| CH₃CH=CH | | CF₃ | | F | H | H | |

| R¹ | | X | | L¹ | L² | L³ | |
|---|---|---|---|---|---|---|---|
| CH₃O | | CF₃ | | F | H | H | |
| C₂H₅O | | CF₃ | | F | H | H | |
| C₃H₇O | | CF₃ | | F | H | H | |
| CH₃OCH₂ | | CF₃ | | F | H | H | |
| CH₃ | | C₂F₅ | | F | H | H | |
| C₂H₅ | | C₂F₅ | | F | H | H | |

| R¹ | | X | | L¹ | L² | L³ | |
|---|---|---|---|---|---|---|---|
| C₃H₇ | | C₂F₅ | | F | H | H | |
| C₄H₉ | | C₂F₅ | | F | H | H | |
| C₅H₁₁ | | C₂F₅ | | F | H | H | |
| C₆H₁₃ | | C₂F₅ | | F | H | H | |
| CH₂=CH₂ | | C₂F₅ | | F | H | H | |
| CH₃CH=CH | | C₂F₅ | | F | H | H | |

| R¹ | | X | | L¹ | L² | L³ | |
|---|---|---|---|---|---|---|---|
| CH₃O | | C₂F₅ | | F | H | H | |
| C₂H₅O | | C₂F₅ | | F | H | H | |
| C₃H₇O | | C₂F₅ | | F | H | H | |
| CH₃OCH₂ | | C₂F₅ | | F | H | H | |

### Beispiel 2

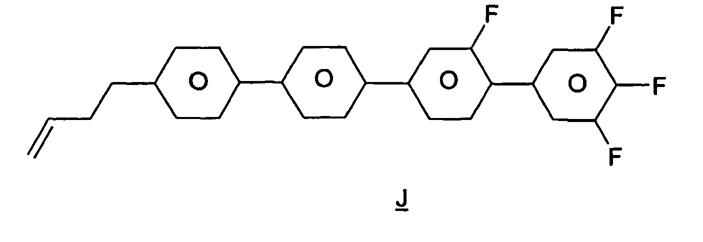

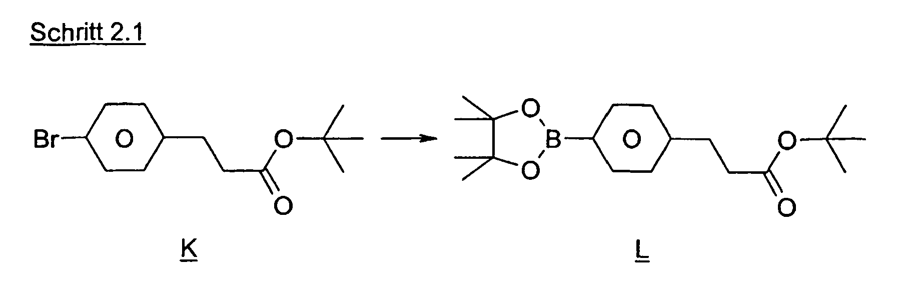

141 mmol K, 4,2 mmol PdCl₂-dppf (dppf = diphenylphosphino-ferrocen), 423 mmol Kaliumacetat und 155 mmol Bis-(pinacolato)-diboron gelöst in 244 ml 1,4-Dioxan werden 16 h bei 100 °C gerührt. Das Reaktionsgemisch wird mit Wasser versetzt und mit Methyl-tert.butylether extrahiert. Die vereinigten organischen Phasen werden mit Wasser gewaschen, über Natriumsulfat getrocknet, filtriert und einrotiert. Der Rückstand wird mit Heptan/Methyl-tert.butylether (3:1) über 2 1 Kieseigel eluiert.

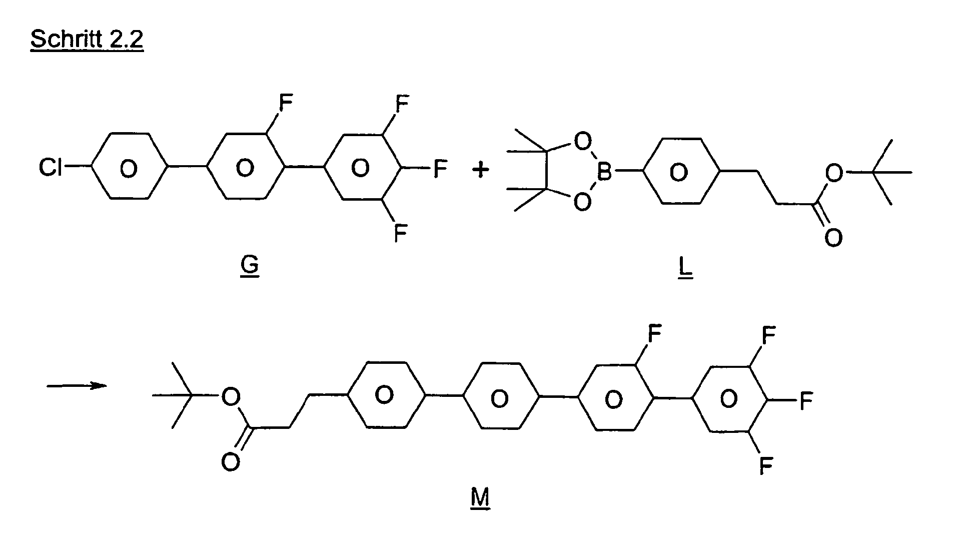

71 mmol G, 78 mmol L, 141 mmol Cäsiumfluorid, 3,55 mmol Bis(tricyclohexylphosphin)PdCl₂ und 396 ml 1,4 Dioxan werden bei 100 °C in einer Stickstoffatmosphäre über Nacht gerührt. Man lässt das Reaktionsgemisch abkühlen, versetzt mit Wasser und extrahiert mit Dichlormethan. Die vereinigten organischen Phasen werden mit Wasser gewaschen, über Natriumsulfat getrocknet, filtriert und einrotiert. Der Rückstand wird mit heißem Toluol über 1,5 l kieseigel eluiert.

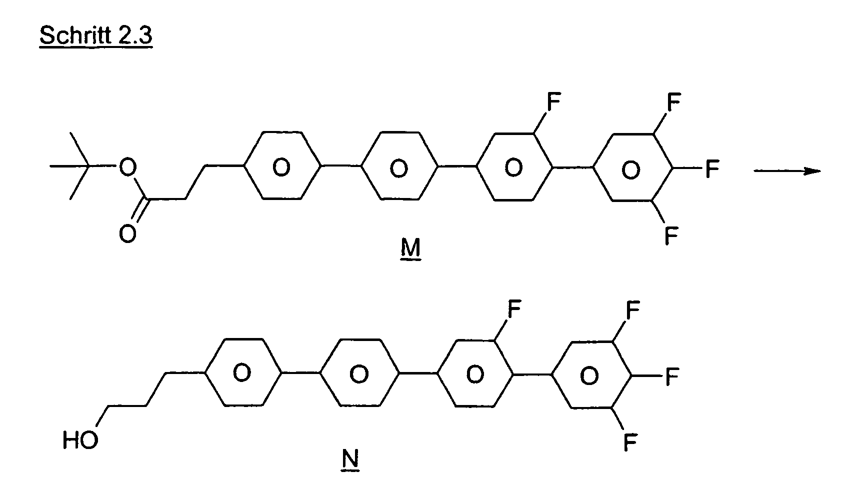

23,7 mmol M in 240 ml Toluol werden bei 0 °C mit 47,4 mmol Diisobutylaluminiumhydrid (DiBALH-Lösung in Toluol) versetzt und über Nacht bei Raumtemperatur gerührt. Das Reaktionsgemisch wird auf Eis gegossen und unter Rühren auf Raumtemperatur erwärmt. Während des Erwärmens wird verdünnte Salzsäure (2N) zugegeben. Nach der Extraktion mit Methyltert.butylether werden die vereinigten organischen Phasen mit Wasser gewaschen, über Natriumsulfat getrocknet, filtriert und einrotiert. Das Rohprodukt wird über 500 ml Kieselgel eluiert. Zuletzt wird mit Dichlormethan / Methyl-tert.butylether (1:1) chromatographiert.

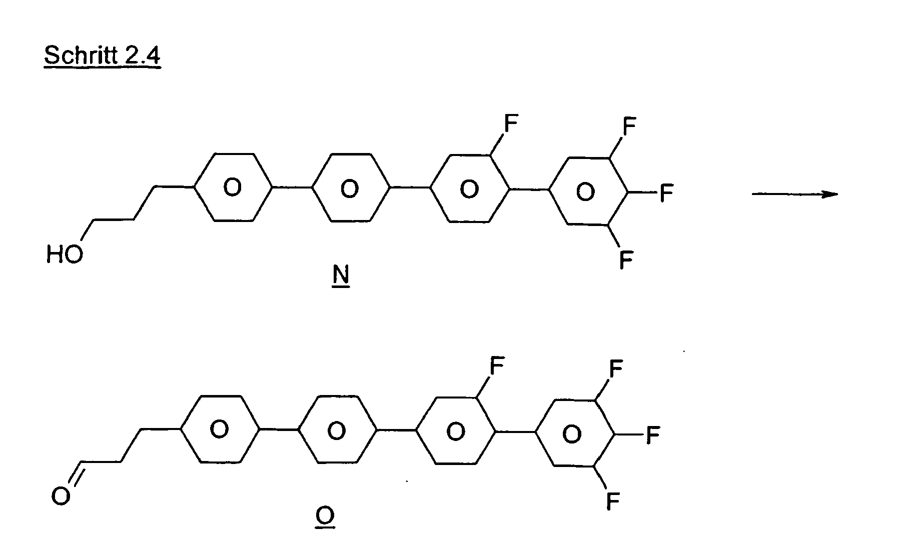

1412 µmol KBr in 2,8 ml VE-Wasser werden vorgelegt und mit 14 mmol N in 26 ml Dichlormethan versetzt. Nach Zugabe von 141 µmol TEMPO (2,2,6,6-Tetramethylpiperidin-1-oxyl) wird das Reaktionsgemisch auf 0 °C gekühlt. Bei dieser Temperatur werden 17,7 mmol Natriumhypochlorit-Lösung (6-14 % aktives Chlor) zugegeben, das zuvor mit Natriumhydrogencarbonat-Lösung auf ca. pH = 8,5 eingestellt wird. Nach 0,5 h Rühren wird erneut Natriumhypochlorit-Lösung zugegeben bis kein Ausgangsmaterial mehr in der Reaktionslösung enthalten ist. Das Reaktionsgemisch wird mit Wasser verdünnt und mit Dichlormethan extrahiert. Die vereinigten organischen Phasen werden mit Wasser gewaschen, über Natriumsulfat getrocknet, filtriert und einrotiert.

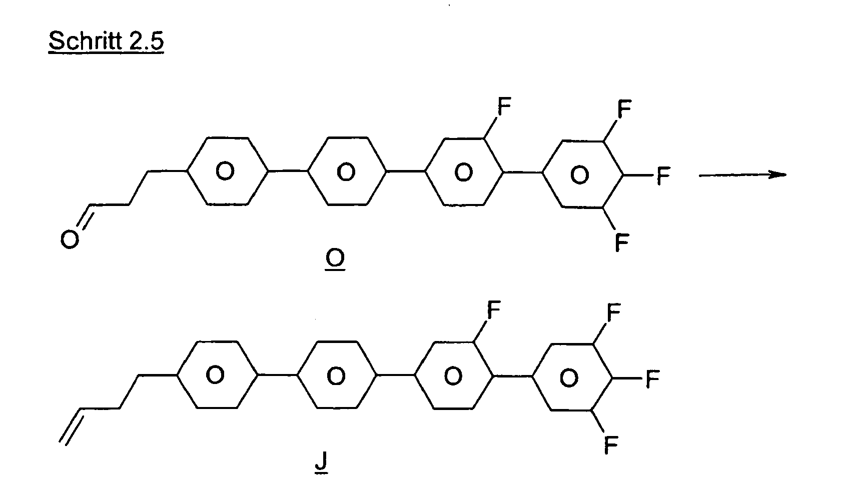

5,3 mmol O, 6,3 mmol Methyl-triphenylphosphoniurn-bromid in 12 ml THF werden bei 0 °C mit 6,3 mmol Kalium-tert.butylat in 13 ml THF versetzt. Man lässt über Nacht bei Raumtemperatur rühren. Das Reaktionsgemisch wird angesäuert und mit Dichlormethan extrahiert. Die vereinigten organischen Phasen werden mit Wasser gewaschen, über Natriumsulfat getrocknet, filtriert und einrotiert. Anschließend wird wie üblich aufgearbeitet.
K 106 S_{E} 124 S_{c} 152 S_{A} 176 N 251,6 l

Analog werden die folgenden Verbindungen der Formel 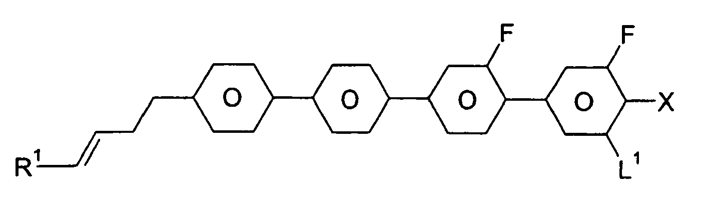 hergestellt:

| X | R¹ | L¹ | |
|---|---|---|---|
| F | H | H | |
| Cl | H | H | |
| Cl | H | F | |
| CN | H | H | |
| CN | H | F | |
| OCF₃ | H | H | |
| OCF₃ | H | F | |
| OCHF₂ | H | H | |
| OCHF₂ | H | F | |
| OC₂F₅ | H | H | |
| OC₂F₅ | H | F | |
| OC₃F₇ | H | H | |
| OC₃F₇ | H | F | |
| OCF₂CHFCF₃ | H | H | |
| OCF₂CHFCF₃ | H | F | |
| NCS | H | H | |
| NCS | H | F | |
| SCN | H | H | |
| SCN | H | F | |
| SF₅ | H | H | |
| SF₅ | H | F | |
| CF₃ | H | H | |
| CF₃ | H | F | |
| C₂F₅ | H | H | |
| C₂F₅ | H | F | |
| OCH₂F | H | H | |
| OCH₂F | H | F | |
| F | CH₃ | H | |
| F | CH₃ | F | S_{E} 165 S_{A} 214 N 263,3 I |
| Cl | CH₃ | H | |
| Cl | CH₃ | F | |
| CN | CH₃ | H | |
| CN | CH₃ | F | |
| OCF₃ | CH₃ | H | |
| OCF₃ | CH₃ | F | |
| OCHF₂ | CH₃ | H | |
| OCHF₂ | CH₃ | F | |
| OC₂F₅ | CH₃ | H | |
| OC₂F₅ | CH₃ | F | |
| OC₃F₇ | CH₃ | H | |
| OC₃F₇ | CH₃ | F | |
| OCF₂CHFCF₃ | CH₃ | H | |
| OCF₂CHFCF₃ | CH₃ | F | |
| NCS | CH₃ | H | |
| NCS | CH₃ | F | |
| SCN | CH₃ | H | |
| SCN | CH₃ | F | |
| SF₅ | CH₃ | H | |
| SF₅ | CH₃ | F | |
| CF₃ | CH₃ | H | |
| CF₃ | CH₃ | F | |
| C₂F₅ | CH₃ | H | |
| C₂F₅ | CH₃ | F | |
| OCH₂F | CH₃ | H | |
| OCH₂F | CH₃ | F | |

### Mischungsbeispiele

### Beispiel M 1

| | | | |
|---|---|---|---|
| PGP-2-3 | 15,00 % | Klärpunkt [°C]: | 88,0 |
| PGP-2-4 | 15,00 % | Δn [589 nm, 20 °C]: | 0,2023 |
| PGP-3-2 | 9,00 % | Δε [1 kHz, 20 °C]: | 5,0 |
| PCH-301 | 19,00 % | K₁ [pN, 20 °C]: | 13,0 |
| GGP-2-F | 9,00 % | γ₁ [mPa·s, 20 °C]: | 154 |
| GGP-3-F | 11,00 % | V₀ [V]: | 1,70 |
| CGG-3-F | 16,00 % | | |
| PPGU-3-F | 6,00 % | | |

### Beispiel M2

| | |
|---|---|
| PGP-2-3 | 14,00 % |
| PGP-2-4 | 14,00 % |
| PGP-3-2 | 10,00 % |
| PCH-301 | 21,00 % |
| GGP-2-F | 9,00 % |
| GGP-3-F | 9,00 % |
| CGG-3-F | 15,00 % |
| PPGU-3-F | 4,00 % |
| PPGU-5-F | 4,00 % |

## Patentansprüche

1. Flüssigkristallines Medium auf der Basis eines Gemisches von polaren Verbindungen, **dadurch gekennzeichnet, dass** es eine, zwei oder mehr Verbindungen der Formeln I-1 bis I-10, 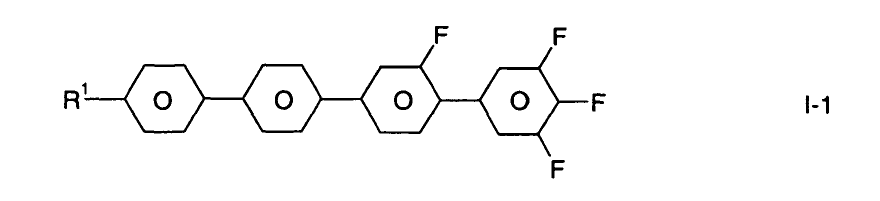 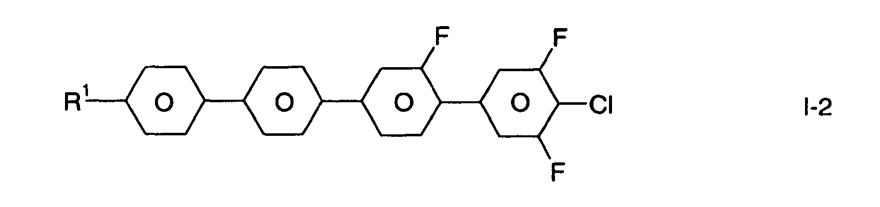 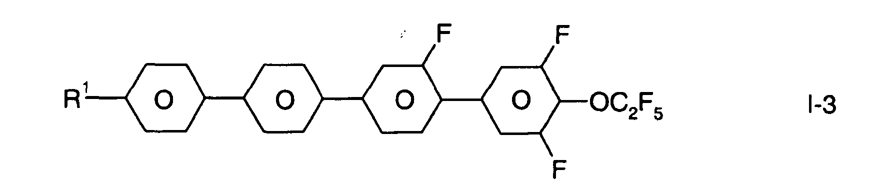 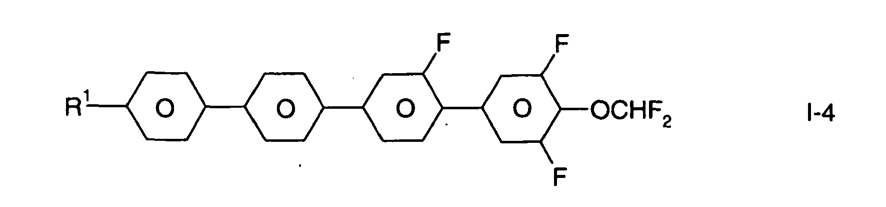 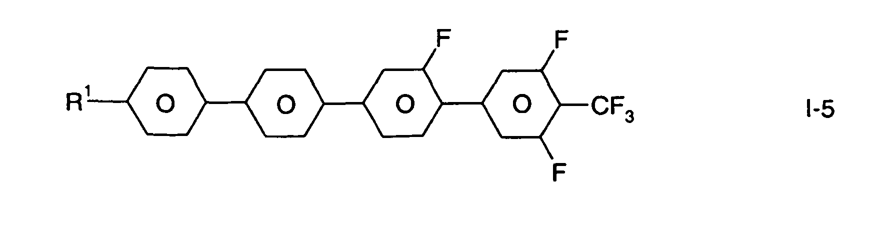 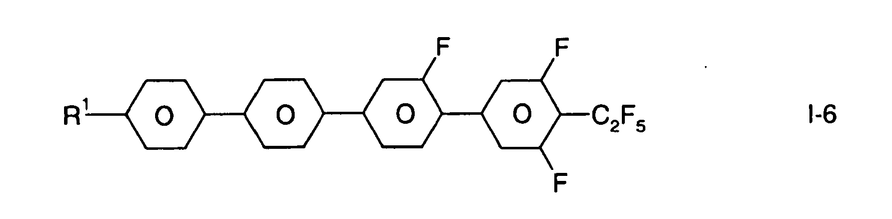 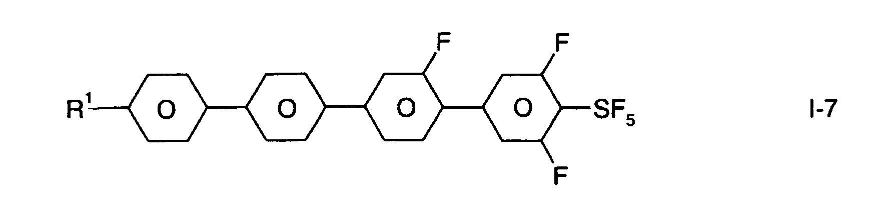 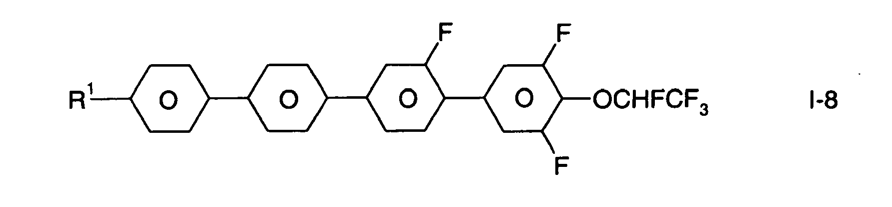 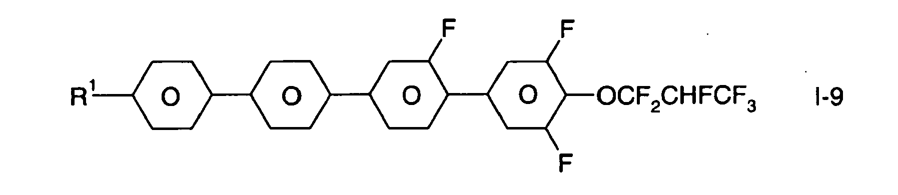 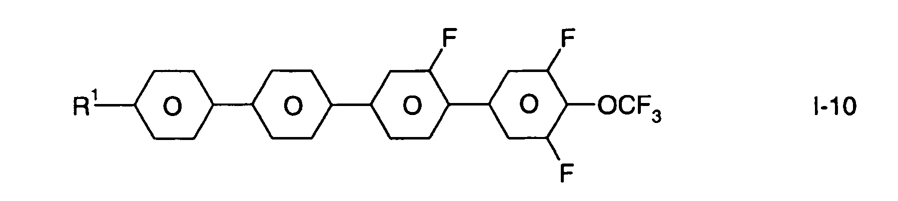 worin
R¹ einen halogenierten oder unsubstituierten Alkyl- oder Alkoxyrest mit 1 bis 15 C-Atomen, wobei in diesen Resten auch eine oder mehrere CH₂-Gruppen jeweils unabhängig voneinander durch 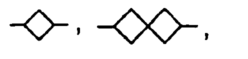 -C≡C-, -CH=CH-, -O-, -CF₂O-, -OCF₂-, -CO-O- oder -O-CO- so ersetzt sein können, dass O-Atome nicht direkt miteinander verknüpft sind,
bedeutet,
und
eine oder mehrere Verbindungen der Formel I* 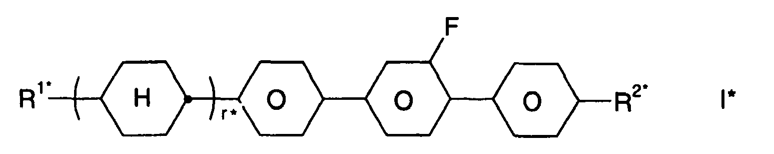 worin
R^{1*} und R^{2*} jeweils unabhängig voneinander Alkyl, Alkoxy, Oxaalkyl, Fluoralkyl, Alkenyloxy oder Alkenyl mit jeweils bis zu 9 C-Atomen, und
r* 0 oder 1.
bedeuten,
enthält.

2. Flüssigkristallines Medium nach Anspruch 1, **dadurch gekennzeichnet, dass** R¹ Alkyl oder Alkenyl bedeutet.

3. Flüssigkristallines Medium nach Anspruch 1 oder 2, **dadurch gekennzeichnet, dass** es eine oder mehrere Verbindungen der Formeln I*-1 bis I*-8, 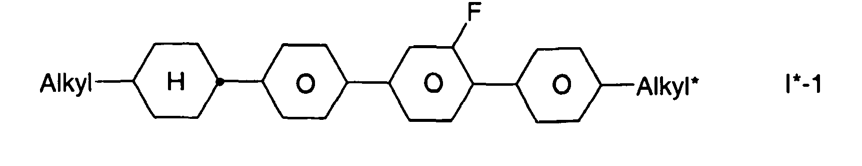 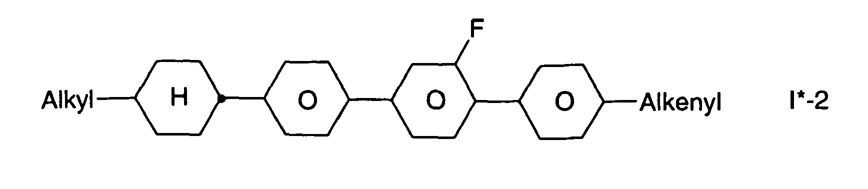 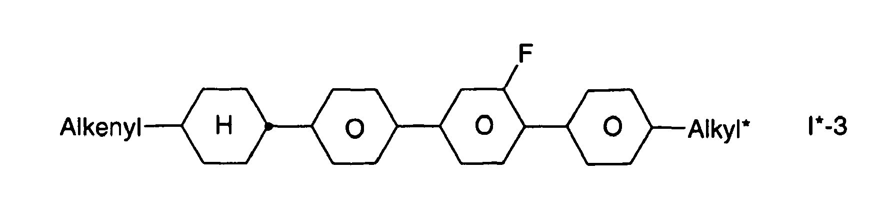 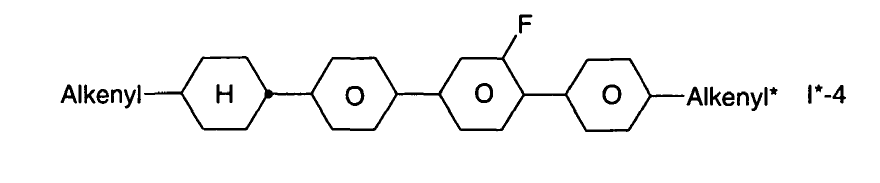 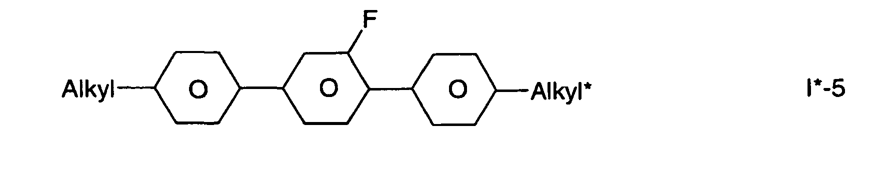 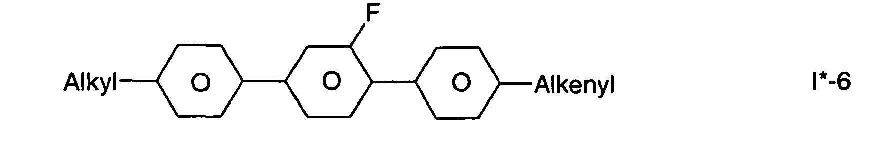 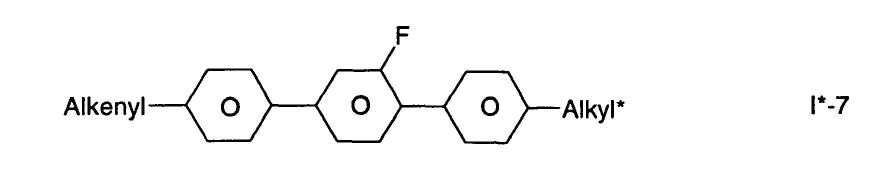 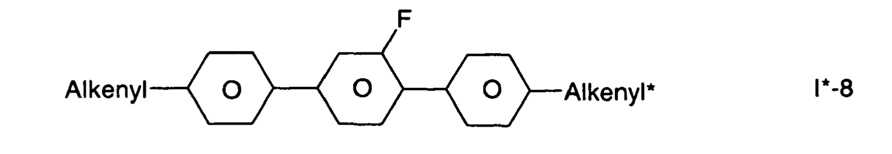 worin
Alkyl und Alkyl* jeweils unabhängig voneinander geradkettiges Alkyl mit 1-6 C-Atomen, und
Alkenyl und Alkenyl* jeweils unabhängig voneinander geradkettiges Alkenyl mit 2-6 C-Atomen,
bedeuten,
enthält.

4. Flüssigkristallines Medium nach einem der Ansprüche 1 bis 3, **dadurch gekennzeichnet, dass** es zusätzlich eine oder mehrere Verbindungen ausgewählt aus der Gruppe bestehend aus den allgemeinen Formeln XIII bis XIX, 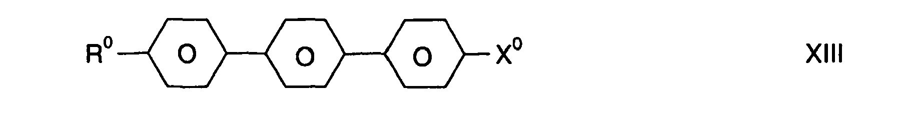 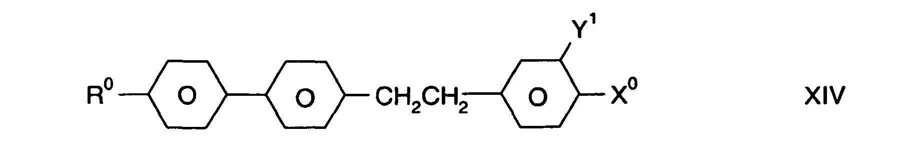 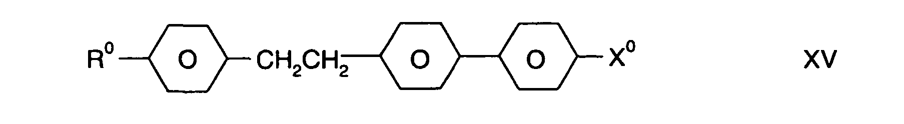 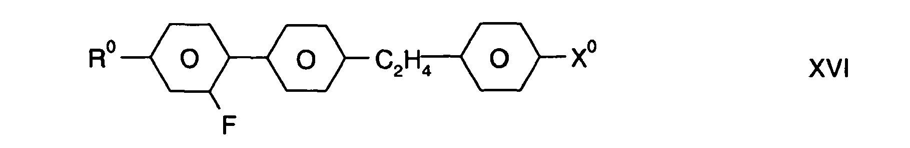 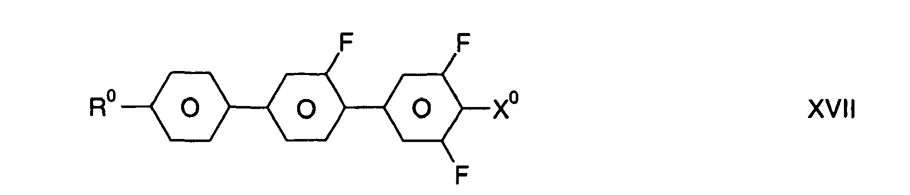 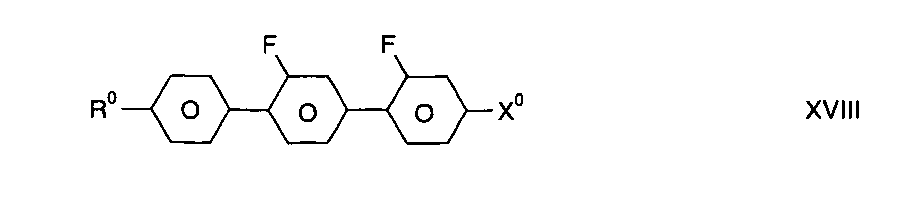 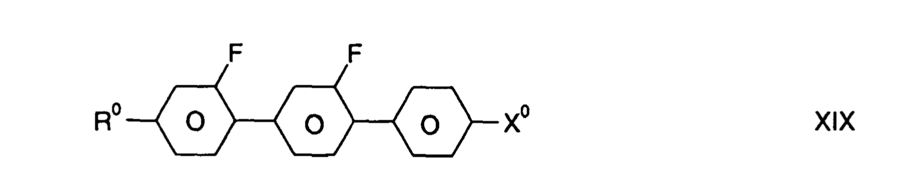 worin
R⁰ Alkyl, Alkoxy, Oxaalkyl, Fluoralkyl oder Alkenyl mit jeweils bis zu 9 C-Atomen und
X⁰ F, CN, halogeniertes Alkyl, halogeniertes Alkenyl, halogeniertes Oxaalkyl, halogeniertes Alkenyloxy oder halogeniertes Alkoxy mit bis zu 6 C-Atomen, 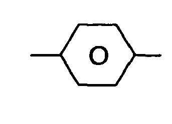
gegebenenfalls ein- oder mehrfach durch Cl, CN oder F substituiert
bedeuten,
enthält.

5. Flüssigkristallines Medium nach Anspruch 4, **dadurch gekennzeichnet, dass** der Anteil an Verbindungen der Formeln XIII bis XIX zusammen im Gesamtgemisch mindestens 0,05-30 Gew.% beträgt.

6. Flüssigkristallines Medium nach mindestens einem der Ansprüche 1 bis 5, **dadurch gekennzeichnet, dass** es zusätzlich eine oder mehrere Verbindungen ausgewählt aus der Gruppe bestehend aus den allgemeinen Formeln II bis VI, 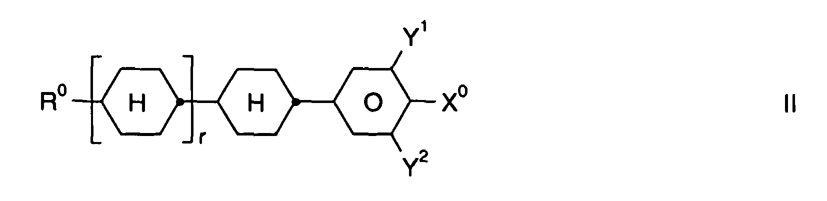 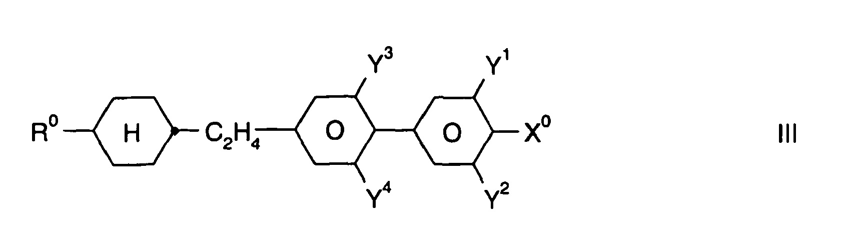 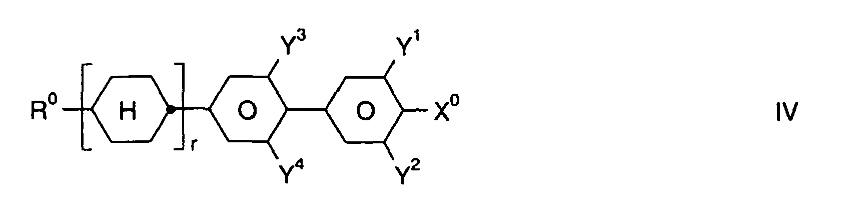 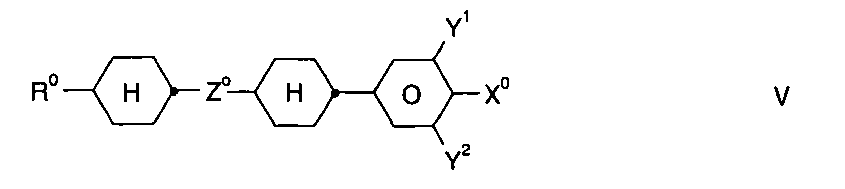 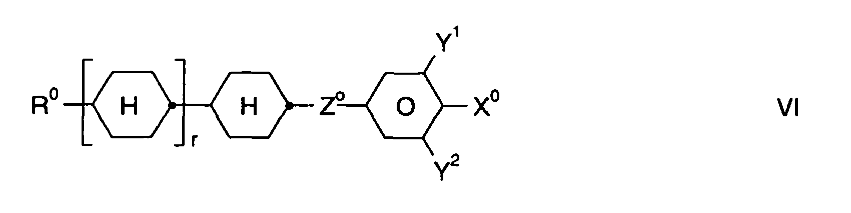 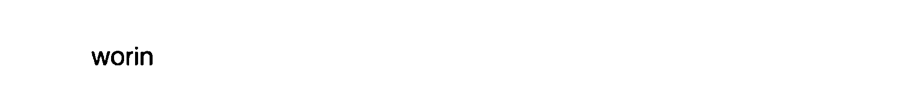
R⁰ Alkyl, Alkoxy, Oxaalkyl, Fluoralkyl oder Alkenyl mit jeweils bis zu 9 C-Atomen,
X⁰ F, Cl, halogeniertes Alkyl, halogeniertes Alkenyl, halogeniertes Oxaalkyl, halogeniertes Alkenyloxy oder halogeniertes Alkoxy mit bis zu 6 C-Atomen,
Z⁰ -C₂F₄₋, -CF=CF, -CH=CH-, -C₂H₄-, -(CH₂)₄-, -OCH₂-, -CH₂O-, -CF₂O- oder -OCF₂-,
Y¹ bis Y⁴ jeweils unabhängig voneinander H oder F,
r 0 oder 1.
bedeuten,
enthält.

7. Flüssigkristallines Medium nach mindestens einem der Ansprüche 1 bis 6, **dadurch gekennzeichnet, dass** der Anteil an Verbindungen der Formeln I-1 bis I-10 im Gesamtgemisch 0,01 bis 30,0 Gew.% beträgt.

8. Flüssigkristallines Medium nach einem der Ansprüche 1 bis 7,
**dadurch gekennzeichnet, dass** der Anteil an Verbindungen der Formeln I-1 bis I-10 und I* im Gesamtgemisch mindestens 5 Gew.% beträgt.

9. Verwendung des flüssigkristallinen Mediums nach mindestens einem der Ansprüche 1 bis 8 für elektrooptische Zwecke.

10. Elektrooptische Flüssigkristallanzeige enthaltend ein flüssigkristallines Medium nach mindestens einem der Ansprüche 1 bis 8.

## Claims

1. Liquid-crystalline medium based on a mixture of polar compounds, **characterised in that** it comprises one, two or more compounds of the formulae I-1 to I-10, 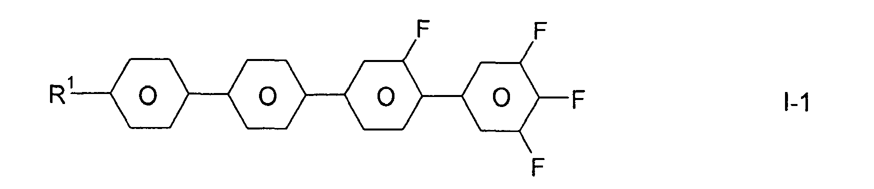 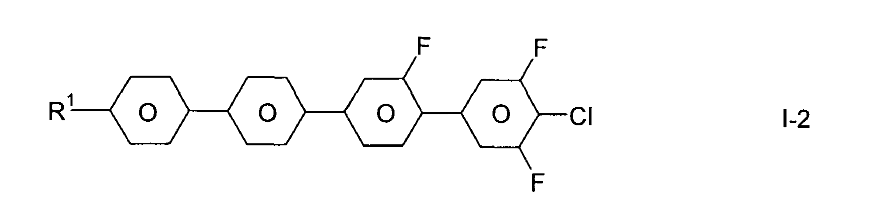 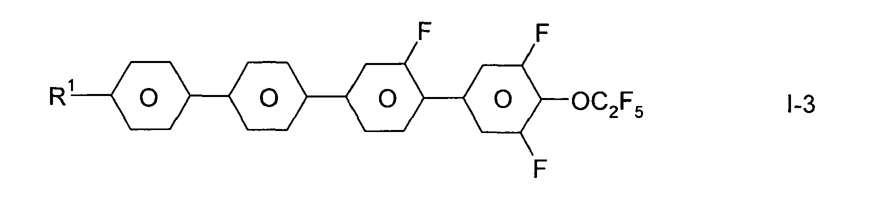 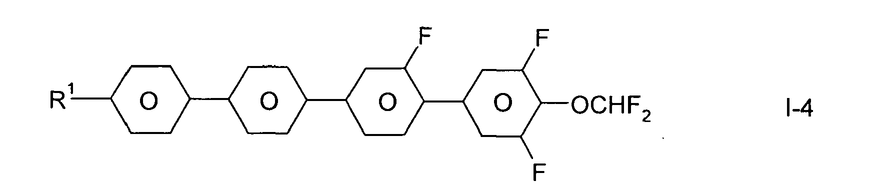 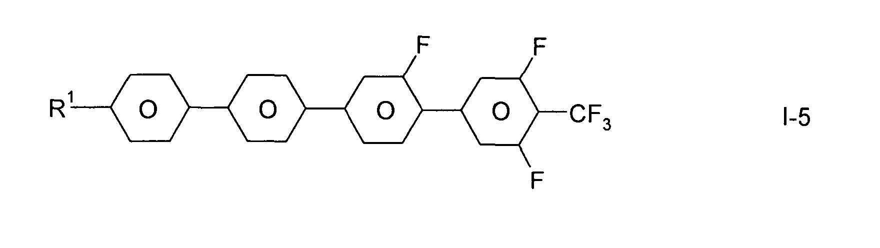 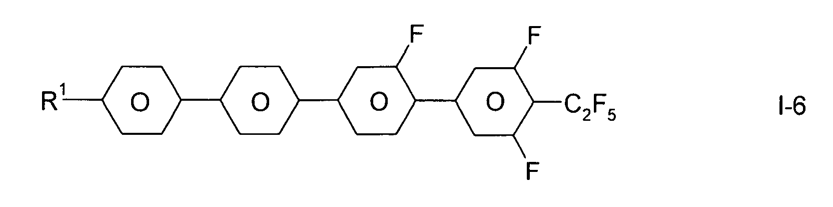 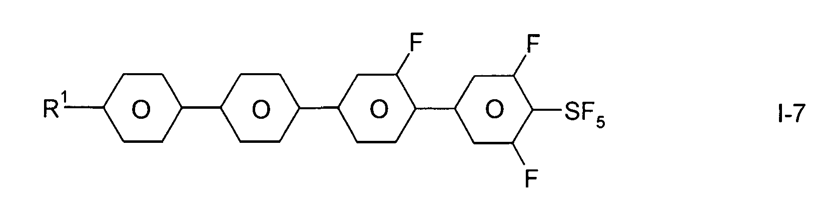 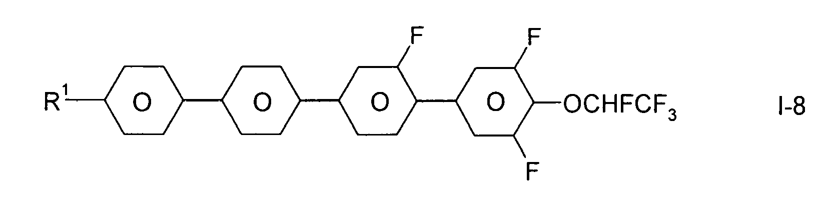 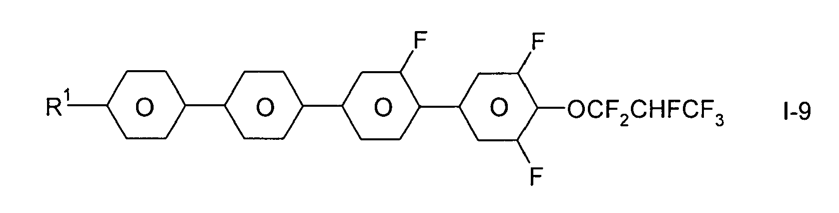 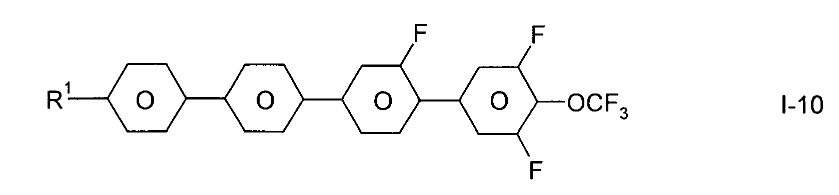 in which
R¹ denotes a halogenated or unsubstituted alkyl or alkoxy radical having 1 to 15 C atoms, where, in addition, one or more CH₂ groups in these radicals may each be replaced, independently of one another, by 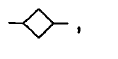 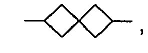 -C≡C-, -CH=CH-, -O-, -CF₂O-, -OCF₂-, -CO-O- or -O-CO- in such a way that O atoms are not linked directly to one another,
and
one or more compounds of the formula I* 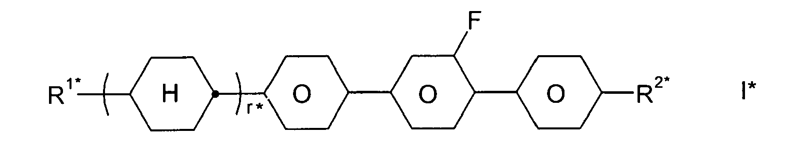 in which
R^{1*} and R^{2*} each, independently of one another, denote alkyl, alkoxy, oxaalkyl, fluoroalkyl, alkenyloxy or alkenyl, each having up to 9 C atoms, and
r* denotes 0 or 1.

2. Liquid-crystalline medium according to Claim 1, **characterised in that** R¹ denotes alkyl or alkenyl.

3. Liquid-crystalline medium according to Claim 1 or 2, **characterised in that** it comprises one or more compounds of the formulae I*-1 to I*-8, 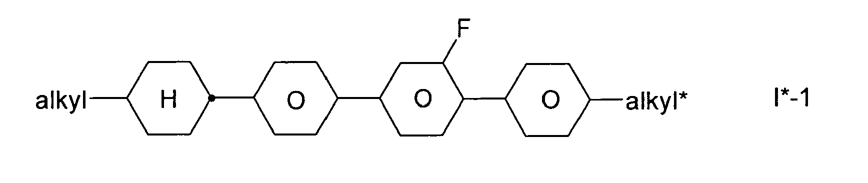 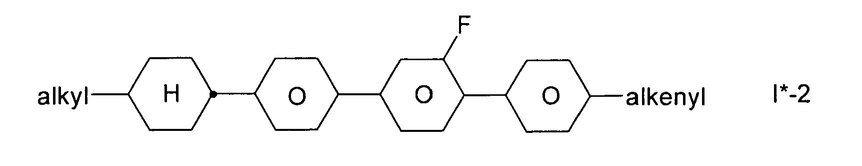 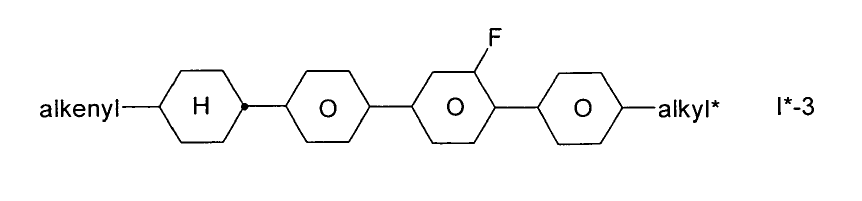 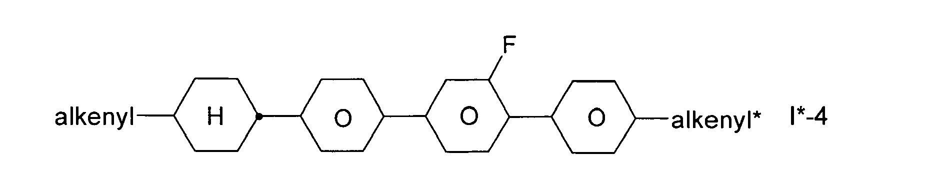 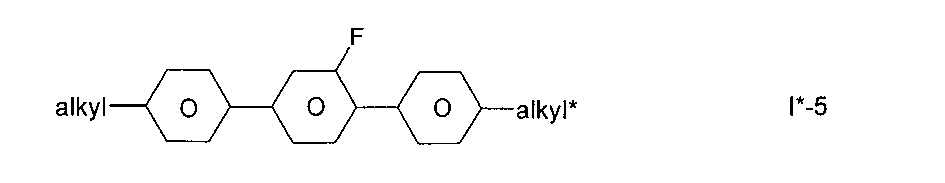 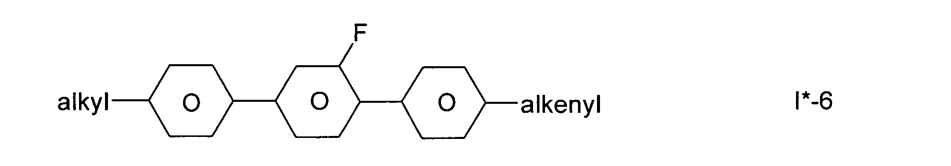 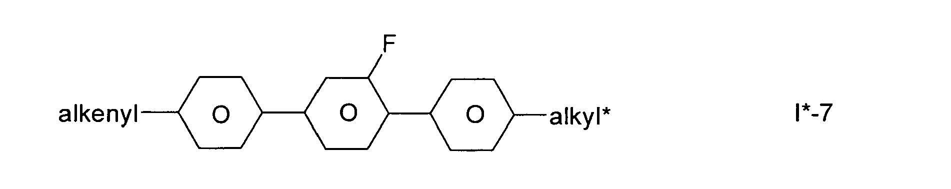 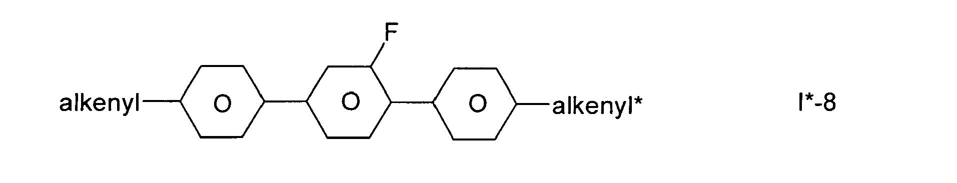 in which
alkyl and alkyl* each, independently of one another, denote straight-chain alkyl having 1-6 C atoms, and
alkenyl and alkenyl* each, independently of one another, denote straight-chain alkenyl having 2-6 C atoms.

4. Liquid-crystalline medium according to one of Claims 1 to 3, **characterised in that** it additionally comprises one or more compounds selected from the group consisting of the general formulae XIII to XIX, 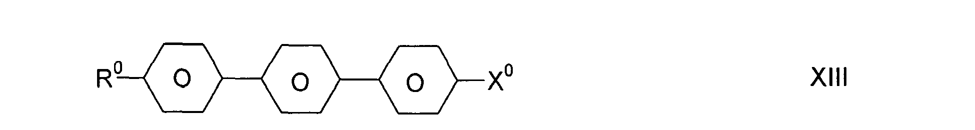 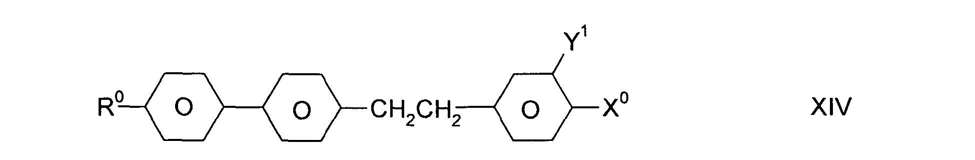 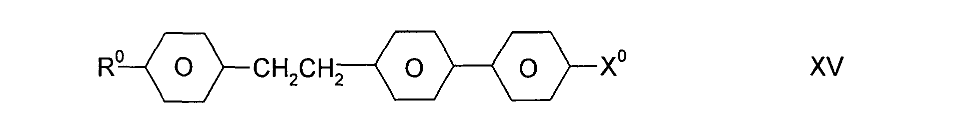 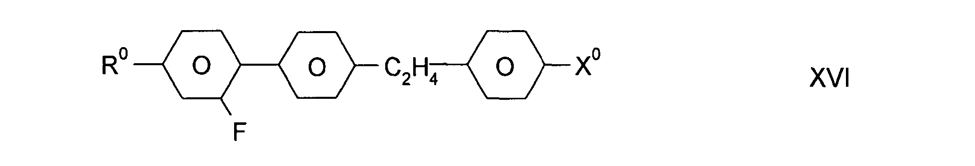 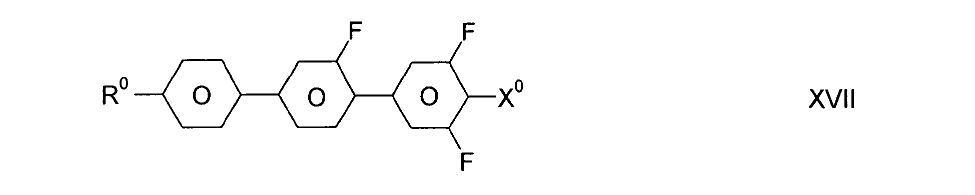 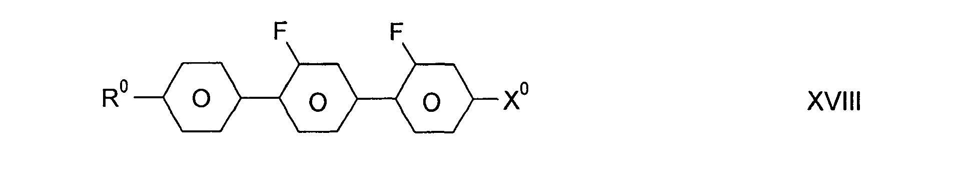 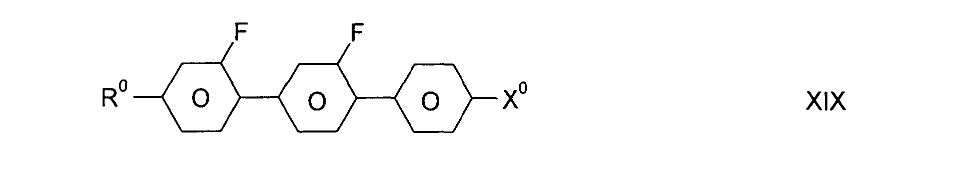 in which
R⁰ denotes alkyl, alkoxy, oxaalkyl, fluoroalkyl or alkenyl, each having up to 9 C atoms, and
X⁰ denotes F, CN, halogenated alkyl, halogenated alkenyl, halogenated oxaalkyl, halogenated alkenyloxy or halo- genated alkoxy having up to 6 C atoms, 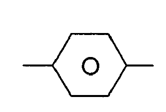
denotes optionally mono- or polysubstituted by Cl, CN or F.

5. Liquid-crystalline medium according to Claim 4, **characterised in that** the proportion of compounds of the formulae XIII to XIX together in the mixture as a whole is at least 0.05-30% by weight.

6. Liquid-crystalline medium according to at least one of Claims 1 to 5, **characterised in that** it additionally comprises one or more compounds selected from the group consisting of the general formulae II to VI, 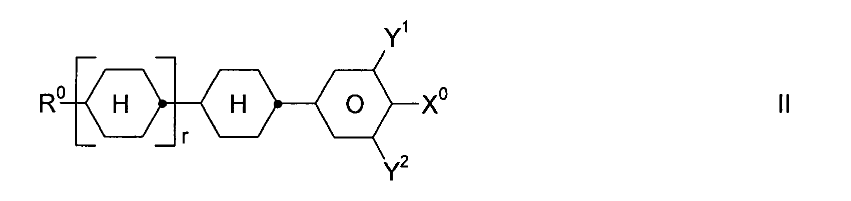 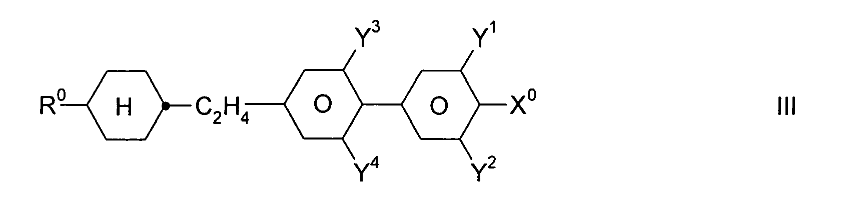 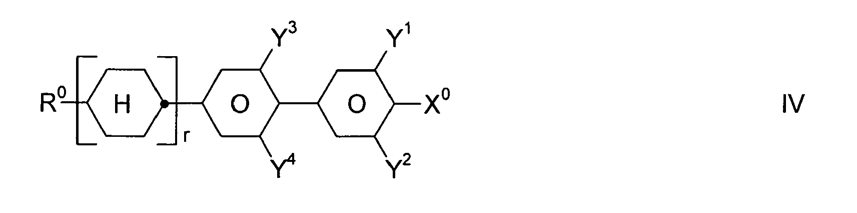 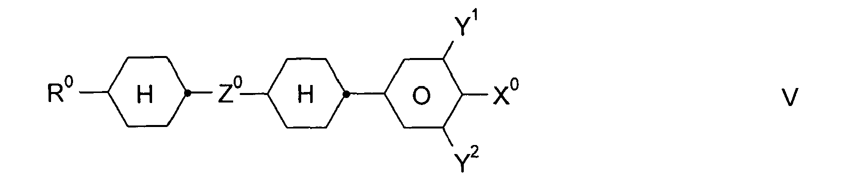 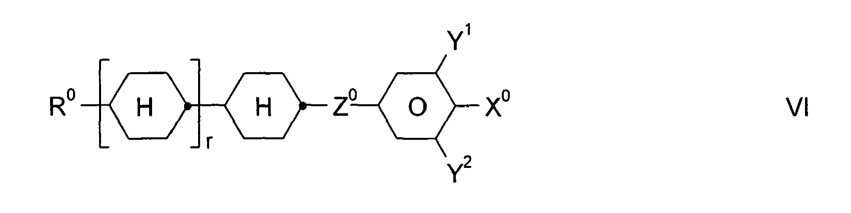 in which
R⁰ denotes alkyl, alkoxy, oxaalkyl, fluoroalkyl or alkenyl, each having up to 9 C atoms,
X⁰ denotes F, Cl, halogenated alkyl, halogenated alkenyl, halogenated oxaalkyl, halogenated alkenyloxy or halo- genated alkoxy having up to 6 C atoms,
Z⁰ denotes -C₂F₄-, -CF=CF-, -CH=CH-, -C₂H₄-, -(CH₂)₄-, -OCH₂-, -CH₂O-, -CF₂O- or -OCF₂-,
Y¹ to Y⁴ each, independently of one another, denote H or F,
r denotes 0 or 1.

7. Liquid-crystalline medium according to at least one of Claims 1 to 6, **characterised in that** the proportion of compounds of the formulae I-1 to I-10 in the mixture as a whole is 0.01 to 30.0% by weight.

8. Liquid-crystalline medium according to one of Claims 1 to 7, **characterised in that** the proportion of compounds of the formulae I-1 to I-10 and I* in the mixture as a whole is at least 5% by weight.

9. Use of the liquid-crystalline medium according to at least one of Claims 1 to 8 for electro-optical purposes.

10. Electro-optical liquid-crystal display containing a liquid-crystalline medium according to at least one of Claims 1 to 8.

## Revendications

1. Milieu cristallin liquide basé sur un mélange de composés polaires, **caractérisé en ce qu'**il comprend un, deux composés ou plus des formules I-1 à I-10, 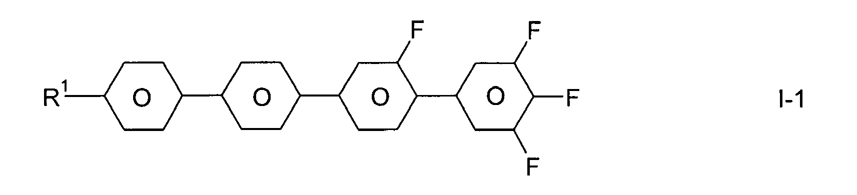 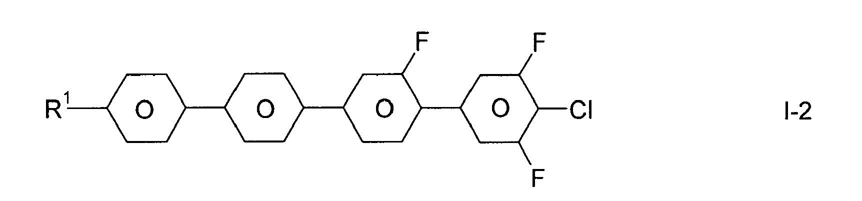 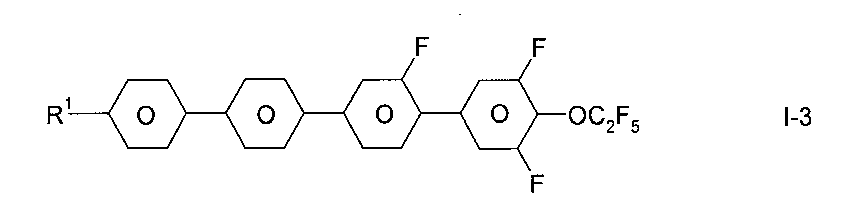 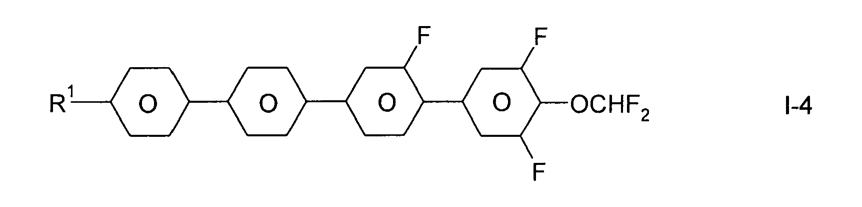 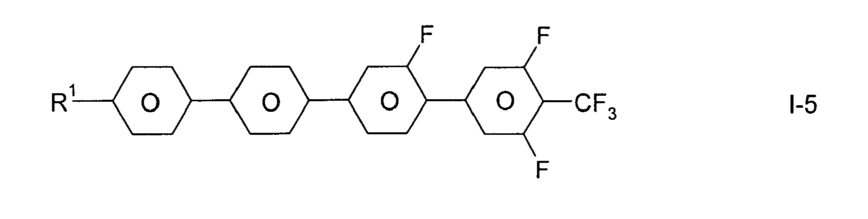 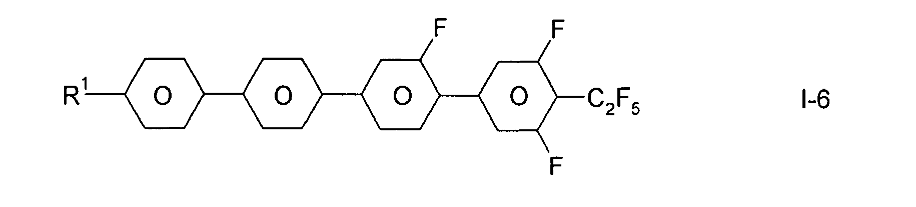 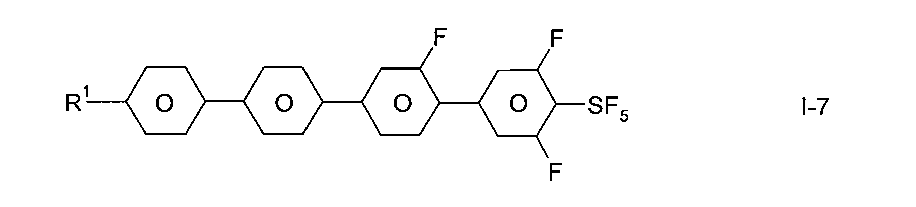 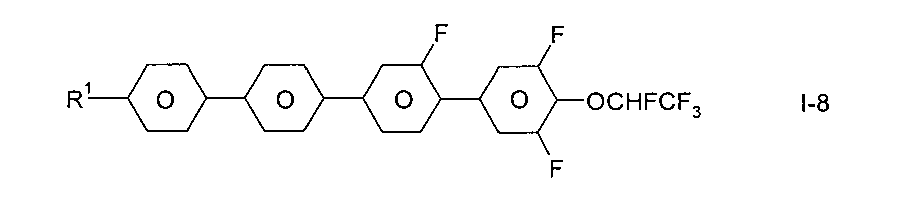 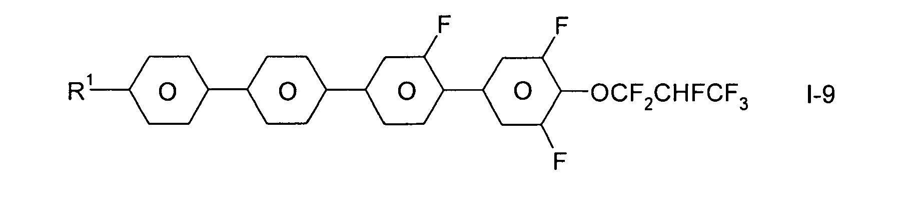 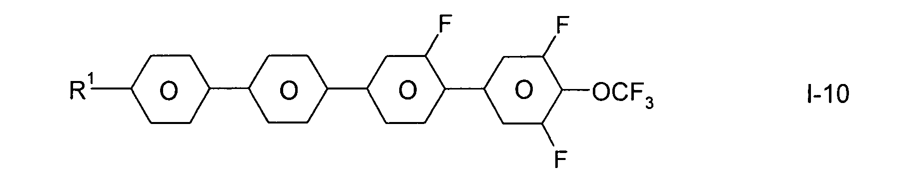 dans lesquelles
R¹ représente un radical alkyle ou alcoxy halogéné ou non
substitué comportant de 1 à 15 atomes de C, où, en outre, un ou plusieurs groupes CH₂ dans ces radicaux peuvent chacun être remplacés, indépendamment les
uns des autres, par 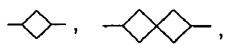 -C≡C-, -CH=CH-, -O-, -CF₂O-, -OCF₂-, -CO-O- ou -O-CO- de telle sorte que des atomes de O ne soient pas liés directement les uns aux autres,
et
un ou plusieurs composés de la formule I* 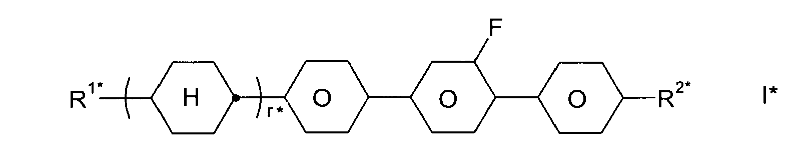 dans laquelle
R^{1*} et R^{2*} représentent, chacun indépendamment l'un de l'autre, alkyle, alcoxy, oxaalkyle, fluoroalkyle, alkényloxy ou alkényle, chacun comportant jusqu'à 9 atomes de C, et
r^{*} représente 0 ou 1.

2. Milieu cristallin liquide selon la revendication 1, **caractérisé en ce que** R¹ représente alkyle ou alkényle.

3. Milieu cristallin liquide selon la revendication 1 ou 2, **caractérisé en ce qu'**il comprend un ou plusieurs composés des formules I*-1 à I*-8, 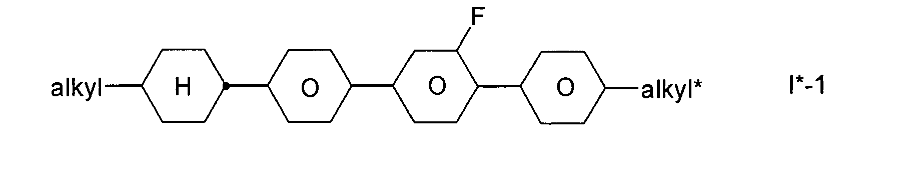 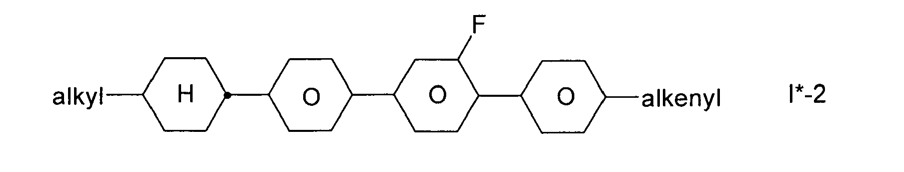 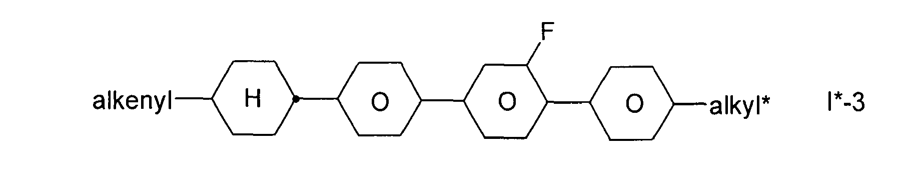 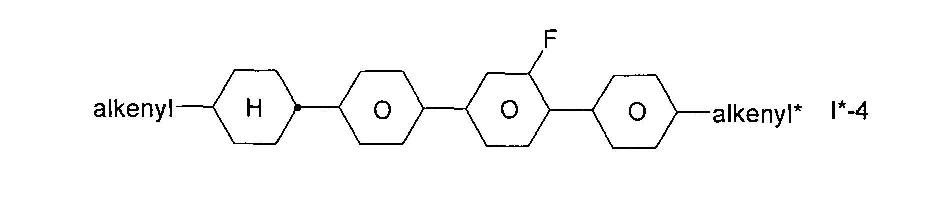 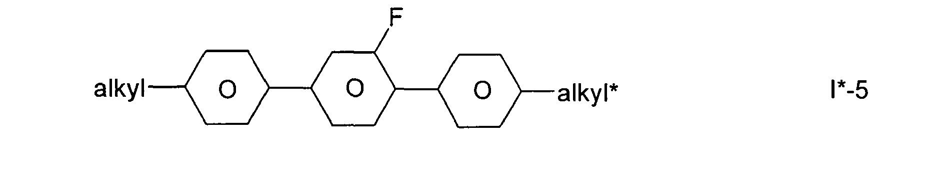 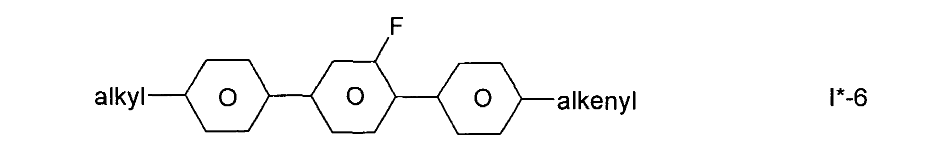 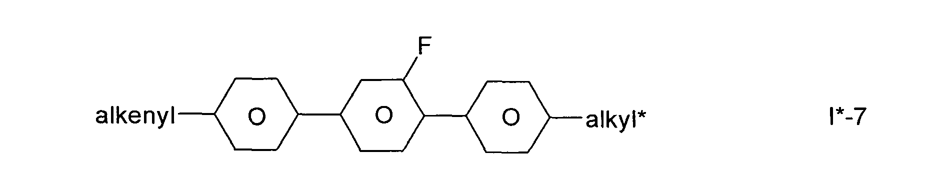 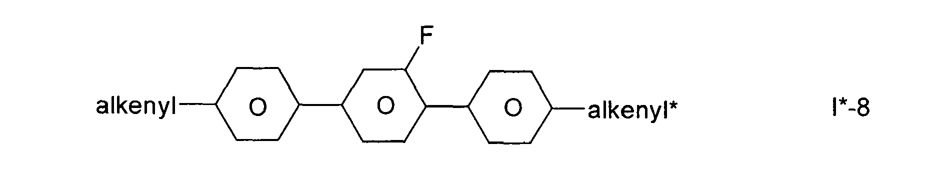 dans lesquelles
alkyl et alkyl* représentent, chacun indépendamment l'un de l'autre, alkyle en chaîne droite comportant 1-6 atomes de C, et
alkenyl et alkenyl* représentent, chacun indépendamment l'un de l'autre, alkényle en chaîne droite comportant 2-6 atomes de C.

4. Milieu cristallin liquide selon l'une des revendications 1 à 3, **caractérisé en ce qu'**il comprend additionnellement un ou plusieurs composés choisis parmi le groupe constitué par les formules générales XIII à XIX, 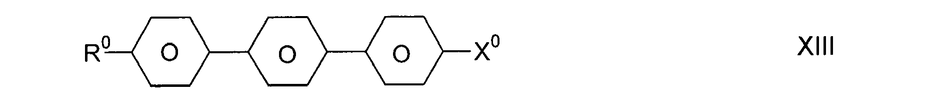 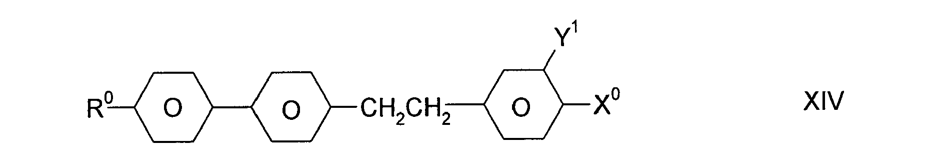 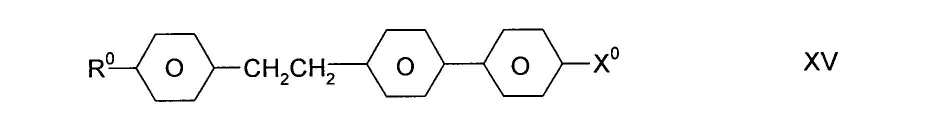 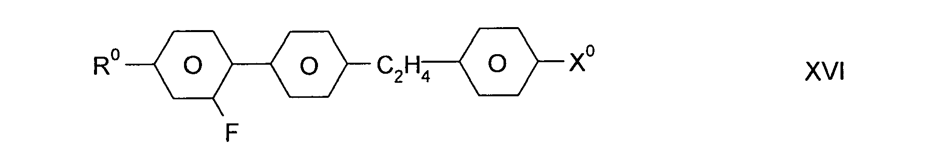 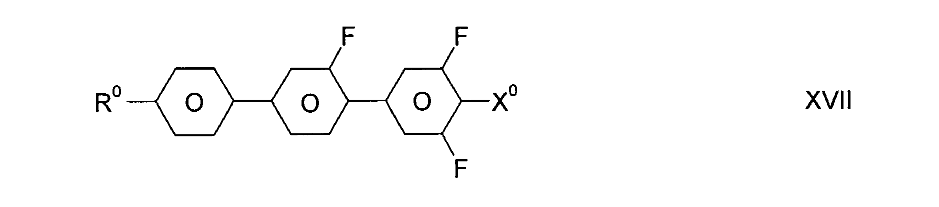 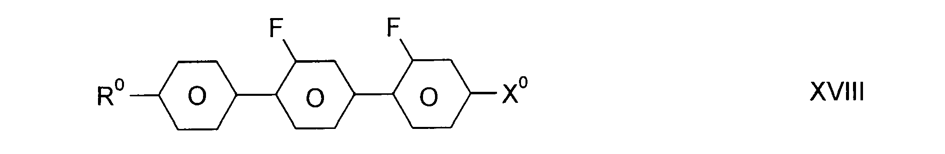 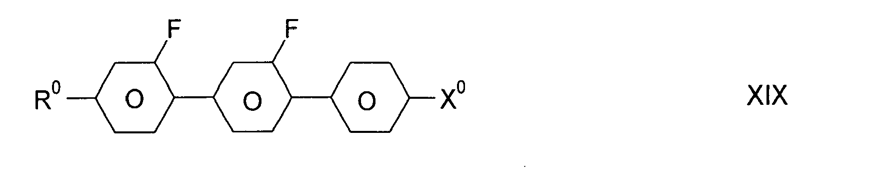 dans lesquelles
R⁰ représente alkyle, alcoxy, oxaalkyle, fluoroalkyle ou alkényle, chacun comportant jusqu'à 9 atomes de C, et
X⁰ représente F, CN, alkyle halogéné, alkényle halogéné, oxaalkyle halogéné, alkényloxy halogéné ou alcoxy halogéné comportant jusqu'à 6 atomes de C, 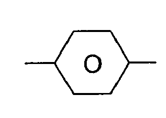
est en option mono- ou polysubstitué par CI, CN ou F.

5. Milieu cristallin liquide selon la revendication 4, **caractérisé en ce que** la proportion de composés des formules XIII à XIX ensemble dans le mélange pris dans sa globalité est d'au moins 0,05-30% en poids.

6. Milieu cristallin liquide selon au moins l'une des revendications 1 à 5, **caractérisé en ce qu'**il comprend additionnellement un ou plusieurs composés choisis parmi le groupe constitué par les formules générales II à VI, 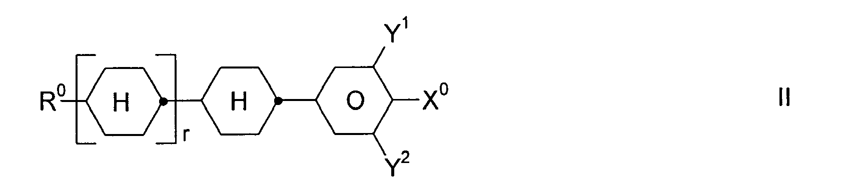 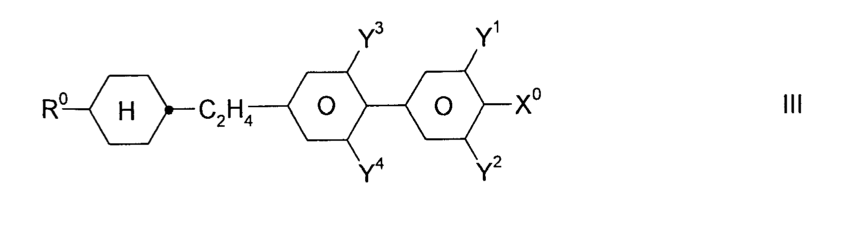 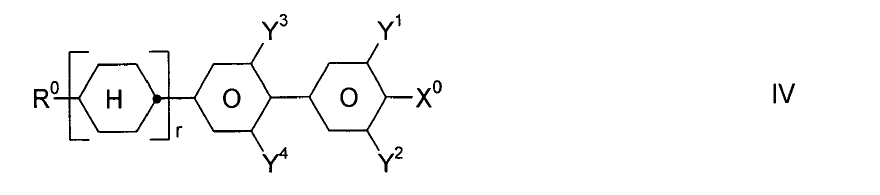 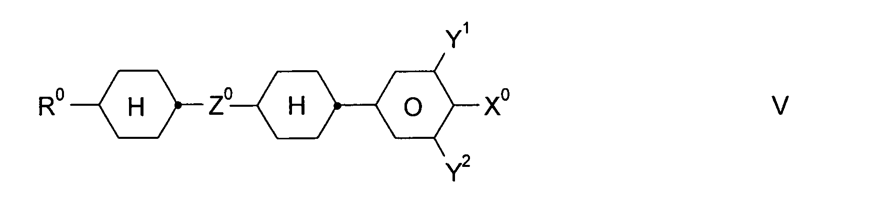 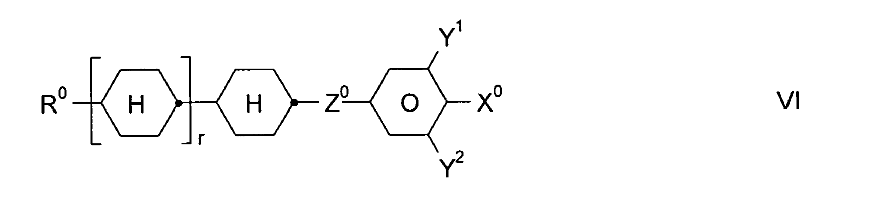 dans lesquelles
R⁰ représente alkyle, alcoxy, oxaalkyle, fluoroalkyle ou alkényle, chacun comportant jusqu'à 9 atomes de C,
X⁰ représente F, CI, alkyle halogéné, alkényle halogéné, oxaalkyle halogéné, alkényloxy halogéné ou alcoxy halogéné comportant jusqu'à 6 atomes de C,
Z⁰ représente -C₂F₄-, -CF=CF-, -CH=CH-, -C₂H₄-, -(CH₂)₄-, -OCH₂-, -CH₂O-, -CF₂O- ou -OCF₂-,
Y¹ à Y⁴ représentent, chacun indépendamment des autres, H ou F,
r représente 0 ou 1.

7. Milieu cristallin liquide selon au moins l'une des revendications 1 à 6, **caractérisé en ce que** la proportion de composés des formules I-1 à I-10 dans le mélange pris dans sa globalité est de 0,01 à 30,0% en poids.

8. Milieu cristallin liquide selon au moins l'une des revendications 1 à 7, **caractérisé en ce que** la proportion de composés des formules I-1 to I-10 et I* dans le mélange pris dans sa globalité est d'au moins 5% en poids.

9. Utilisation du milieu cristallin liquide selon au moins l'une des revendications 1 à 8 à des fins électro-optiques.

10. Affichage à cristaux liquides électro-optique contenant un milieu cristallin liquide selon au moins l'une des revendications 1 à 8.
